(19) **Europäisches Patentamt / European Patent Office / Office européen des brevets**

(11) **EP 4 736 867 A1**

(12) **EUROPEAN PATENT APPLICATION**
published in accordance with Art. 153(4) EPC

(43) Date of publication:
**06.05.2026 Bulletin 2026/19**

(21) Application number: **23943827.8**

(22) Date of filing: **06.07.2023**

(51) International Patent Classification (IPC):
*A61K 38/45* (2006.01)    *A61K 38/17* (2006.01)
*A61P 25/00* (2006.01)    *A61K 9/00* (2006.01)

(52) Cooperative Patent Classification (CPC):
Y02A 50/30

(86) International application number:
**PCT/KR2023/009608**

(87) International publication number:
**WO 2025/005334 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.06.2023 KR 20230083845**

(71) Applicants:
• **Standup Therapeutics Inc.**
Seoul 04418 (KR)
• **Yoo, Jun Sang**
Seoul 03675 (KR)
• **Shim, Hyun Soo**
Seoul 04572 (KR)

(72) Inventors:
• **CHANG, Yujung**
Seoul 04616 (KR)
• **KIM, Chunggu**
Incheon 22810 (KR)
• **IM, Hyeonjoo**
Seoul 06116 (KR)
• **YOO, Jun Sang**
Seoul 03675 (KR)
• **SHIM, Hyun Soo**
Seoul 04572 (KR)

(74) Representative: **Vossius & Partner
Patentanwälte Rechtsanwälte mbB
Siebertstraße 3
81675 München (DE)**

Remarks:
The complete document including Reference
Table(s) and the Sequence Listing(s) can be
downloaded from the EPO website

(54) **COMPOSITION FOR TREATING SPINAL CORD INJURY DISEASES AND USE THEREOF**

(57)    The present disclosure relates to a composition for treating spinal cord injury disorders and a method of using the same.

EP 4 736 867 A1

【Fig. 8】

**Description**

[Technical Field]

The present disclosure relates to a composition for treating spinal cord injury disorders and a method for treating spinal cord injury disorders using the same.

[Background Art]

The spinal cord, which is a part of the central nervous system located within the spine, is difficult to recover once it is damaged. In particular, damage to the spinal cord disrupts neural transmission between the brain and the body, resulting in loss of motor function and sensory function.

Various neuronal cells and glial cells, including astrocytes, microglia, and motor neurons, are present in the spinal cord.

When motor neurons are destroyed, progressive degeneration occurs in motor nerves, which may affect upper motor neurons that transmit signals from the brain to the medulla or spinal cord, or lower motor neurons that transmit signals from the spinal cord to muscles. In addition, it has been reported that astrocytes form a glial scar at the site of spinal cord injury, thereby blocking ascending and descending neural signal transmission.

Spinal cord injury, which is a damage to the central nervous system, has not yet been provided with a clearly established therapeutic regimen despite advances in medical and surgical treatments. To date, when the spinal cord is damaged, treatments such as steroid therapy that may cause severe side effects and invasive surgical procedures have been frequently employed.

Furthermore, although cell-based therapies using stem cells and compound-based therapies have been developed as therapeutic agents for spinal cord injury, stem cell therapies involve the risk of teratoma formation or tumorigenesis, and compound-based therapies only exhibit temporary improvement in motor function and are insufficient to restore the damaged spinal cord.

Accordingly, there is a need to develop new therapeutic agents for spinal cord injury.

[Detailed Description of the Invention]

[Technical Problem]

An object of the present disclosure is to provide a composition for treating spinal cord injury disorders. In particular, the composition comprises differentiation factors for directly converting somatic cells into motor neurons. Specifically, the composition comprises a Gsta4 (Glutathione S-transferase A4) protein or a nucleic acid encoding the same.

In another object of the present disclosure, a further composition for treating spinal cord injury disorders is provided. In particular, the composition comprises a Gsta4 (Glutathione S-transferase A4) protein or a nucleic acid encoding the same, an Hb9 (MNX1) protein or a nucleic acid sequence encoding the same, and an Lhx3 protein or a nucleic acid sequence encoding the same, as differentiation factors for directly converting somatic cells into motor neurons.

In another object of the present disclosure, a method for preparing the composition is provided.

Another object of the present disclosure is to provide a method for treating spinal cord injury disorders.

A further object of the present disclosure is to provide various uses of the composition.

[Technical Solution]

In order to solve the above-described objects, according to one aspect of the present disclosure, there is provided a pharmaceutical composition for treating a spinal cord injury disorder.

The pharmaceutical composition for treating a spinal cord injury disorder may comprise:

a nucleic acid sequence encoding a Gsta4 (Glutathione S-Transferase Alpha 4) protein;

a nucleic acid sequence encoding an Lhx3 (LIM homeobox 3) protein; and

a nucleic acid sequence encoding an MNX1 (Motor neuron and pancreas homeobox 1) protein.

At this time, the spinal cord injury disorder may be selected from the group consisting of paraplegia, quadriplegia, amyotrophic lateral sclerosis (ALS), primary lateral sclerosis (PLS), progressive pseudobulbar palsy, progressive muscular atrophy, progressive bulbar palsy, and post-polio syndrome.

The nucleic acid sequence encoding the Gsta4 protein, the nucleic acid sequence encoding the Lhx3 protein, and the nucleic acid sequence encoding the MNX1 protein may be independently contained in a vector, or two or more of the nucleic acid sequences may be combined and contained in a vector.

At this time, the vector may be an adeno-associated virus (AAV). The adeno-associated virus may be selected from the group consisting of AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.

The nucleic acid sequence encoding the Gsta4 protein may be the sequence of SEQ ID NO: 12, the nucleic acid sequence encoding the Lhx3 protein may be the sequence of SEQ ID NO: 16, and the nucleic acid sequence encoding the MNX1 protein may be the sequence of SEQ ID NO: 14.

The vector may further comprise a promoter, a 2A self-cleaving peptide, and an inverted terminal repeat (ITR). In this case, the promoter may be a CMV promoter, and the 2A self-cleaving peptide may be P2A or T2A. In addition, the CMV promoter may be the sequence of SEQ ID NO: 37, the P2A may be the sequence of SEQ ID NO: 38, and the T2A may be the sequence of SEQ ID NO: 39.

The pharmaceutical composition may be formulated as an injectable preparation. In this case, the AAV contained in the pharmaceutical composition may be administered to a subject in a dose ranging from $1\times10^5$ GC/kg to $1\times10^{15}$ GC/kg. In addition, the AAV may be administered to a subject in a dose ranging from $1\times10^6$ GC/kg to $1\times10^{12}$ GC/kg.

The pharmaceutical composition may be administered to an injured spinal cord region of a subject having a spinal cord injury disorder. In this case, the injured spinal cord region may be selected from the group consisting of L1 (lumbar 1), L2 (lumbar 2), L3 (lumbar 3), L4 (lumbar 4), L5 (lumbar 5), T1 (thoracic 1), T2 (thoracic 2), T3 (thoracic 3), T4 (thoracic 4), T5 (thoracic 5), T6 (thoracic 6), T7 (thoracic 7), T8 (thoracic 8), T9 (thoracic 9), T10 (thoracic 10), T11 (thoracic 11), T12 (thoracic 12), T13 (thoracic 13), C1 (cervical 1), C2 (cervical 2), C3 (cervical 3), C4 (cervical 4), C5 (cervical 5), C6 (cervical 6), C7 (cervical 7), C8 (cervical 8), S1 (sacrum 1), S2 (sacrum 2), S3 (sacrum 3), and S4 (sacrum 4). The pharmaceutical composition may be administered to a subject in a volume ranging from 1 µL to 1 mL per administration.

According to another aspect of the present disclosure, in order to solve the above-described objects, there is provided a pharmaceutical composition for treating a spinal cord injury disorder, the composition comprising a nucleic acid sequence encoding a Gsta4 (Glutathione S-Transferase Alpha 4) protein.

At this time, the nucleic acid sequence encoding the Gsta4 protein may be contained in a vector. The nucleic acid sequence encoding the Gsta4 protein may be the sequence of SEQ ID NO: 12.

In addition, the vector may further comprise a promoter sequence. In this case, the promoter sequence may be the sequence of SEQ ID NO: 37.

[Advantageous Effects]

According to the present disclosure, the following effects may be achieved.

According to the present disclosure, a composition for treating spinal cord injury disorders may be provided. **In** particular, the composition comprises a Gsta4 (Glutathione S-transferase A4) protein or a nucleic acid sequence encoding the Gsta4 protein, which serves as a differentiation factor for directly converting somatic cells into motor neurons.

In one embodiment, the composition comprises a Gsta4 protein or a nucleic acid encoding the same, an Hb9 protein or a nucleic acid sequence encoding the same, and an Lhx3 protein or a nucleic acid sequence encoding the same, as differentiation factors for directly converting somatic cells into motor neurons.

According to the present disclosure, a method for treating a spinal cord injury disorder using the composition may be provided.

[Brief Description of Drawings]

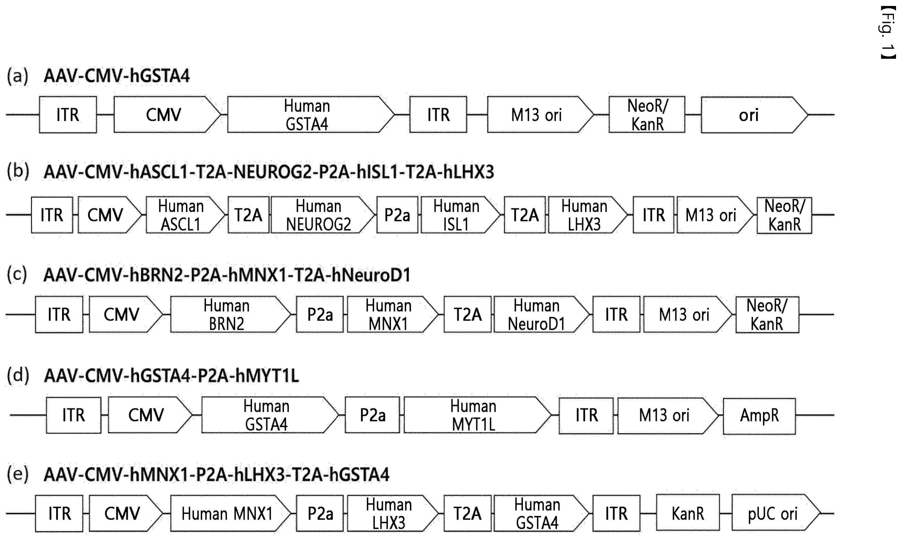

Fig. 1 is a schematic diagram of AAV vectors used in the experimental examples.
(a) is a schematic diagram of an AAV vector comprising Gsta4; (b) is a schematic diagram of an AAV vector comprising Ascl1, Isl1, Lhx3, and Ngn2 (also referred to as Neurog2); (c) is a schematic diagram of an AAV vector comprising Brn2, Hb9 (also referred to as Mnx1 (Motor neuron and pancreas homeobox 1)), and NeuroD1; (d) is a schematic diagram of an AAV vector comprising Gsta4 and Myt1I; and (e) is a schematic diagram of an AAV vector comprising Gsta4, Hb9, and Lhx3.

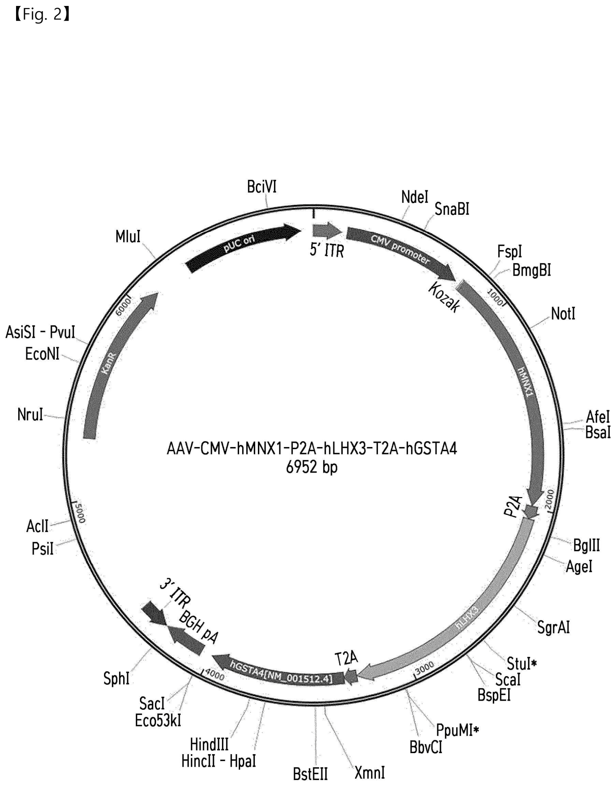

Fig. 2 is a schematic diagram of an AAV vector used in the experimental examples, which is a detailed schematic diagram of the AAV vector comprising Gsta4, Hb9, and Lhx3 as shown in Fig. 1(e).

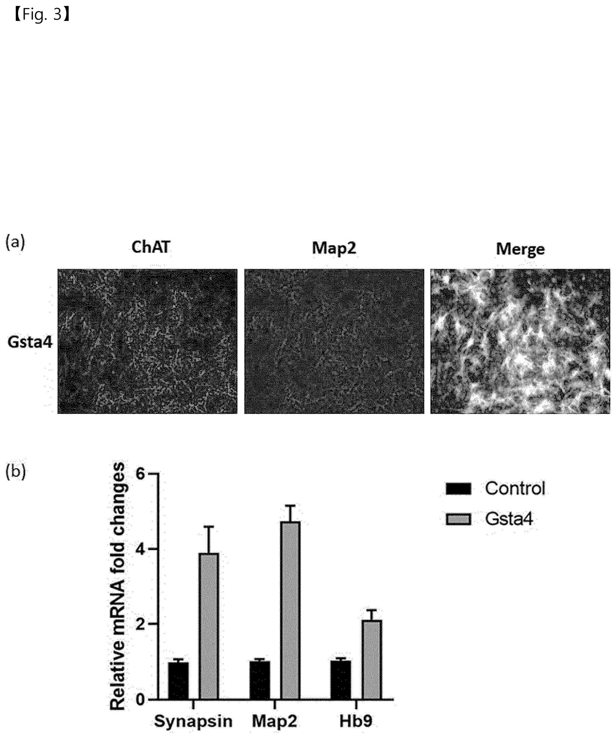

Fig. 3 shows the results of confirming the extent of direct conversion into motor neurons by introducing the direct conversion factor Gsta4 into mouse-derived fibroblasts, based on the expression of neuronal markers ChAT and Map2. In this case, the control refers to a negative control in which no treatment was performed. Fig. 3(a) shows the results of immunofluorescence staining, and Fig. 3(b) quantifies the results shown in Fig. 3(a).

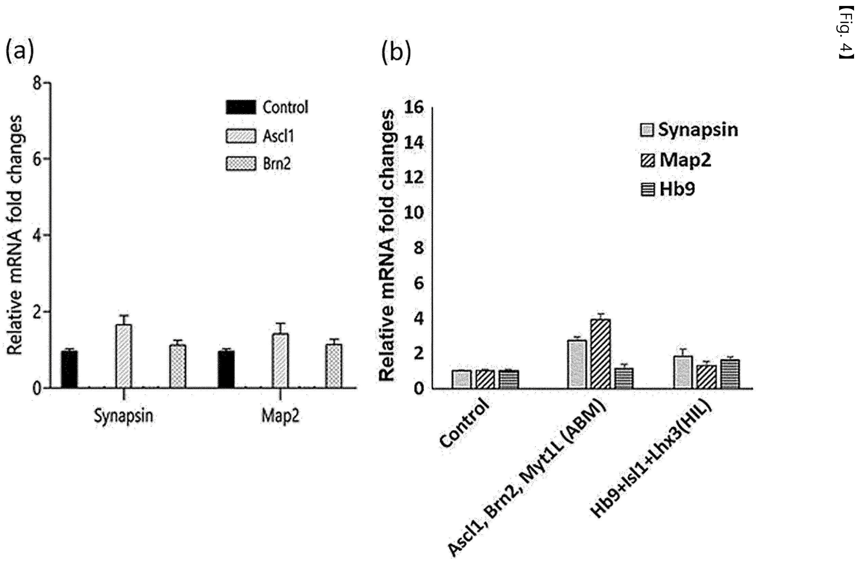

Fig. 4 shows the results of confirming the extent of direct conversion into motor neurons by introducing a composition comprising conventional direct conversion factors into fibroblasts, based on changes in the expression of neuronal markers (Synapsin, Map2, and Hb9). Fig. 4(a) shows the results obtained by introducing the composition into mouse-derived fibroblasts, and Fig. 4(b) shows the results obtained by introducing the composition into human-derived fibroblasts.

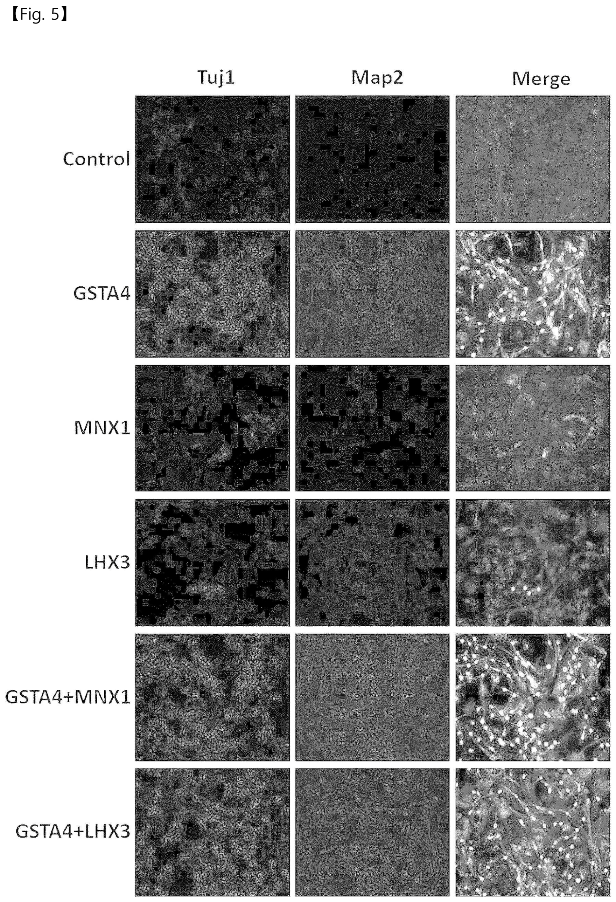

Fig. 5 shows the results of confirming the expression of neuronal markers Tuj1 and Map2 after introducing direct conversion factors into mouse-derived primary astrocytes. In this case, the direct conversion factors used were Gsta4 alone, MNX1 (also referred to as Hb9) alone, Lhx3 alone, a combination of Gsta4 and MNX1, and a combination of Gsta4 and Lhx3.

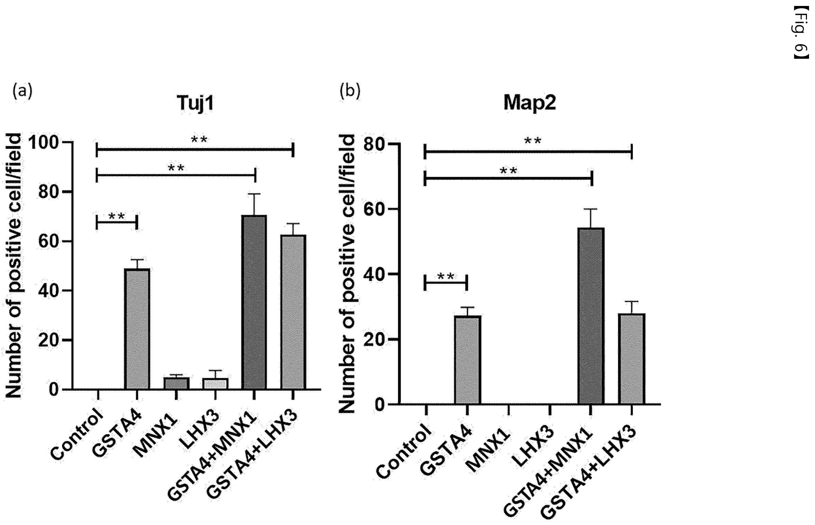

Fig. 6 is a graph quantifying the results shown in Fig. 5.

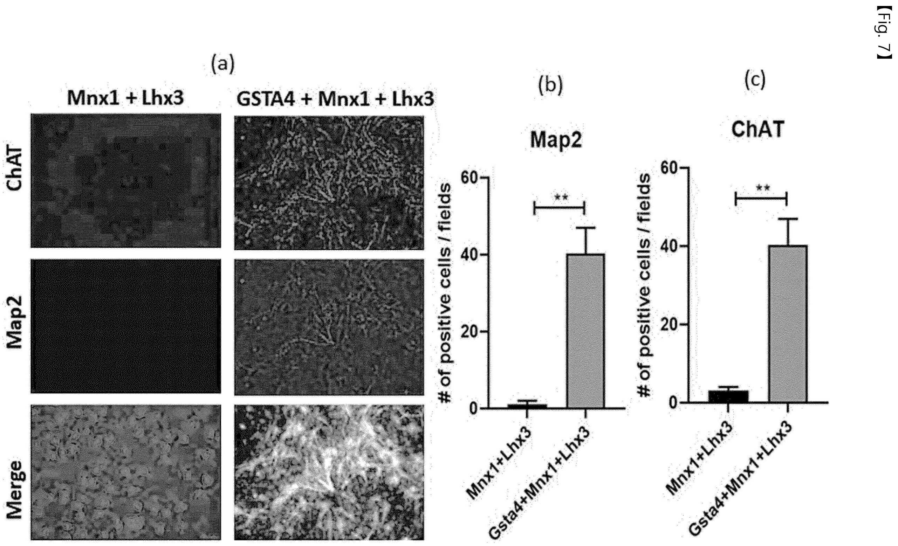

Fig. 7 shows the results of confirming the extent of direct conversion into motor neurons by introducing a composition comprising differentiation factors into mouse-derived fibroblasts, based on the expression of neuronal markers (ChAT and Map2) determined by immunofluorescence staining. In this case, the differentiation factors used were a combination of Mnx1 and Lhx3, and a combination of Gsta4, Mnx1, and Lhx3. Fig. 7(a) shows the results of immunofluorescence staining, and Fig. 7(b) and Fig. 7(c) are graphs quantifying the results shown in Fig. 7(a).

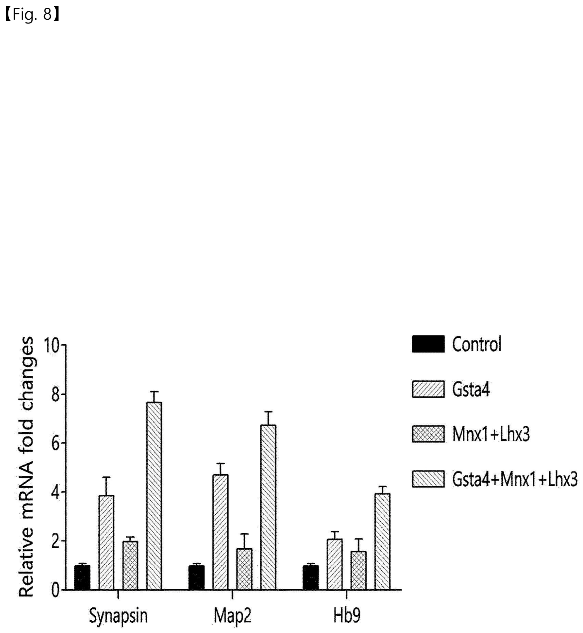

Fig. 8 shows the results of confirming the extent of direct conversion into motor neurons by introducing a composition comprising differentiation factors into mouse-derived fibroblasts, based on the expression of neuronal markers Synapsin, Map2, and Hb9. In this case, the differentiation factors used were Gsta4 alone, a combination of Mnx1 and Lhx3, and a combination of Gsta4, Mnx1, and Lhx3.

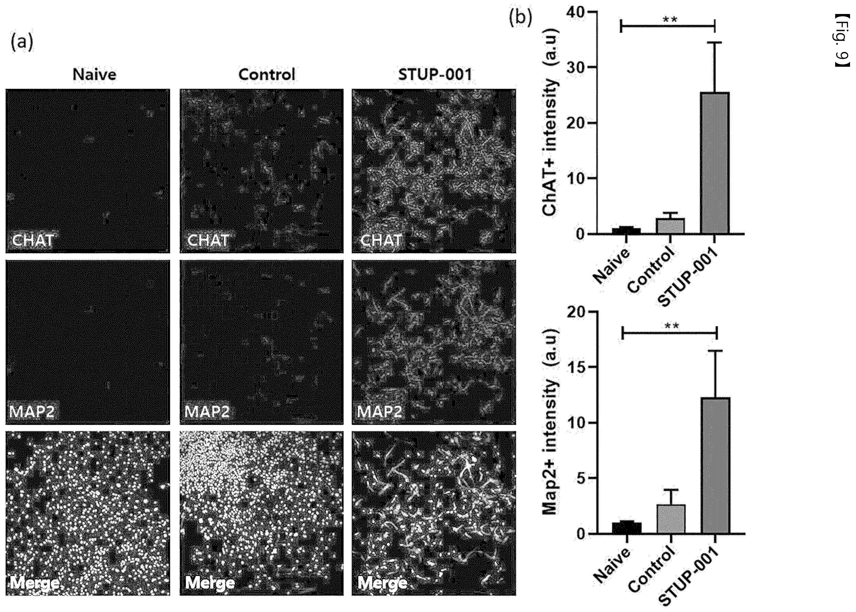

Fig. 9 shows the results of confirming the extent of differentiation into motor neurons by introducing a composition comprising differentiation factors into mouse-derived primary astrocytes, based on the expression of neuronal markers Tuj1 and Map2. "Naive" refers to a negative control in which no substance was administered, and "control" refers to a vehicle control in which an AAV2 buffer was administered. "STUP-001" refers to a composition comprising Gsta4, Hb9, and Lhx3.

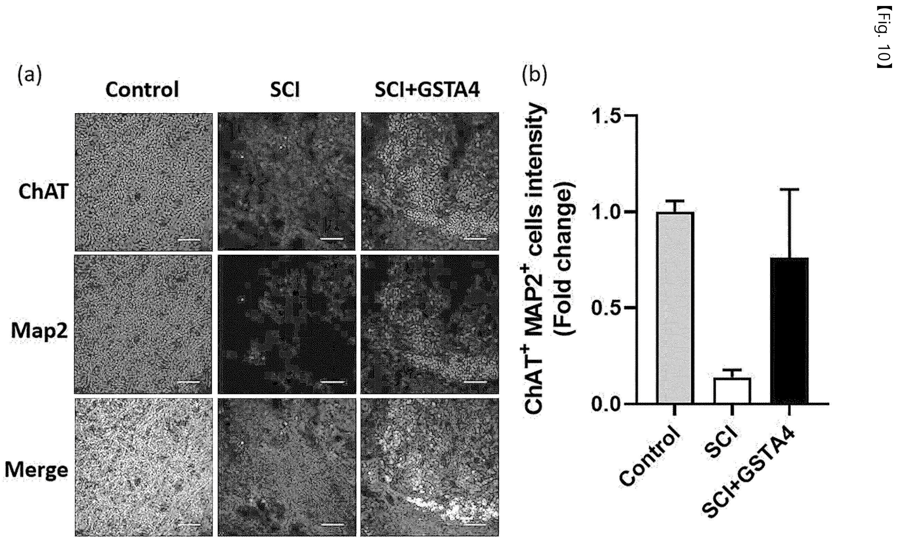

Fig. 10 shows the results of confirming changes in the expression of motor neuron markers ChAT and Map2 after administering a composition comprising Gsta4 to the spinal cord injury site of an SCI (spinal cord injury) mouse model, which is a paraplegia model induced by spinal cord injury. Fig. 10(a) shows the results of immunofluorescence staining, and Fig. 10(b) is a graph quantifying the results shown in Fig. 10(a).

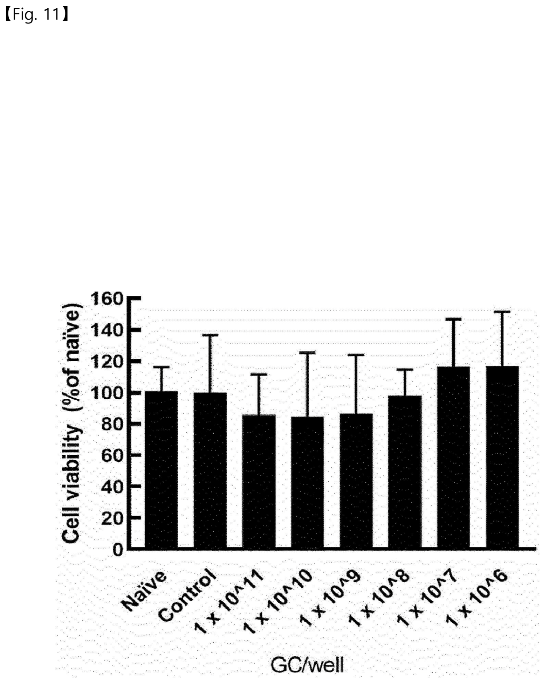

Fig. 11 shows the results of evaluating cell viability after treating mouse-derived primary astrocytes with various concentrations of a composition comprising Gsta4, MNX1, and Lhx3 (hereinafter referred to as "STUP-001"). In this case, "Naive" refers to a negative control in which no substance was administered, and "control" refers to a vehicle control in which an AAV2 buffer was administered.

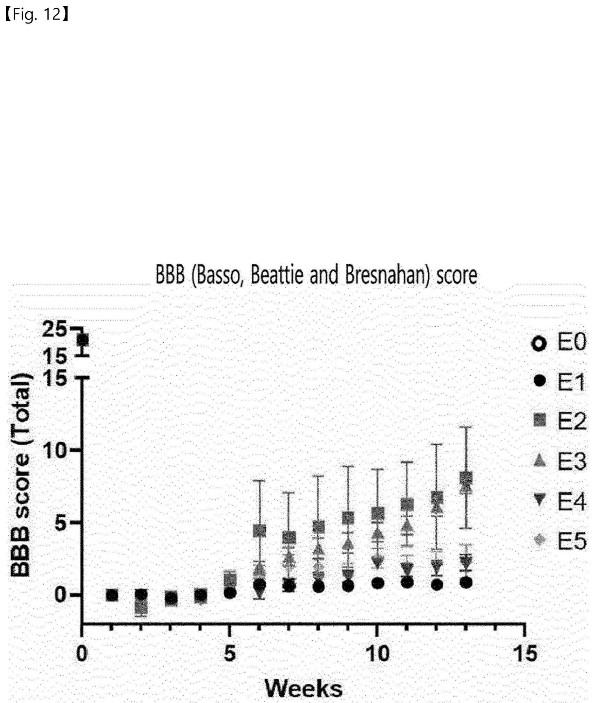

Figs. 12 to 15 show the results obtained by analyzing the Basso Beattie and Bresnahan (BBB) score, the action potential (AP) firing ratio, the resting membrane potential (RMP), and the efficacy-related protein quantity after treating an SCI mouse model with various concentrations of STUP-001.

In this case, "E0" refers to a normal ICR mouse as a control; "E1" refers to a vehicle control in which an AAV2 buffer was administered to an SCI mouse model; "E2" refers to a group in which STUP-001 was administered to an SCI mouse model at $2.0 \times 10^8$ GC/head; "E3" refers to a group in which STUP-001 was administered at $2.0 \times 10^7$ GC/head; "E4" refers to a group in which STUP-001 was administered at $2.0 \times 10^6$ GC/head; and "E5" refers to a group in which STUP-001 was administered at $2.0 \times 10^5$ GC/head.

[Best Mode for Carrying Out the Invention]

The present disclosure will be described in further detail below with reference to the accompanying drawings and the following description. The accompanying drawings illustrate embodiments of the present disclosure but do not encompass all possible embodiments. In addition, the present disclosure may be embodied in various forms and is not limited to the specific embodiments described herein. That is, the present disclosure may be modified in various ways and may include numerous experimental examples. The composition and characteristics of the present disclosure are described based on the experimental examples provided herein; however, the present disclosure is not limited thereto. It will be apparent to those skilled in the art to which the present disclosure pertains that various changes or modifications may be made within the spirit and scope of the present disclosure. Accordingly, such changes or modifications fall within the scope of the appended claims.

Unless otherwise defined, all technical and scientific terms used in the present specification have the same meaning as commonly understood by those skilled in the art to which the present disclosure pertains. Methods and materials similar or identical to those described in the present specification may be used in the practice or testing of the present disclosure. All publications, patent applications, patents, and other references cited herein are incorporated herein in their entirety by reference. In addition, the materials, methods, and experimental examples described herein are merely illustrative and are not intended to be limiting.

## Definition of Terms

### Spinal cord injury (SCI)

In the present disclosure, the term "spinal cord injury (SCI)" refers to damage occurring in the spinal cord, which is a part of the central nervous system located within the vertebral column. The term "spinal cord injury disorder" is interpreted to include damage to the spinal cord caused by disease or trauma, as well as disorders resulting therefrom. In particular, in the present disclosure, a representative example of spinal cord injury refers to a condition in which damage to motor neurons leads to abnormalities in motor nerve function. The term "spinal cord injury" may be used interchangeably with "injured spinal cord."

### Neuron

In the present disclosure, the term "neuron" refers to a nerve cell that constitutes the nervous system. Unlike other cell types, neurons are capable of transmitting signals through electrical mechanisms. Neurons are classified into sensory neurons that constitute the sensory nervous system, interneurons that constitute the central nervous system, and motor neurons that constitute the motor nervous system. All neurons exhibit electrical excitability. Before receiving a signal, neurons maintain a resting membrane potential of approximately -70 mV. The present disclosure particularly relates to a technique for efficiently generating motor neurons (motor nerve cells).

### Induced motor neuron (iMN)

In the present disclosure, the term "induced motor neuron (iMN)" refers to a motor neuron that is induced (generated) through direct conversion. The induced motor neuron refers to an artificially produced motor neuron obtained by directly converting somatic cells or the like using the novel differentiation factors of the present disclosure. The induced motor neuron performs the same functions as motor neurons present in vivo (i.e., naturally occurring motor neurons rather than artificially generated ones). For example, the induced motor neuron may transmit motor signals generated in the brain or spinal cord to muscles; however, this is merely an example and is not intended to limit the present disclosure. The term "induced motor neuron" may be used interchangeably with "motor neuron" or "iMN."

### Direct reprogramming / Direct conversion / Transdifferentiation

In the present disclosure, the term "direct reprogramming," "direct conversion," or "transdifferentiation" refers to converting a differentiated cell into a desired cell type by introducing specific factors. In particular, direct reprogramming induces conversion between mature cell types that are entirely different from each other. In other words, it refers to converting a somatic cell into a desired cell type without passing through a pluripotent state. The terms "direct reprogramming," "direct conversion," "cell converting," and "transdifferentiation" may be used interchangeably.

### Direct conversion factor / Converting factor

In the present disclosure, the term "direct conversion factor" refers to a factor involved in the above-described direct conversion. The factor may be a gene, a compound, a protein, a nucleic acid, or the like. The term "direct conversion factor" may be used interchangeably with "converting factor."

**Treatment**

In the present disclosure, the term "treatment" refers to alleviating a disease, disorder, or symptom, or inhibiting the progression thereof. The treatment refers to any act in which a condition is improved or favorably altered by the composition of the present disclosure or by the direct conversion factors.

**About**

In the present disclosure, the term "about" refers to a variation of approximately 30, 25, 20, 15, 10, 9, 8, 7, 6, 5, 4, 3, 2, or 1% with respect to a referenced quantity, level, value, number, frequency, percentage, dimension, size, amount, weight, or length.

Hereinafter, the invention disclosed in the present disclosure will be described in further detail.

**1. Composition for Treating Spinal Cord Injury Disorders**

Overview of the composition for treating spinal cord injury disorders

One aspect of the present disclosure relates to a composition for treating spinal cord injury disorders. In particular, the composition for treating spinal cord injury disorders of the present disclosure comprises direct conversion factors.

Hereinafter, the mechanism of spinal cord injury, conventional treatments for spinal cord injury, Gsta4, and the active ingredients of the composition will be described.

Spinal Cord Injury

The invention disclosed in the present disclosure is intended for treating spinal cord injury disorders.

The spinal cord is composed of neurons, oligodendrocytes, astrocytes, microglia, and the like.

When the spinal cord is damaged, the cells that constitute the spinal cord undergo different fates. Neurons and oligodendrocytes undergo programmed cell death and consequently die. In contrast, astrocytes and microglia do not die; instead, they become activated, rapidly increase in number, and enlarge in cell size. In addition, these activated cells secrete various substances that interfere with the recovery of the injured spinal cord.

In particular, the mechanisms of cell death in neurons and glial cells are closely associated with the loss of neuronal function, and it is known that necrosis-induced loss of neurons occurs at all stages of spinal cord injury (Cell death in models of spinal cord injury. Prog Brain Res. 2002;137:37-47).

Accordingly, in order to restore or treat spinal cord injury, it is important to regenerate the neurons that have undergone cell death and thereby recover neuronal function. In particular, the invention of the present disclosure restores or treats spinal cord injury by effectively generating and regenerating motor neurons at the site of spinal cord injury.

Disorders Associated with Spinal Cord Injury

In the present disclosure, the term "spinal cord injury disorder" encompasses both damage to the spinal cord, which is part of the central nervous system located within the vertebral column, and disorders resulting therefrom.

Spinal cord injury disorders may include, for example, traumatic spinal cord injury; Lou Gehrig's disease (amyotrophic lateral sclerosis, ALS); primary lateral sclerosis (PLS); multifocal motor neuropathy (MMN); Werdnig-Hoffmann disease; spinal muscular atrophy (SMA); Kugelberg-Welander syndrome; stroke; ataxia telangiectasia; familial motor neuron disease; bulbar palsy; paraplegia; tetraplegia; hemiplegia; quadriplegia; diplegia; and cerebral palsy. Preferably, the spinal cord injury disorder is selected from traumatic spinal cord injury, ALS, paraplegia, tetraplegia, hemiplegia, quadriplegia, diplegia, and cerebral palsy.

Problems with Conventional Treatments for Spinal Cord Injury

As described above, no effective therapeutic agent for treating spinal cord injury has been reported to date.

However, studies have been conducted with the aim of transplanting cells or promoting axonal regeneration to treat spinal cord injury.

To regenerate the neural tissue lost due to spinal cord injury, cell-based therapeutics have been investigated. Research has mainly focused on the transplantation of Schwann cells, neural progenitor cells, and stem cells. However, inhibitory effects caused by glial scarring have been reported to limit the growth of axons after transplantation. In addition, in the case of stem cells, the risk of teratoma or tumor formation has been raised as a significant concern. Moreover, there is insufficient evidence to support whether the transplanted cells differentiate into neurons, and the survival rate of the transplanted cells is extremely low, resulting in very limited therapeutic efficacy.

In addition to such cell-based therapies, antibodies and small-molecule compounds have also been developed for the treatment of spinal cord injury. In the central nervous system, regeneration is suppressed following injury, and oligodendrocyte-associated axonal inhibitors are particularly known to act as major suppressors of axonal growth. Accordingly, studies have focused primarily on inhibitors of axonal growth. For example, inhibitors of the Rho/ROCK pathway and chondroitinase ABC (ChABC), an enzyme that degrades chondroitin sulfate proteoglycan (CSPG), which suppresses axonal regeneration, have been investigated. However, small-molecule compounds and similar agents not only have toxicity issues, but also regenerate axons of residual neurons near the injury site. As a result, they may provide only temporary improvement of spinal cord function and are difficult to employ as fundamental therapeutic agents.

Composition for Treating Spinal Cord Injury According to the Present Disclosure

The composition for treating spinal cord injury disorders of the present disclosure comprises direct conversion factors as active ingredients.

In particular, the direct conversion factors of the present disclosure have the ability to directly convert somatic cells into motor neurons. The direct conversion is characterized in that somatic cells are converted into motor neurons without undergoing a dedifferentiation step into induced pluripotent stem cells.

The composition for treating spinal cord injury disorders of the present disclosure converts somatic cells in the body of a patient to whom the composition is administered-for example, somatic cells at the site of spinal cord injury-into motor neurons. As a result, a large number of motor neurons can be generated and regenerated at the injured spinal cord site. The somatic cells may be selected from fibroblasts, epithelial cells, endothelial cells, muscle cells, neurons, hair cells, hair root cells, hair follicle cells, oral epithelial cells, somatic cells isolated from urine, gastric mucosal cells, goblet cells, gastrin cells (G cells), B cells, parietal cells, astrocytes, blood cells, and oligodendrocyte progenitor cells.

Specifically, the composition for treating spinal cord injury disorders of the present disclosure essentially comprises a Gsta4 (glutathione S-transferase alpha 4) protein or a nucleic acid encoding the same.

Conventional Functions of Gsta4

Gsta4 is an enzyme belonging to the glutathione S-transferase (GST) family. In mammalian cytosol, eight classes of GSTs-alpha, kappa, mu, omega, pi, sigma, theta, and zeta-are known to be produced. Among these, the alpha class is located on chromosome 6 and includes GSTA1, GSTA2, GSTA3, GSTA4, and GSTA5.

The Gsta4 protein, also referred to as GTA4, is known to be involved in cellular defense mechanisms, detoxification, and anticancer effects. It is also known to play a significant role in reducing cellular stress by decreasing reactive oxygen species. In addition, the absence of Gsta4 has been reported to be negatively associated with diseases such as Parkinson's disease and Alzheimer's disease. However, the function of Gsta4 as a direct conversion factor between cell types, particularly its ability to induce transdifferentiation into motor neurons, has not been reported.

Novel Function of Gsta4 in the Present Invention

The composition for treating spinal cord injury disorders of the present disclosure essentially comprises Gsta4 as an active ingredient that enables direct conversion.

The inventors of the present disclosure have identified a novel function of Gsta4-namely, its role as a direct conversion factor for inducing somatic cells to become motor neurons-which is distinct from the conventionally known functions of Gsta4.

As demonstrated in the experimental examples of the present disclosure, Gsta4 induces the direct conversion of somatic cells into motor neurons, and furthermore, contributes to the recovery of injured spinal cord tissue in animal models of spinal cord injury.

Accordingly, the term "Gsta4" as used herein is preferably interpreted as referring to Gsta4 having the function of a direct conversion factor.

Function of Gsta4 in Spinal Cord Injury

As described above, when the spinal cord is injured, neurons and oligodendrocytes undergo cell death, while astrocytes and microglia become activated and increase in number. In particular, when astrocytes proliferate, a glial scar is formed, which suppresses axonal regeneration and regrowth. Consequently, signal transmission between neurons is blocked.

The Gsta4 protein of the present disclosure functions to directly convert abnormally increased astrocytes into motor neurons. By acting in this manner at the site of spinal cord injury, Gsta4 not only facilitates neuronal signal transmission that has been blocked, but also contributes to the direct regeneration or recovery of damaged motor neurons, thereby enabling the treatment of spinal cord injury.

In particular, since the composition for treating spinal cord injury according to the present disclosure comprises GSTA4, which can directly convert somatic cells such as astrocytes into motor neurons, it offers the advantage of being applicable in vivo. This is expected to overcome various drawbacks of conventional cell-based therapies for spinal cord injury, which generally require ex vivo manipulation prior to application.

One composition for treating spinal cord injury disorders according to the present disclosure comprises Gsta4 alone as an active ingredient.

Another composition for treating spinal cord injury disorders according to the present disclosure comprises Gsta4 and one or more known direct conversion factors as active ingredients.

Examples of Known Direct Conversion Factors

Examples of known direct conversion factors used for direct conversion include: Ascl1 (Achaete-scute homolog 1); Nurr1 (Nuclear receptor subfamily 4 group A member 2); Lmx1a (LIM homeobox transcription factor 1 alpha); Foxa2 (Forkhead box protein A2); Brn2 (POU class 3 homeobox 2); Sox2 (sex-determining region Y-box 2); Foxg1 (Forkhead box G1); Lhx3 (LIM homeobox 3); Hb9 (Homeobox HB9; also referred to as Mnx1 (motor neuron and pancreas homeobox 1)); Isl1 (ISL LIM homeobox 1); Ngn2 (neurogenin 2; also referred to as Neurog2); NeuroD1 (neuronal differentiation 1); Myt1I (myelin transcription factor 1-like); NeuroD2 (neuronal differentiation 2); miR-9 (microRNA-9); miR-124 (microRNA-124); Zic1 (Zic family member 1); Gata5 (GATA-binding protein 5); Hnf4$\alpha$ (hepatocyte nuclear factor 4 alpha); Hnf1$\alpha$ (hepatocyte nuclear factor 1 alpha); Foxa1 (Forkhead box A1); Foxa3 (Forkhead box A3); Tbx5 (T-box transcription factor 5); Mef2c (myocyte enhancer factor 2C); Oct4 (octamer-binding transcription factor 4); PRDM16 (PR/SET domain 16); MyoD (myoblast determination protein 1); Klf4 (Kruppel-like factor 4); and c-Myc (cellular Myc) (Mol. Cell. 2012 Sep 28;47(6):827-838).

In particular, the known direct conversion factors used in the present disclosure may be one or more factors selected from the group consisting of Ascl1, Brn2, Myt1I, Hb9, Isl1, Lhx3, Ngn2, and NeuroD1.

Hereinafter, specific examples of the active ingredients included in the composition for treating spinal cord injury disorders of the present disclosure are described; however, the present disclosure is not limited thereto.

For convenience, the compositional examples described below are categorized as comprising Gsta4 alone, Gsta4 in combination with one known direct conversion factor, or Gsta4 in combination with two known direct conversion factors; however, the number of known direct conversion factors included in the composition is not limited thereto. In other words, Gsta4 may be further combined with one to eight of the aforementioned "direct conversion factors used for inducing direct conversion into motor neurons."

In particular, the exemplary compositions necessarily comprise the aforementioned Gsta4, which serves as an essential active ingredient for the treatment of spinal cord injury disorders. In addition, one or more known direct conversion factors may also function as active ingredients of the composition of the present disclosure.

Compositional Examples

Compositional Example (1): Gsta4 Alone

The composition for treating spinal cord injury disorders according to the present disclosure may comprise a Gsta4 protein or a nucleic acid sequence encoding the same.

Compositional Example (2): Gsta4 in Combination with One Known Direct Conversion Factor

The composition for treating spinal cord injury disorders according to the present disclosure may comprise a Gsta4 protein or a nucleic acid sequence encoding the same, and may further comprise one known direct conversion factor.

For example, the known direct conversion factor may be one protein selected from the group consisting of Ascl1, Brn2, Myt1I, Hb9, Isl1, Lhx3, Ngn2, and NeuroD1, or a nucleic acid sequence encoding the selected protein.

By way of example, the composition for treating spinal cord injury disorders may comprise a Gsta4 protein or a nucleic acid sequence encoding the same, and may comprise an Ascl1 protein or a nucleic acid sequence encoding the Ascl1 protein.

By way of example, the composition for treating spinal cord injury disorders may comprise a Gsta4 protein or a nucleic acid sequence encoding the same, and may comprise a Brn2 protein or a nucleic acid sequence encoding the Brn2 protein.

By way of example, the composition for treating spinal cord injury disorders may comprise a Gsta4 protein or a nucleic acid sequence encoding the same, and may comprise an Hb9 protein or a nucleic acid sequence encoding the Hb9 protein.

By way of example, the composition for treating spinal cord injury disorders may comprise a Gsta4 protein or a nucleic acid sequence encoding the same, and may comprise an Lhx3 protein or a nucleic acid sequence encoding the Lhx3 protein.

Compositional Example (3): Gsta4 in Combination with Two Known Direct Conversion Factors

The composition for treating spinal cord injury disorders according to the present disclosure may comprise a Gsta4 protein or a nucleic acid encoding the same, and may further comprise two known direct conversion factors.

For example, the two known direct conversion factors may be two proteins selected from the group consisting of Ascl1, Brn2, Myt1I, Hb9, Isl1, Lhx3, Ngn2, and NeuroD1, or nucleic acid sequences encoding the selected proteins.

By way of example, the composition for treating spinal cord injury disorders may comprise a Gsta4 protein or a nucleic acid sequence encoding the same; an Hb9 protein or a nucleic acid sequence encoding the Hb9 protein; and an Ngn2 protein or a nucleic acid sequence encoding the Ngn2 protein.

By way of example, the composition for treating spinal cord injury disorders may comprise a Gsta4 protein or a nucleic acid sequence encoding the same; an Lhx3 protein or a nucleic acid sequence encoding the Lhx3 protein; and an Ngn2 protein or a nucleic acid sequence encoding the Ngn2 protein.

By way of example, the composition for treating spinal cord injury disorders may comprise a Gsta4 protein or a nucleic acid sequence encoding the same; an Hb9 protein or a nucleic acid sequence encoding the Hb9 protein; and an Isl1 protein or a nucleic acid sequence encoding the Isl1 protein.

By way of example, the composition for treating spinal cord injury disorders may comprise a Gsta4 protein or a nucleic acid sequence encoding the same; an Ngn2 protein or a nucleic acid sequence encoding the Ngn2 protein; and an Isl1 protein or a nucleic acid sequence encoding the Isl1 protein.

Preferably, the composition for treating spinal cord injury disorders comprises a Gsta4 protein or a nucleic acid sequence encoding the same; an Hb9 protein or a nucleic acid sequence encoding the Hb9 protein; and an Lhx3 protein or a nucleic acid sequence encoding the Lhx3 protein.

Combination Ratio of Gsta4 and Known Direct Conversion Factors

As described above, **in** the composition for treating spinal cord injury disorders, Gsta4 and the known direct conversion factors may be combined and used in various ratios as needed by a person skilled in the art.

At this time, Gsta4 and the one or more known direct conversion factors may be used in the same ratio or in different ratios relative to each other.

For example, when Gsta4 is used in combination with one known direct conversion factor, the two components may be used at a ratio of 1:1, 1:2, or 2:1.

In one specific example, when Gsta4 and Hb9 are used, the two components may be used at a ratio of 1:1, 2:1, or 1:2.

In one specific example, when Gsta4 and Lhx3 are used, the two components may be used at a ratio of 1:1, 2:1, or 1:2.

As another example, when Gsta4 is used in combination with two known direct conversion factors, the three components may be used at a ratio of 1:1:1, 2:1:1, 2:1:2, or 1:2:1.

In one specific example, when Gsta4, Hb9, and Lhx3 are used, the three components may be used at a ratio of 1:1:1, 2:1:1, 2:1:2, or 1:2:1.

Form of the Direct Conversion Factors

The direct conversion factors included in the composition for treating spinal cord injury disorders of the present disclosure may be in the form of a protein, a nucleic acid, or a mixture of a protein and a nucleic acid, but are not limited thereto.

Forms of Gsta4

The Gsta4 may be derived from a mammal. For example, the mammal may be a human, dog, horse, cat, mouse, rat, pig, rabbit, sheep, monkey, or chimpanzee. Preferably, the Gsta4 is human-derived.

The composition of the present disclosure may comprise a Gsta4 protein.

The Gsta4 protein may consist of a wild-type amino acid sequence, or may be a variant in which one or more amino acids of the wild-type amino acid sequence are deleted, substituted, or inserted.

The amino acid sequence of the wild-type Gsta4 protein may be obtained from publicly available databases. The Gsta4 protein of the present disclosure may have an amino acid sequence that exhibits at least 60% sequence identity to the wild-type amino acid sequence. The sequence identity may be a value arbitrarily selected from any range defined by two numbers among about 60%, 65%, 70%, 75%, 80%, 85%, 90%, 95%, and 100%. Preferably, the Gsta4 protein may have about 90% to 100% sequence identity to the wild-type amino acid sequence.

By way of example, the Gsta4 protein for direct conversion according to the present disclosure may have the amino acid sequence set forth in SEQ ID NO: 1.

SEQ ID NO: 1: maarpklhyp ngrgrmesvr wvlaaagvef deefletkeq lyklqdgnhl lfqqvpmvei dgmklvqtrs ilhyiadkhn lfgknlkert lidmyvegtl dllellimhp flkpddqqke wnmaqkaii ryfpvfekil rghgqsflvg nqlsladvil lqtilaleek ipnilsafpf lqeytvklsn iptikrflep gskkkpppde iyvrtvynif rp

In another embodiment, the Gsta4 protein may have an amino acid sequence that exhibits 70% to 100% sequence identity to the amino acid sequence set forth in SEQ ID NO: 1.

In addition, the composition of the present disclosure may comprise a nucleic acid encoding the Gsta4 protein. The nucleic acid may be DNA or RNA.

The nucleic acid encoding the Gsta4 protein may have a wild-type nucleic acid sequence, or may have a sequence in which one or more nucleotides of the wild-type nucleic acid sequence are deleted, substituted, or inserted.

The wild-type Gsta4 nucleic acid, for example a DNA sequence, may be obtained from publicly available databases.

The Gsta4 nucleic acid sequence of the present disclosure may have a sequence that exhibits at least 60% sequence identity to the wild-type nucleic acid sequence. The sequence identity may be a value arbitrarily selected from any range defined by two numbers among about 60%, 65%, 70%, 75%, 80%, 85%, 90%, 95%, and 100%. Preferably, the nucleic acid encoding the Gsta4 protein may have about 90% to 100% sequence identity to the wild-type nucleic acid sequence.

The nucleic acid sequence may be a full sequence of the Gsta4 gene including intron and exon sequences.

Alternatively, the nucleic acid sequence may be a sequence that contains only exons. For example, the nucleic acid sequence encoding the Gsta4 protein may comprise a coding sequence (CDS) from which introns have been removed.

By way of example, the nucleic acid sequence may be a DNA sequence encoding the Gsta4 protein and a corresponding mRNA sequence that is the transcription product thereof.

As another example, the nucleic acid sequence may be an mRNA sequence corresponding to the exon sequence of the Gsta4 gene, which is the transcription product thereof.

In one specific example, the nucleic acid encoding the Gsta4 protein may comprise the full DNA sequence of the Gsta4 gene, which is set forth in SEQ ID NO: 2.

SEQ ID NO: 2: gctttgtgcg gctccaggcc tccgagtgga ctccagaaag cctgaaaagc tatcatggca

gcaaggccca agctccacta tcccaacgga agaggccgga tggagtccgt gagatgggtt ttagctgccg ccggagtcga

gtttgatgaa gaatttctgg aaacaaaaga acagttgtac aagttgcagg atggtaacca cctgctgttc caacaagtgc

ccatggttga aattgacggg atgaagttgg tacagacccg aagcattctc cactacatag cagacaagca caatctcttt

ggcaagaacc tcaaggagag aaccctgatt gacatgtacg tggaggggac actggatctg ctggaactgc ttatcatgca

tcctttctta aaaccagatg atcagcaaaa ggaagtggtt aacatggccc agaaggctat aattagatac tttcctgtgt

ttgaaaagat tttaaggggt cacggacaaa gctttcttgt tggtaatcag ctgagccttg cagatgtgat tttactccaa

accattttag ctctagaaga gaaaattcct aatatcctgt ctgcatttcc tttcctccag gaatacacag tgaaactaag

taatatccct acaattaaga gattccttga acctggcagc aagaagaagc ctcccctga tgaaatttat gtgagaaccg

tctacaacat ctttaggcca taaaacaaca catccatgtg tgagtgacag tgtgttccta gagatggtat tgtctacagt

catgtcttaa tggatcccag ctctgtcatg gtgctatcta tgtattaagt tgggtcctaa gttgggtctt ttgtgtcaac

gagatcatct cttctagaaa tatcaacctt ttttgtccag taaataattg ttaggggatc tttattggaa aactttttg

gagaggctgg tatttaagtt agatctgatt gggctactca tgtcctgtag ccagttcatc ctcataataa gaatgggcag

gatctcttgt tctctcctga gtgtctttct actctcctga gcgtctttct gctctcctta tcctgttctc ttatccttat cccctccagt

ctctgcctaa tttttagtgt ttaataacaa ccgaatgtct agtaaatgac tctcctctga gctgtaataa ataaaatggt

agtaatgaat gcaatcagta ttagccaaaa taaagaattt atgagtcatt

In another specific example, the nucleic acid encoding the Gsta4 protein may have the coding sequence (CDS) set forth in SEQ ID NO: 12.

SEQ ID NO: 12:

Atggcagcaaggcccaagctccactatcccaacggaagaggccggatggagtccgtgagatgggttttagctgccg

ccggagtcgagtttgatgaagaatttctggaaacaaaagaacagttgtacaagttgcaggatggtaaccacctgctgttccaacaa

gtgcccatggttgaaattgacgggatgaagttggtacagacccgaagcattctccactacatagcagacaagcacaatctctttggc

aagaacctcaaggagagaaccctgattgacatgtacgtggaggggacactggatctgctggaactgcttatcatgcatcctttctta

aaaccagatgatcagcaaaaggaagtggttaacatggcccagaaggctataattagatactttcctgtgtttgaaaagattttaagg

ggtcacggacaaagctttcttgttggtaatcagctgagccttgcagatgtgattttactccaaaccattttagctctagaagagaaaat

tcctaatatcctgtctgcatttcctttcctccaggaatacacagtgaaactaagtaatatccctacaattaagagattccttgaacctggc

agcaagaagaagcctcccctgatgaaatttatgtgagaaccgtctacaacatctttaggccataa

In another embodiment, the nucleic acid encoding the Gsta4 protein may comprise a DNA sequence that exhibits 90% to 100% sequence identity to the DNA sequence set forth in SEQ ID NO: 2.

In another embodiment, the nucleic acid encoding the Gsta4 protein may comprise a sequence that exhibits 90% to 100% sequence identity to the coding sequence (CDS) set forth in SEQ ID NO: 12.

Forms of the Known Direct Conversion Factors

The known direct conversion factors may be derived from a mammal. For example, the mammal may be a human, dog, horse, cat, mouse, rat, pig, rabbit, sheep, monkey, or chimpanzee. Preferably, the factors are human-derived.

The composition of the present disclosure may further comprise, in addition to the Gsta4 protein, one or more known direct conversion factors in protein form.

When the known direct conversion factor is in protein form, it may consist of a wild-type amino acid sequence, or may be a variant in which one or more amino acids of the wild-type amino acid sequence are deleted, substituted, or inserted.

The wild-type amino acid sequence may be obtained from publicly available databases. The direct conversion factor protein of the present disclosure may have an amino acid sequence that exhibits at least 60% sequence identity to the wild-type amino acid sequence. The sequence identity may be a value arbitrarily selected from any range defined by two numbers among about 60%, 65%, 70%, 75%, 80%, 85%, 90%, 95%, and 100%. Preferably, the known direct conversion factor protein may have about 90% to 100% sequence identity to the wild-type amino acid sequence.

By way of example, the known direct conversion factor protein for direct conversion according to the present disclosure may have an amino acid sequence set forth in any one of the following SEQ ID NOs:

In another embodiment, the known direct conversion factor protein may comprise a sequence that exhibits 70% to 100% sequence identity to any one of the amino acid sequences set forth in the following SEQ ID NOs:

the amino acid sequence of the Ascl1 protein is set forth in SEQ ID NO: 17;

the amino acid sequence of the Brn2 protein is set forth in SEQ ID NO: 19;

the amino acid sequence of the Myt1l protein is set forth in SEQ ID NO: 21;

the amino acid sequence of the Hb9 (also referred to as Mnx1) protein is set forth in SEQ ID NO: 13;

the amino acid sequence of the Isl1 protein is set forth in SEQ ID NO: 23;

the amino acid sequence of the Lhx3 protein is set forth in SEQ ID NO: 15;

the amino acid sequence of the Ngn2 (also referred to as Neurog2) protein is set forth in SEQ ID NO: 25; and

the amino acid sequence of the NeuroD1 protein is set forth in SEQ ID NO: 27.

In addition, the composition of the present disclosure may comprise a nucleic acid encoding the known direct conversion factor protein. The nucleic acid may be DNA or RNA.

The nucleic acid encoding the known direct conversion factor protein may have a wild-type nucleic acid sequence, or may comprise a sequence in which one or more nucleotides of the wild-type nucleic acid sequence are deleted, substituted, or inserted.

The wild-type nucleic acid of the known direct conversion factor, for example a DNA sequence, may be obtained from a publicly available database.

The nucleic acid sequence of the known direct conversion factor according to the present disclosure may have a sequence identity of at least 60% with a wild-type nucleic acid sequence. In this context, the sequence identity may be a value within a range defined by any two numbers selected from about 60%, 65%, 70%, 75%, 80%, 85%, 90%, 95%, and 100%. Preferably, the nucleic acid sequence encoding the known direct conversion factor protein may have a sequence identity of about 90% to 100% with the wild-type nucleic acid sequence. The nucleic acid sequence may be a full-length sequence of a gene of the known direct conversion factor, including both intron and exon sequences.

Alternatively, the nucleic acid sequence may be a sequence comprising only exons. For example, the nucleic acid sequence encoding the known direct conversion factor protein may comprise a coding sequence (CDS) from which introns have been removed.

For example, the nucleic acid sequence may be a DNA sequence encoding the known direct conversion factor protein, or an mRNA sequence corresponding thereto (i.e., a transcription product).

Alternatively, the nucleic acid sequence may be an mRNA sequence corresponding to the exon sequence of the gene encoding the known direct conversion factor (i.e., a transcription product).

In one embodiment, the nucleic acid encoding the known direct conversion factor protein may comprise a sequence selected from the following Sequence Identification Numbers:

In another embodiment, the nucleic acid encoding the known direct conversion factor protein may comprise a sequence having about 90% to 100% sequence identity to a sequence selected from the following Sequence Identification Numbers:

The nucleic acid sequence encoding the Ascl1 protein being the sequence of SEQ ID NO: 18;

the nucleic acid encoding the Brn2 protein being the sequence of SEQ ID NO: 20;

the nucleic acid encoding the Myt1L protein being the sequence of SEQ ID NO: 22;

the nucleic acid encoding the Hb9 (also referred to as Mnx1) protein being the sequence of SEQ ID NO: 14;

the nucleic acid encoding the Isl1 protein being the sequence of SEQ ID NO: 24;

the nucleic acid encoding the Lhx3 protein being the sequence of SEQ ID NO: 16;

the nucleic acid encoding the Ngn2 (also referred to as Neurog2) protein being the sequence of SEQ ID NO: 26; and

the nucleic acid encoding the NeuroD1 protein being the sequence of SEQ ID NO: 28.

Form of the Active Ingredient Contained in the Composition of the Present Disclosure

The active ingredient contained in the composition for treating a spinal cord injury disorder according to the present disclosure may be in the form of a protein, a nucleic acid sequence, or a mixture of a protein and a nucleic acid sequence, without being limited thereto. In this context, the nucleic acid may be DNA, RNA, or a mixture of DNA and RNA.

The active ingredient of the composition for treating a spinal cord injury disorder according to the present disclosure may be a GSTA4 protein or a nucleic acid sequence encoding the same. In one embodiment, the active ingredient of the composition for treating a spinal cord injury disorder may be a nucleic acid sequence encoding a GSTA4 protein.

The active ingredient of the composition for treating a spinal cord injury disorder according to the present disclosure may be a GSTA4 protein or a nucleic acid sequence encoding the same; and one or more conventional direct-conversion factor proteins or one or more nucleic acid sequences encoding such conventional direct-conversion factors.

In one embodiment, the active ingredient of the composition for treating a spinal cord injury disorder according to the present disclosure may be a nucleic acid sequence encoding a Gsta4 protein; a nucleic acid sequence encoding an Hb9 protein; and a nucleic acid sequence encoding an Lhx3 protein.

Hereinafter, the active ingredient of the composition for treating a spinal cord injury disorder will be described with respect to various forms of nucleic acid sequences. This description is provided merely for convenience of explanation in terms of compositions comprising nucleic acid sequences and is not intended to be limiting.

In one example, when the active ingredient of the composition for treating a spinal cord injury disorder is in the form of a nucleic acid sequence, it may be contained in a vector.

Form of Active Ingredient: Vector

In another aspect of the present disclosure, the composition for treating a spinal cord injury disorder may include a vector comprising a nucleic acid sequence of a direct-conversion factor, which enables expression of the direct-conversion factor as an active ingredient in vivo.

Essential Factor

The vector of the present disclosure necessarily comprises a nucleic acid sequence encoding a GSTA4 protein.

The nucleic acid sequence encoding the GSTA4 protein may be a full-length sequence of the GSTA4 gene or a partial sequence thereof.

For example, the partial sequence of the GSTA4 gene may be an exon sequence of GSTA4. In this case, the exon sequence may be a coding sequence (CDS) from which introns are removed within the GSTA4 gene.

In one example, the vector may comprise the sequence of SEQ ID NO: 2 or SEQ ID NO: 12. In another example, the vector may comprise a sequence having 70% to 100% homology to the sequence of SEQ ID NO: 2 or SEQ ID NO: 12.

Optional Factor

The vector of the present disclosure may further comprise one or more nucleic acid sequences encoding conventional direct-conversion factors, in addition to the nucleic acid sequence encoding the GSTA4 protein.

The nucleic acid sequence encoding the conventional direct-conversion factor may be a full-length sequence or a partial sequence of the conventional direct-conversion factor. For example, the partial sequence of the conventional direct-conversion factor may be an exon sequence of the conventional direct-conversion factor.

In one example, the vector may optionally further comprise one or more nucleic acid sequences encoding one or more factors selected from Ascl1, Brn2, Myt1L, Hb9, Isl1, Lhx3, Ngn2, and NeuroD1.

When the vector further comprises the optional factor, the vector may be constituted as one vector or as two or more vectors. The vector may comprise the essential factor and the optional factor within a single vector, or may comprise them across two or more vectors. That is, independent vectors may be used according to the number of differentiation factors, or two or more differentiation factors may be combined and included within a single vector.

Examples of Vectors

The vector necessarily includes the above-described essential factor, and the optional factor may be combined in various manners as described below, without being limited thereto.

Embodiment (1): Vector Containing Only the Essential Factor

The vector may comprise a nucleic acid sequence encoding the GSTA4 protein.

For example, the vector may comprise the sequence of SEQ ID NO: 2 or SEQ ID NO: 12.

In another example, the vector may comprise a sequence having 90% to 100% homology to the sequence of SEQ ID NO: 2 or SEQ ID NO: 12.

Embodiment (2): Vector Containing the Essential Factor and One Optional Factor

The vector may comprise a nucleic acid sequence encoding the GSTA4 protein and a nucleic acid sequence encoding one conventional direct-conversion factor.

For example, the vector may comprise a nucleic acid sequence encoding the GSTA4 protein and a nucleic acid sequence encoding the Ascl1 protein. In one embodiment, the vector may comprise SEQ ID NOs: 2 and 18. In another embodiment, the vector may comprise SEQ ID NOs: 12 and 18.

For example, the vector may comprise a nucleic acid sequence encoding the GSTA4 protein and a nucleic acid sequence encoding the Brn2 protein. In one embodiment, the vector may comprise SEQ ID NOs: 2 and 20. In another embodiment, the vector may comprise SEQ ID NOs: 12 and 20.

For example, the vector may comprise a nucleic acid sequence encoding the GSTA4 protein and a nucleic acid sequence encoding the Hb9 protein. In one embodiment, the vector may comprise SEQ ID NOs: 2 and 14. In another embodiment, the vector may comprise SEQ ID NOs: 12 and 14.

For example, the vector may comprise a nucleic acid sequence encoding the GSTA4 protein and a nucleic acid sequence encoding the Lhx3 protein. In one embodiment, the vector may comprise SEQ ID NOs: 2 and 16. In another embodiment, the vector may comprise SEQ ID NOs: 12 and 16.

Embodiment (3): Vector Containing the Essential Factor and Two Optional Factors

The vector may comprise a nucleic acid sequence encoding the GSTA4 protein and nucleic acid sequences encoding two conventional direct-conversion factors.

For example, the vector may comprise a nucleic acid sequence encoding the GSTA4 protein, a nucleic acid sequence encoding the Hb9 protein, and a nucleic acid sequence encoding the Ngn2 protein. In one embodiment, the vector may comprise SEQ ID NOs: 2, 14, and 26. In another embodiment, the vector may comprise SEQ ID NOs: 12, 14, and 26.

For example, the vector may comprise a nucleic acid sequence encoding the GSTA4 protein, a nucleic acid sequence encoding the Lhx3 protein, and a nucleic acid sequence encoding the Ngn2 protein. In one embodiment, the vector may comprise SEQ ID NOs: 2, 16, and 26. In another embodiment, the vector may comprise SEQ ID NOs: 12, 16, and 26.

For example, the vector may comprise a nucleic acid sequence encoding the GSTA4 protein, a nucleic acid sequence encoding the Hb9 protein, and a nucleic acid sequence encoding the Isl1 protein. In one embodiment, the vector may comprise SEQ ID NOs: 2, 14, and 24. In another embodiment, the vector may comprise SEQ ID NOs: 12, 14, and 24.

For example, the vector may comprise a nucleic acid sequence encoding the GSTA4 protein, a nucleic acid sequence encoding the Ngn2 protein, and a nucleic acid sequence encoding the Isl1 protein. In one embodiment, the vector may comprise SEQ ID NOs: 2, 26, and 24. In another embodiment, the vector may comprise SEQ ID NOs: 12, 26, and 24.

For example, the vector may comprise a nucleic acid sequence encoding the GSTA4 protein, a nucleic acid sequence encoding the Hb9 protein, and a nucleic acid sequence encoding the Lhx3 protein. In one embodiment, the vector may comprise SEQ ID NOs: 2, 14, and 16. In another embodiment, the vector may comprise SEQ ID NOs: 12, 14, and 16.

Expression Elements of the Vector

The vector may further comprise expression elements required for the expression of the target protein, in addition to the above-described essential and optional components.

For example, the expression elements may include a "regulatory element" that controls the expression of the differentiation factor. In addition, the expression elements may further comprise, as needed, a "selection element" that facilitates selection of the differentiation factor.

The following describes exemplary regulatory and selection elements of the vector; however, the vector may further comprise any element known in the art for effectively expressing a target protein therein.

Regulatory Elements

The regulatory element may be selected from a promoter, an enhancer, a polyadenylation signal, a Kozak consensus sequence, an inverted terminal repeat (ITR), a long terminal repeat (LTR), a terminator, an origin of replication, a multicloning site (MCS), an internal ribosome entry site (IRES), and 2A self-cleaving peptides.

For example, the promoter may be selected from an SV40 early promoter, an LTR (mouse mammary tumor virus long terminal repeat) promoter, an Ad MLP (adenovirus major late) promoter, an HSV (herpes simplex virus) promoter, a CMV (cytomegalovirus) promoter, an RSV (rous sarcoma virus) promoter, a U6 promoter, a CBA promoter, a PGK promoter, and a CAG promoter.

For example, the 2A self-cleaving peptide may be selected from T2A, P2A, E2A, and F2A. The vector for expressing the differentiation factor may comprise one or more 2A self-cleaving peptides. In this case, the 2A self-cleaving peptide generates multiple proteins from the same transcript. Accordingly, one or more 2A self-cleaving peptides may be positioned between two or more different proteins intended to be expressed by the vector.

Selection Elements

The selection element may be selected from a fluorescent protein gene, a tag, a reporter gene, and an antibiotic resistance gene.

For example, the fluorescent protein gene may be a GFP gene, a YFP gene, an RFP gene, or an mCherry gene.

For example, the tag may be selected from a histidine (His) tag, a V5 tag, a FLAG tag, an influenza hemagglutinin (HA) tag, a Myc tag, a VSV-G tag, and a thioredoxin (Trx) tag.

For example, the reporter gene may be selected from glutathione-S-transferase (GST), horseradish peroxidase (HRP), chloramphenicol acetyltransferase (CAT), β-galactosidase, and β-glucuronidase.

For example, the antibiotic resistance gene may be selected from an ampicillin resistance gene, a hygromycin resistance gene, a neomycin resistance gene, a kanamycin resistance gene, a blasticidin resistance gene, and a zeocin resistance gene.

Specific Embodiments of the Vector

The following provides exemplary descriptions of vectors comprising the above-described essential components, optional components, and expression elements; however, the invention is not limited thereto.

The embodiments described below are merely illustrative examples, and a person skilled in the art may modify the design as necessary for the expression of the target protein.

Example (1): Vector Containing GSTA4 Alone

In one example of the present disclosure, the vector may comprise a promoter and a nucleic acid sequence encoding the GSTA4 protein. In this case, the nucleic acid sequence encoding the GSTA4 protein may be derived from a human. Preferably, the nucleic acid sequence encoding the GSTA4 protein may be SEQ ID NO: 2 or SEQ ID NO: 12. The promoter may be selected from a CBA promoter, a PGK promoter, a CMV promoter, and a CAG promoter. Preferably, the promoter may be a CMV promoter. In one embodiment, the sequence of the promoter may be the sequence represented by SEQ ID NO: 37.

In one embodiment, the vector of the present disclosure may comprise SEQ ID NO: 37 and SEQ ID NO: 2.

In another embodiment, the vector of the present disclosure may comprise SEQ ID NO: 37 and SEQ ID NO: 12.

Example (2): Vector Containing GSTA4 and One Conventional Direct-Conversion Factor

In another example of the present disclosure, the vector may comprise a promoter, a nucleic acid sequence encoding the GSTA4 protein, and one nucleic acid sequence encoding a conventional direct-conversion factor.

By way of example, the vector may comprise a promoter, a nucleic acid sequence encoding the GSTA4 protein, and a nucleic acid sequence encoding the Ascl1 protein.

The vector may comprise a promoter, a nucleic acid sequence encoding the GSTA4 protein, and a nucleic acid sequence encoding the Brn2 protein.

The vector may comprise a promoter, a nucleic acid sequence encoding the GSTA4 protein, and a nucleic acid sequence encoding the MNX1 (Motor neuron and pancreas homeobox 1; also referred to as Hb9) protein.

The vector may comprise a promoter, a nucleic acid sequence encoding the GSTA4 protein, and a nucleic acid sequence encoding the Lhx3 protein.

In this case, the nucleic acid sequence encoding the GSTA4 protein, the nucleic acid sequence encoding the Ascl1 protein, the nucleic acid sequence encoding the Brn2 protein, the nucleic acid sequence encoding the Hb9 protein, and the nucleic acid sequence encoding the Lhx3 protein may be derived from a human.

In one embodiment, for example, the nucleic acid sequence encoding the GSTA4 protein may be SEQ ID NO: 2 or SEQ ID NO: 12.

For example, the nucleic acid sequence encoding the Ascl1 protein may be SEQ ID NO: 18.

For example, the nucleic acid sequence encoding the Brn2 protein may be SEQ ID NO: 20.

For example, the nucleic acid sequence encoding the Hb9 (MNX1) protein may be SEQ ID NO: 14.

For example, the nucleic acid sequence encoding the Lhx3 protein may be SEQ ID NO: 16.

In this case, the promoter contained in the vector of Embodiment (2) may be selected from a CBA promoter, a PGK promoter, a CMV promoter, and a CAG promoter. Preferably, the promoter may be a CMV promoter. In one embodiment, the CMV promoter sequence may be the sequence represented by SEQ ID NO: 37.

In this case, the vector of Embodiment (2) may optionally further comprise a 2A self-cleaving peptide. In particular, inclusion of a 2A self-cleaving peptide in the vector of the present application may be the case where the vector comprises nucleic acid sequences encoding two or more different proteins intended to be expressed therein. In such cases, the 2A self-cleaving peptide may be positioned between the two or more different proteins.

The 2A self-cleaving peptide may be selected from T2A, P2A, E2A, and F2A. In one embodiment, the 2A self-cleaving peptide may be T2A or P2A. For example, P2A may be the sequence of SEQ ID NO:38, and T2A may be the sequence of SEQ ID NO:39.

In one example, the vector may comprise a promoter; a nucleic acid sequence encoding the GSTA4 protein; a nucleic acid sequence encoding T2A; and a nucleic acid sequence encoding the Lhx3 protein.

The vector may comprise a promoter; a nucleic acid sequence encoding the GSTA4 protein; a nucleic acid sequence encoding P2A; and a nucleic acid sequence encoding the Lhx3 protein.

The vector may comprise a promoter; a nucleic acid sequence encoding the GSTA4 protein; a nucleic acid sequence encoding T2A; and a nucleic acid sequence encoding the Hb9 protein.

The vector may comprise a promoter; a nucleic acid sequence encoding the GSTA4 protein; a nucleic acid sequence encoding P2A; and a nucleic acid sequence encoding the Hb9 protein.

As specific examples of Example (2), the vector may comprise the following sequences:

Specific examples of a vector comprising (promoter, Gsta4, and MNX1) include:

SEQ ID NO:37, SEQ ID NO:12, and SEQ ID NO:14;
SEQ ID NO:37, SEQ ID NO:2, and SEQ ID NO:14;

Specific examples of a vector comprising (promoter, Gsta4, 2A, and MNX1) include:

SEQ ID NO:37, SEQ ID NO:12, SEQ ID NO:38, and SEQ ID NO:14;
SEQ ID NO:37, SEQ ID NO:12, SEQ ID NO:39, and SEQ ID NO:14;
SEQ ID NO:37, SEQ ID NO:2, SEQ ID NO:38, and SEQ ID NO:14;
SEQ ID NO:37, SEQ ID NO:2, SEQ ID NO:39, and SEQ ID NO:14;

Specific examples of a vector comprising (promoter, Gsta4, and Lhx3) include:

SEQ ID NO:37, SEQ ID NO:12, and SEQ ID NO:16;
SEQ ID NO:37, SEQ ID NO:2, and SEQ ID NO:16;

Specific examples of a vector comprising (a promoter, Gsta4, 2A, and Lhx3) include:

SEQ ID NO:37, SEQ ID NO:12, SEQ ID NO:38, and SEQ ID NO:16;
SEQ ID NO:37, SEQ ID NO:12, SEQ ID NO:39, and SEQ ID NO:16;
SEQ ID NO:37, SEQ ID NO:2, SEQ ID NO:38, and SEQ ID NO:16;
SEQ ID NO:37, SEQ ID NO:2, SEQ ID NO:39, and SEQ ID NO:16.

Example 3: Vector comprising GSTA4 and two conventional direct-conversion factors

In another embodiment of the present disclosure, the vector may comprise a nucleic acid sequence encoding the GSTA4 protein and two nucleic acid sequences encoding conventional direct-conversion factors.

By way of example, the vector may comprise a nucleic acid sequence encoding the GSTA4 protein; a nucleic acid sequence encoding the Hb9 protein; and a nucleic acid sequence encoding the Lhx3 protein.

In this instance, the nucleic acid sequence encoding the GSTA4 protein, the nucleic acid sequence encoding the Hb9 protein, and the nucleic acid sequence encoding the Lhx3 protein may be of human origin.

For example, the nucleic acid sequence encoding the GSTA4 protein may be the sequence of SEQ ID NO: 2 or 12.

For example, the nucleic acid sequence encoding the Hb9 protein may be the sequence of SEQ ID NO: 14.

For example, the nucleic acid sequence encoding the Lhx3 protein may be the sequence of SEQ ID NO: 16.

In this case, the promoter contained in the vector of Embodiment (3) may be selected from a CBA promoter, a PGK promoter, a CMV promoter, or a CAG promoter. Preferably, the promoter is a CMV promoter. In one embodiment, the CMV promoter sequence is the sequence represented by SEQ ID NO: 37.

The vector may optionally further include a 2A self-cleaving peptide. Preferably, the 2A self-cleaving peptide may be selected from T2A, P2A, E2A, and F2A. More preferably, the 2A self-cleaving peptide may be T2A or P2A. In one embodiment, P2A may be the sequence of SEQ ID NO: 38, and T2A may be the sequence of SEQ ID NO: 39. The 2A self-cleaving peptide may be positioned between three or more different proteins. In addition, the 2A self-cleaving peptide may be one or two in number. In particular, when two 2A self-cleaving peptides are included, they may be identical to or different from each other.

By way of example, the vector may include a promoter; a nucleic acid sequence encoding an Hb9 protein; a nucleic acid sequence encoding P2A; a nucleic acid sequence encoding an Lhx3 protein; and a nucleic acid sequence encoding a GSTA4 protein.

The vector may include a promoter; a nucleic acid sequence encoding an Hb9 protein; a nucleic acid sequence encoding T2A; a nucleic acid sequence encoding an Lhx3 protein; and a nucleic acid sequence encoding a GSTA4 protein.

The vector may include a promoter; a nucleic acid sequence encoding an Hb9 protein; a nucleic acid sequence encoding an Lhx3 protein; a nucleic acid sequence encoding P2A; and a nucleic acid sequence encoding a GSTA4 protein.

The vector may include a promoter; a nucleic acid sequence encoding an Hb9 protein; a nucleic acid sequence encoding an Lhx3 protein; a nucleic acid sequence encoding T2A; and a nucleic acid sequence encoding a GSTA4 protein.

The vector may include a promoter; a nucleic acid sequence encoding an Hb9 protein; a nucleic acid sequence encoding P2A; a nucleic acid sequence encoding an Lhx3 protein; a nucleic acid sequence encoding T2A; and a nucleic acid sequence encoding a GSTA4 protein.

The vector may include a promoter; a nucleic acid sequence encoding an Hb9 protein; a nucleic acid sequence encoding T2A; a nucleic acid sequence encoding an Lhx3 protein; a nucleic acid sequence encoding P2A; and a nucleic acid sequence encoding a GSTA4 protein.

The vector may include a promoter; a nucleic acid sequence encoding an Hb9 protein; a nucleic acid sequence encoding P2A; a nucleic acid sequence encoding an Lhx3 protein; a nucleic acid sequence encoding P2A; and a nucleic acid sequence encoding a GSTA4 protein.

The vector may include a promoter; a nucleic acid sequence encoding an Hb9 protein; a nucleic acid sequence encoding T2A; a nucleic acid sequence encoding an Lhx3 protein; a nucleic acid sequence encoding T2A; and a nucleic acid sequence encoding a GSTA4 protein.

As specific examples of the vector of Example (3), the vector may comprise the following sequences:
Specific examples of a vector comprising (promoter, Gsta4, MNX1, and Lhx3) include:

SEQ ID NOs: 37, 12, 14, and 16;

SEQ ID NOs: 37, 2, 14, and 16;

Specific examples of a vector comprising (promoter, MNX1, 2A, Lhx3, and Gsta4) include:

SEQ ID NOs: 37, 14, 38, 16, and 2;

SEQ ID NOs: 37, 14, 39, 16, and 2;

SEQ ID NOs: 37, 14, 38, 16, and 12;

SEQ ID NOs: 37, 14, 39, 16, and 12;

Specific examples of a vector comprising (promoter, MNX1, Lhx3, 2A, and Gsta4) include:

SEQ ID NOs: 37, 14, 16, 38, and 2;

SEQ ID NOs: 37, 14, 16, 39, and 2;

SEQ ID NOs: 37, 14, 16, 38, and 12;

SEQ ID NOs: 37, 14, 16, 39, and 12;

Specific examples of a vector comprising (promoter, MNX1, 2A, Lhx3, 2A, and Gsta4) include:

SEQ ID NOs: 37, 14, 38, 16, 39, and 2;

SEQ ID NOs: 37, 14, 38, 16, 39, and 12;

SEQ ID NOs: 37, 14, 39, 16, 38, and 2;

SEQ ID NOs: 37, 14, 39, 16, 38, and 12;

SEQ ID NOs: 37, 14, 38, 16, 39, and 12;

SEQ ID NOs: 37, 14, 39, 16, 38, and 12.

Type of Vector

The vector may be a viral vector.

In one example, the viral vector may be selected from the group consisting of retrovirus, lentivirus, adenovirus, adeno-associated virus (AAV), vaccinia virus, poxvirus, human immunodeficiency virus (HIV), murine leukemia virus (MLV), avian sarcoma/leukosis virus (ASLV), spleen necrosis virus (SNV), Rous sarcoma virus (RSV), mouse mammary tumor virus (MMTV), herpes simplex virus (HSV), episomal virus, or simplexed virus. Preferably, the viral vector is an adeno-associated virus.

Alternatively, the vector may be a non-viral vector.

In one example, the non-viral vector may be a plasmid, phage, naked DNA, a DNA-lipid complex, a DNA-polymer complex, or mRNA.

Function of the vector: carrier

The vector described above serves as a carrier for delivering the direct conversion factor for treating spinal cord injury according to the present disclosure into a cell.

In particular, in the case of a viral vector, the nucleic acid sequence encoding the direct conversion factor is loaded onto the viral capsid, thereby enabling delivery into a cell.

Currently, the most widely used viral vectors include lentiviral vectors, adenoviral vectors, and adeno-associated viral vectors.

Lentiviral vectors are mainly utilized for ex vivo therapies in which cells harvested from a patient are modified in the laboratory prior to re-implantation.

Adenoviral vectors are capable of accommodating large genetic cargos; however, they exhibit the limitation of transient expression.

Adeno-associated virus (AAV) vectors are widely utilized as therapeutics because they can overcome the limitations associated with lentiviral and adenoviral vectors. AAV vectors are capable of infecting cells independently of cell division and, therefore, are extensively employed in the development of in vivo therapeutics. Consequently, AAV vectors are recognized as the safest and most effective viral vectors for delivering genes into cells, compared to other viral vector systems, and represent the only viral-vector-based therapeutic modality that has received approval from the U.S. FDA. In the present specification, the terms "AAV vector" and "AAV particle" are used interchangeably.

The composition for treating spinal cord injury according to the present disclosure may be delivered to the injured spinal cord region by incorporating a direct conversion factor into the AAV vector. That is, therapeutic efficacy attributable to the overproduction of motor neurons may be achieved by administering the AAV vector to the injured spinal cord region and inducing expression of the direct conversion factor in situ.

The composition comprising the AAV vector is described in further detail below.

A Composition Comprising an AAV Vector

Another aspect of the present disclosure relates to a composition for treating a spinal cord injury, the composition comprising an AAV vector.

The composition of the present disclosure for treating a spinal cord injury comprises an AAV vector.

In one example, the AAV vector comprises a nucleic acid sequence encoding a GSTA4 protein.

In another example, the AAV vector comprises a nucleic acid sequence encoding a GSTA4 protein and one or more nucleic acid sequences encoding respective conventional direct conversion factors.

Hereinafter, the structure and characteristics of AAV, an AAV vector comprising GSTA4, and an AAV vector comprising GSTA4 together with conventional direct conversion factors are described.

Structure and characteristics of adeno-associated virus (AAV)

An AAV used as a gene delivery vehicle comprises an icosahedral capsid with a diameter of approximately 18-25 nm and a single-stranded DNA genome of approximately 4.7-6 kb in length.

At both termini of the AAV genome, there is a hairpin structure referred to as an inverted terminal repeat (ITR). The ITR participates in the replication of the AAV genome and in the packaging of the AAV particle.

In addition, the AAV genome contains two open reading frames (ORFs), namely a replication (Rep) region and a capsid (Cap) region. These open reading frames respectively encode replication and capsid gene products. The replication and capsid gene products are involved in the replication, assembly, and packaging of complete AAV viral particles. The AAV replication region expresses four viral proteins, for example, Rep78, Rep68, Rep52, and Rep40. In addition, the AAV capsid region encodes at least three proteins, for example, VP1, VP2, and VP3.

Herein, the term "AAV vector" (also referred to as "AAV particle" or "AAV") is interpreted as comprising at least a capsid, an ITR, and the active ingredient of the present disclosure.

The ITR functions as a replication and packaging signal for vector generation, and the capsid protein forms the capsid to encapsulate the direct conversion factor DNA therein, while also determining tissue tropism so as to deliver the direct conversion factor DNA to target cells and tissues.

Serotypes of AAV

AAV comprises more than 20 serotypes and over 100 variants, which may be appropriately selected and used by a person skilled in the art as needed.

AAVs usable in the present disclosure may be selected from AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.

By way of example, the AAV may be AAV1, AAV2, AAV4, AAV5, AAV6, AAV8, AAV9, or AAV10. In one specific embodiment, the AAV may be AAV1, AAV2, AAV4, AAV9, or AAV10. In one embodiment, the AAV of the present disclosure may be AAV2.

In a preferred specific embodiment, the pharmaceutical composition for treating a spinal cord injury of the present disclosure may employ an AAV2 vector. That is, the pharmaceutical composition for treating a spinal cord injury of the present disclosure may comprise an AAV2 vector including a nucleic acid sequence encoding a direct conversion factor. **In** one embodiment of the present disclosure, the AAV2 vector comprises an inverted terminal repeat (ITR), a replication region, a capsid region, and a nucleic acid sequence encoding the direct conversion factor.

Advantages of the AAV Vector

In the present disclosure, when an AAV vector is used as a delivery vehicle for the active ingredient, the following advantages may be obtained.

Advantage (1): Since the AAV genome is not integrated into the host cell genome, safety issues such as genotoxicity are substantially minimized.

Advantage (2): The AAV vector persists in the target cell in a stable episomal state, enabling long-term expression of the gene.

Advantage (3): The AAV vector targets both dividing and non-dividing cells. Accordingly, it can be used to target tissues such as the central nervous system, muscle, and the eye, in which transfection is generally difficult. Thus, the AAV vector can be effectively applied to cells present at the site of spinal cord injury (central nervous system), which is the therapeutic target of the present disclosure.

Advantage (4): Because the AAV vector lacks viral structural genes, it does not elicit an immune response.

Advantage (5): The AAV vector can be produced at a high titer.

Hereinafter, specific examples of the composition for treating spinal cord injury according to the present disclosure-namely, an AAV vector comprising a nucleic acid sequence encoding the GSTA4 protein, and an AAV vector comprising a nucleic acid sequence encoding the GSTA4 protein and a nucleic acid sequence encoding a conventional differentiation factor-are described.

These descriptions are provided solely for convenience of explanation and are not intended to limit the scope of the present disclosure.

Examples of Compositions Comprising an AAV Vector

In one example, the composition for treating a spinal cord injury may comprise an AAV vector including a nucleic acid sequence encoding the GSTA4 protein.

In another example, the composition for treating a spinal cord injury may comprise an AAV vector including a nucleic acid sequence encoding the GSTA4 protein and a nucleic acid sequence encoding a conventional direct-conversion factor. The conventional direct-conversion factor may be selected from Ascl1, Brn2, Myt1L, Mnx1 (also referred to as Motor neuron and pancreas homeobox 1 or Hb9), Isl1, Lhx3, Ngn2, and NeuroD1. Preferably, the composition for treating a spinal cord injury comprises an AAV vector including a nucleic acid sequence encoding the GSTA4 protein, a nucleic acid sequence encoding the Mnx1 protein, and a nucleic acid sequence encoding the Lhx3 protein.

The AAV vector may be selected from AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.

In one example, the AAV vector may be AAV1, AAV2, AAV4, AAV5, AAV6, AAV8, AAV9, or AAV10. In a more specific example, the AAV vector may be AAV1, AAV2, AAV4, AAV9, or AAV10. In one embodiment, the AAV vector may be AAV2.

A method for preparing a composition for treating a spinal cord injury disorder

In another aspect of the present disclosure, there is provided a method for preparing the composition for treating a spinal cord injury disorder.

As described above, the composition for treating a spinal cord injury disorder may be provided in various forms, including a protein form, a nucleic acid form, or a mixed form of protein and nucleic acid. Methods for preparing such various forms may be carried out using methods well known to those skilled in the art.

However, hereinafter, by way of example, a method for preparing a composition for treating a spinal cord injury disease comprising an AAV vector is described, but the present disclosure is not limited to such description.

Method for Producing an AAV Vector

The method for producing the AAV vector of the present application may employ methods known in the art.

The method for producing an AAV vector is typically carried out using three or more plasmids.

For example, a method for producing an AAV vector may comprise:
introducing into a host cell a first plasmid comprising at least ITRs, a promoter, and a direct conversion factor; a second plasmid comprising a nucleic acid sequence encoding at least a replication protein and/or a capsid protein; and a third plasmid comprising a sequence encoding at least one helper gene; and isolating AAV particles from the host cell.

In this case, the AAV particles may be AAV2 particles.

The AAV particles refer to particles comprising at least a capsid, ITRs, and a direct conversion factor, that is, particles in which at least the ITRs and the direct conversion factor are encapsidated within the capsid.

In this regard, the method for producing the AAV vector may be carried out by introducing the first and second plasmids into the host cell without introducing the third plasmid.

In one specific example, the first plasmid may comprise at least ITRs, a promoter, and a nucleic acid sequence encoding a GSTA4 protein. In this case, the AAV particles may be AAV2 particles.

In another specific example, the first plasmid may comprise at least ITRs, a promoter, a nucleic acid sequence encoding a GSTA4 protein; a nucleic acid sequence encoding an Mnx1 protein; and a nucleic acid sequence encoding an Lhx3 protein.

In another specific example, the first plasmid may comprise at least ITRs, a promoter, a nucleic acid sequence encoding a GSTA4 protein; and a nucleic acid sequence encoding an Mnx1 protein.

In another specific example, the first plasmid may comprise at least ITRs, a promoter, a nucleic acid sequence encoding a GSTA4 protein; and a nucleic acid sequence encoding an Lhx3 protein.

The host cell may be a cell that can be infected by an AAV vector. For example, the host cell may be HEK293, HEK293T, Huh-7, HeLa, HepG2, Hep1A, SV40, CHO, COS, MeWo, NIH3T3, A549, PER.C6, HT1080, a monocyte, or a dendritic cell, but is not limited thereto. Preferably, the host cell may be a HEK293T cell.

The replication protein may be Rep78, Rep68, Rep52, Rep40, or the like.

The capsid protein may be VP1, VP2, VP3, or the like.

The helper gene may be E2A, E4, VA, or the like.

The promoter may be a CBA promoter, a PGK promoter, a CMV promoter, a CAG promoter, or the like.

The method for producing the AAV vector may further comprise culturing a host cell into which the first to third plasmids have been introduced.

In this case, the culturing may be performed until the first to third plasmids introduced into the host cell result in expression of the direct conversion factor(s). Such culturing conditions and duration may be appropriately determined by a person skilled in the art depending on, for example, the type of the direct conversion factor(s) and the type of the host cell.

Methods known in the art for introducing the plasmids into a host cell may be appropriately selected and performed by a person skilled in the art.

For example, electroporation, gene gun delivery, sonoporation, magnetofection, microinjection, transient cell compression or squeezing, cationic liposome methods, lithium acetate-DMSO methods, lipid-mediated transfection, calcium phosphate precipitation, lipofection, PEI (polyethyleneimine)-mediated transfection, and DEAE-dextran-mediated transfection may be used, but the methods are not limited thereto.

In this regard, the order in which the plasmids are introduced into the host cells is not particularly limited.

The AAV particles may be prepared in the form of exosomes or microparticles, but are not limited thereto. Methods for isolating the AAV particles may be performed using techniques well known in the art.

For example, methods for isolating AAV particles may include centrifugation, precipitation, immunoprecipitation, affinity chromatography, filtration, methods using magnetic beads coated with specific antibodies or aptamers, freeze-thaw methods, ultrasonic disruption, and the use of commercial kits.

AAV particles may optionally be concentrated using methods known in the art. For example, such concentration may be performed using immunomagnetic capture, organic flocculation, or polyethylene glycol (PEG) precipitation, without being limited thereto.

In addition, the AAV particles may optionally be further subjected to quality testing. For example, such quality testing may include titer determination, sterility testing for bacteria and fungi, and mycoplasma detection testing, without being limited thereto.

The nucleic acid sequences encoding the replication proteins and/or the capsid proteins contained in the plasmids introduced into the host cell form an AAV capsid, and nucleic acid sequences comprising ITRs, a promoter, and a nucleic acid sequence encoding a direct conversion factor are packaged inside the AAV capsid. The AAV particles extracted from the host cells into which such plasmids have been introduced penetrate into the nucleus of a target cell via receptors present on the surface of the target cell, and when the nucleic acid sequence encoding the direct conversion factor contained within the capsid is delivered to the target, the nucleic acid sequence encoding the direct conversion factor is expressed in vivo.

In a preferred specific embodiment, the AAV particle of the present application may be an AAV2 particle comprising the sequence of SEQ ID NO: 2 or SEQ ID NO: 12.

In another preferred specific embodiment, the AAV particle of the present application may be an AAV2 particle comprising the sequences of SEQ ID NO: 2, SEQ ID NO: 14, and SEQ ID NO: 16.

In another preferred specific embodiment, the AAV particle of the present application may be an AAV2 particle comprising the sequences of SEQ ID NO: 12, SEQ ID NO: 14, and SEQ ID NO: 16.

Various Uses of the Composition for Treating Spinal Cord Injury

Another aspect of the present application relates to various uses of a composition for treating spinal cord injury.

The composition for treating spinal cord injury may be used for pharmaceutical purposes, as a kit, or the like.

Hereinafter, pharmaceutical uses and kits will be described.

Use (1): Pharmaceutical Use

The composition of the present application may be utilized for treating a subject in need of treatment. For example, the composition for treating spinal cord injury may be used for pharmaceutical purposes.

Pharmaceutical Composition

In another aspect, the present application discloses the composition for treating spinal cord injury as a pharmaceutical composition.

The pharmaceutical composition may comprise the above-described active ingredient as an active ingredient of the pharmaceutical composition.

The active ingredient may be one of the following (1) or (2):

(1) a GSTA4 protein or a nucleic acid sequence encoding the same; or

(2) a GSTA4 protein or a nucleic acid sequence encoding the same; and a conventional direct conversion factor protein or a nucleic acid sequence encoding the same.

In this case, the conventional direct conversion factor may be one to eight factors selected from the group consisting of Ascl1, Brn2, Myt1L, Hb9 (MNX1), Isl1, Lhx3, Ngn2, and NeuroD1.

Preferably, the conventional direct conversion factor may be a nucleic acid sequence encoding an Hb9 (MNX1) protein or a nucleic acid sequence encoding an Lhx3 protein. Most preferably, the conventional direct conversion factor may be a nucleic acid sequence encoding an Hb9 (MNX1) protein and a nucleic acid sequence encoding an Lhx3 protein.

In addition, the indications of the pharmaceutical composition and the delivery of the active ingredient are the same as those described for the composition for treating spinal cord injury.

By way of example, the pharmaceutical composition of the present application may be an AAV vector comprising the active ingredient.

By way of example, the pharmaceutical composition of the present application may comprise an AAV vector including a nucleic acid sequence encoding GSTA4. In this case, the AAV vector may be an AAV2 vector or an AAV4 vector, and preferably, the AAV vector is an AAV2 vector.

As another example, the pharmaceutical composition of the present application may comprise an AAV vector including a nucleic acid sequence encoding GSTA4; a nucleic acid sequence encoding an Hb9 protein; and a nucleic acid sequence encoding an Lhx3 protein. **In** this case, the AAV vector may be an AAV2 vector or an AAV4 vector, and preferably, the AAV vector is an AAV2 vector.

As another example, the pharmaceutical composition of the present application may be an AAV particle comprising the active ingredient.

As a non-limiting example, the pharmaceutical composition of the present application may comprise an AAV particle comprising a nucleic acid sequence encoding GSTA4. **In** this case, the AAV particle may be an AAV2 particle or an AAV4 particle. Preferably, the AAV particle is an AAV2 particle.

As another non-limiting example, the pharmaceutical composition of the present application may comprise an AAV particle comprising a nucleic acid sequence encoding GSTA4, a nucleic acid sequence encoding the Hb9 protein, and a nucleic acid sequence encoding the Lhx3 protein. In this case, the AAV particle may be an AAV2 particle or an AAV4 particle. Preferably, the AAV particle is an AAV2 particle.

Additional Components of the Pharmaceutical Composition

The pharmaceutical composition may optionally further comprise, in addition to the active ingredient(s), one or more pharmaceutically acceptable additional components.

In the present application, the term "pharmaceutically acceptable" is used herein to refer to materials, compositions, and/or dosage forms which, within the scope of sound medical judgment, are suitable for use in contact with the tissues of humans and animals without causing excessive toxicity, irritation, allergic response, or other problems or complications, and which are commensurate with a reasonable benefit/risk ratio.

The pharmaceutically acceptable additional components may be physiologically acceptable components that contribute to stabilization, solubilization, absorption, or delivery efficiency of the active ingredient of the present application. For example, the pharmaceutically acceptable additional components may include carriers, excipients, diluents, preservatives, and the like, without being limited thereto.

For example, the above carriers, excipients, and diluents may include lactose, dextrose, sucrose, sorbitol, mannitol, xylitol, erythritol, maltitol, starch, acacia gum, alginate, gelatin, calcium phosphate, calcium silicate, cellulose, methyl cellulose, distilled water, physiological saline, glycerol, ethanol, HSA (human serum albumin), and the like.

For example, the preservatives may include benzoic acid, sodium benzoate, sorbic acid, paraoxybenzoic acid, chlorobutanol, and the like.

When the pharmaceutical composition is formulated, it may further comprise excipients such as fillers, bulking agents, binders, and wetting agents.

Formulation of the Pharmaceutical Composition

The pharmaceutical composition may be formulated for oral administration or parenteral administration.

For example, when formulated for oral administration, it may be prepared in the form of solid formulations, liquid formulations, suspensions, granules, capsules, or semi-solid formulations.

In another example, when formulated for parenteral administration, the pharmaceutical composition may be prepared in the form of an injectable formulation, an aerosol formulation, or the like. Preferably, it may be formulated as an injectable formulation.

In the case of an injectable formulation, methods known in the art for delaying drug absorption and/or prolonging the pharmacological effect of the drug may be additionally employed, as necessary. For example, an injectable depot formulation may be prepared by encapsulating the active ingredient using a biodegradable polymer. In another example, an injectable depot formulation may be prepared by entrapping the active ingredient **in a** liposome or a microemulsion.

Specific Examples of Pharmaceutical Compositions

Hereinafter, specific examples of pharmaceutical compositions are described; however, the present disclosure is not limited to the examples described below.

Composition Example (1): Pharmaceutical Composition Comprising a GSTA4 Protein or a Nucleic Acid Sequence Encoding the Same

In one example, the pharmaceutical composition for treating spinal cord injury according to the present disclosure may comprise a GSTA4 protein or a nucleic acid sequence encoding the same.

In another example, the pharmaceutical composition for treating spinal cord injury according to the present disclosure may comprise:

a promoter; and

a GSTA4 protein or a nucleic acid sequence encoding the same.

At this time, the promoter may be selected from a CBA promoter, a PGK promoter, a CMV promoter, or a CAG promoter. Preferably, the promoter may be a CMV promoter. In one specific embodiment, the sequence of the promoter may be the sequence represented by SEQ ID NO: 37.

As another example, the pharmaceutical composition for treating spinal cord injury according to the present application may comprise:

a promoter;

a GSTA4 protein or a nucleic acid sequence encoding the same; and

one or more selected from a pharmaceutically acceptable carrier, excipient, diluent, and preservative.

In this case, the promoter, pharmaceutically acceptable carrier, excipient, diluent, preservative, and the like may be the same as those described above.

As a specific example, the pharmaceutical composition for treating spinal cord injury according to the present application may comprise:

Sequence ID No. 37;

Sequence ID No. 2 or 12; and

a buffer comprising one or more selected from sodium chloride, potassium chloride, potassium dihydrogen phosphate, dibasic sodium phosphate, and Poloxamer 188.

In this case, the pharmaceutical composition may be formulated as an injectable preparation.

Composition Example (2): A pharmaceutical composition comprising a GSTA4 protein or a nucleic acid sequence encoding the same; and an MNX1 (Hb9) protein or a nucleic acid sequence encoding the same

As one example, the pharmaceutical composition for treating spinal cord injury according to the present application may comprise: a GSTA4 protein or a nucleic acid sequence encoding the same; and an MNX1 (Hb9) protein or a nucleic acid sequence encoding the same.

As another example, the pharmaceutical composition for treating spinal cord injury according to the present application may comprise:

a promoter;

a GSTA4 protein or a nucleic acid sequence encoding the same; and an MNX1 (Hb9) protein or a nucleic acid sequence encoding the same.

At this time, the promoter may be selected from a CBA promoter, a PGK promoter, a CMV promoter, or a CAG promoter. Preferably, the promoter may be a CMV promoter. In one specific embodiment, the sequence of the promoter may be the sequence represented by SEQ ID NO: 37.

As another example, the pharmaceutical composition for treating spinal cord injury according to the present application may comprise:

a promoter;

a GSTA4 protein or a nucleic acid sequence encoding the same;

an MNX1 (Hb9) protein or a nucleic acid sequence encoding the same; and

one or more pharmaceutically acceptable carriers, excipients, diluents, or preservatives.

In this case, the promoter, the pharmaceutically acceptable carriers, excipients, diluents, preservatives, and the like may be the same as those described above.

In a specific example, the pharmaceutical composition for treating spinal cord injury according to the present application may comprise:

Sequence ID No. 37;

Sequence ID No. 2;

Sequence ID No. 14; and

a buffer comprising one or more selected from the group consisting of sodium chloride, potassium chloride, potassium dihydrogen phosphate, dibasic sodium phosphate, and poloxamer 188.

In another specific example, the pharmaceutical composition for treating spinal cord injury according to the present application may comprise:

Sequence ID No. 37;

Sequence ID No. 12;

Sequence ID No. 14; and

a buffer comprising one or more selected from the group consisting of sodium chloride, potassium chloride, potassium dihydrogen phosphate, dibasic sodium phosphate, and poloxamer 188.

In this case, the pharmaceutical composition may be formulated as an injectable preparation.

Composition Example (3): A pharmaceutical composition comprising a GSTA4 protein or a nucleic acid sequence encoding the same; and an Lhx3 protein or a nucleic acid sequence encoding the same

In one example, the pharmaceutical composition for treating spinal cord injury according to the present application may comprise: a GSTA4 protein or a nucleic acid sequence encoding the same; and an Lhx3 protein or a nucleic acid sequence encoding the same.

In another example, the pharmaceutical composition for treating spinal cord injury according to the present application may comprise:

a promoter;
a GSTA4 protein or a nucleic acid sequence encoding the same; and an Lhx3 protein or a nucleic acid sequence encoding the same.

In this case, the promoter may be selected from a CBA promoter, a PGK promoter, a CMV promoter, or a CAG promoter. Preferably, the promoter may be a CMV promoter. In one specific embodiment, the promoter sequence may be the sequence represented by SEQ ID NO: 37.

In another example, the pharmaceutical composition for treating spinal cord injury according to the present application may comprise:

a promoter;

a GSTA4 protein or a nucleic acid sequence encoding the same;

an Lhx3 protein or a nucleic acid sequence encoding the same; and

one or more pharmaceutically acceptable carriers, excipients, diluents, or preservatives.

In this case, the promoter and the pharmaceutically acceptable carrier, excipient, diluent, preservative, and the like may be the same as those described above.

In a specific example, the pharmaceutical composition for treating spinal cord injury according to the present application may comprise:

Sequence ID No. 37;

Sequence ID No. 2;

Sequence ID No. 16; and

a buffer comprising one or more selected from sodium chloride, potassium chloride, potassium dihydrogen phosphate, dibasic sodium phosphate, and Poloxamer 188.

In another specific example, the pharmaceutical composition for treating spinal cord injury according to the present application may comprise:

Sequence ID No. 37;

Sequence ID No. 12;

Sequence ID No. 16; and

a buffer comprising one or more selected from sodium chloride, potassium chloride, potassium dihydrogen phosphate, dibasic sodium phosphate, and Poloxamer 188.

In this case, the pharmaceutical composition may be formulated as an injectable formulation.

Composition Example (4): A pharmaceutical composition comprising a GSTA4 protein or a nucleic acid sequence encoding the same; an MNX1 (Hb9) protein or a nucleic acid sequence encoding the same; and an Lhx3 protein or a nucleic acid sequence encoding the same

In one example, the pharmaceutical composition for treating spinal cord injury according to the present application may comprise: a GSTA4 protein or a nucleic acid sequence encoding the same; a nucleic acid sequence encoding an MNX1 (Hb9) protein; and an Lhx3 protein or a nucleic acid sequence encoding the same.

In another example, the pharmaceutical composition for treating spinal cord injury according to the present application may comprise:

a promoter;

a GSTA4 protein or a nucleic acid sequence encoding the same; a nucleic acid sequence encoding an MNX1 (Hb9) protein; and an Lhx3 protein or a nucleic acid sequence encoding the same.

In this case, the promoter may be selected from a CBA promoter, a PGK promoter, a CMV promoter, or a CAG promoter. Preferably, the promoter may be a CMV promoter. In one specific embodiment, the promoter sequence may be the sequence set forth in SEQ ID NO: 37.

In another example, the pharmaceutical composition for treating spinal cord injury according to the present application may comprise:

a promoter;

a GSTA4 protein or a nucleic acid sequence encoding the same;

an Lhx3 protein or a nucleic acid sequence encoding the same;

27

a nucleic acid sequence encoding an MNX1 (Hb9) protein; and one or more selected from pharmaceutically acceptable carriers, excipients, diluents, and preservatives.

In this case, the promoter and the pharmaceutically acceptable carrier, excipient, diluent, and preservative may be the same as those described above.

In a specific example, the pharmaceutical composition for treating spinal cord injury according to the present application may comprise:

Sequence ID No. 37;

Sequence ID No. 2;

Sequence ID No. 14;

Sequence ID No. 16; and

a buffer comprising one or more selected from sodium chloride, potassium chloride, potassium dihydrogen phosphate, dibasic sodium phosphate, and Poloxamer 188.

In another specific example, the pharmaceutical composition for treating spinal cord injury according to the present application may comprise:

Sequence ID No. 37;

Sequence ID No. 12;

Sequence ID No. 14;

Sequence ID No. 16; and

a buffer comprising one or more selected from sodium chloride, potassium chloride, potassium dihydrogen phosphate, dibasic sodium phosphate, and Poloxamer 188.

In this case, the pharmaceutical composition may be formulated as an injectable preparation.

Use (2): Kit

In another aspect of the present application, a kit comprising the composition for treating spinal cord injury is disclosed.

The kit may comprise one or more containers including instructions for use according to methods known in the art. In general, the instructions for use may include directions for administration of the pharmaceutical composition for treating, diagnosing, or preventing a disease.

The instructions for use may include information regarding the dosage, dosing schedule, and route of administration for the intended treatment.

For example, the instructions for use may include information on the dosage, dosing schedule, and route of administration for treating spinal cord injury using the composition for treating spinal cord injury of the present disclosure.

The kit may further include one or more containers. For example, the container may be a vial, a bottle, or the like.

The container may contain the composition for treating spinal cord injury of the present disclosure, wherein the type of the container may be determined depending on the formulation form of the composition for treating spinal cord injury.

**2. Method for Treating a Spinal Cord Injury Disease**

Overview of the Method for Treating a Spinal Cord Injury Disease

Another aspect of the present disclosure relates to a method for treating a spinal cord injury disease.

The spinal cord injury disease is the same as described above.

The method for treating a spinal cord injury disease utilizes the pharmaceutical composition described above.

As described above, the pharmaceutical composition for treating a spinal cord injury disease according to the present disclosure comprises a direct conversion factor.

In particular, the method for treating a spinal cord injury disease according to the present disclosure has an advantage in that it can be directly administered to an injured spinal cord. The ability to function as an in vivo therapeutic agent is attributable to the following characteristics of the pharmaceutical composition for treating a spinal cord injury disease according to the present disclosure:

   Feature (1): The direct conversion factor comprised in the pharmaceutical composition for treating a spinal cord injury disease is capable of directly converting somatic cells into motor neurons.

   Feature (2): A nucleic acid sequence encoding the direct conversion factor can be delivered to target cells by an AAV vector.

Accordingly, the method for treating a spinal cord injury disease according to the present disclosure may be used as an alternative that overcomes the limitations of conventional methods for treating spinal cord diseases.

Hereinafter, a method for treating a spinal cord injury disease, including administration and dosage, will be described.

Method of Treatment

The method for treating a spinal cord injury disease according to the present disclosure comprises administering a pharmaceutical composition (therapeutic agent) for treating a spinal cord injury disease to a subject.

The pharmaceutical composition may be in the form of a vector, cells comprising a vector, a protein, or cells comprising a protein, but is not limited thereto. Preferably, the pharmaceutical composition is in the form comprising a vector. More preferably, the pharmaceutical composition is in the form comprising an AAV vector (AAV particle).

By way of example, a method for treating a spinal cord injury disease may comprise administering to a subject a pharmaceutical composition comprising an AAV vector that includes a nucleic acid sequence encoding a direct conversion factor.

In one specific example, a method for treating a spinal cord injury disease may comprise administering to a subject a pharmaceutical composition comprising an AAV vector that includes a nucleic acid sequence encoding a GSTA4 protein. Preferably, the AAV vector may be an AAV2 vector.

In another specific example, a method for treating a spinal cord injury disease may comprise administering to a subject a pharmaceutical composition comprising an AAV vector that includes a nucleic acid sequence encoding a GSTA4 protein and a nucleic acid sequence encoding an Lhx3 protein. Preferably, the AAV vector may be an AAV2 vector.

In another specific example, a method for treating a spinal cord injury disease may comprise administering to a subject a pharmaceutical composition comprising an AAV vector that includes a nucleic acid sequence encoding a GSTA4 protein and a nucleic acid sequence encoding an Hb9 (MNX1) protein. Preferably, the AAV vector may be an AAV2 vector.

In another specific example, a method for treating a spinal cord injury disease may comprise administering to a subject a pharmaceutical composition comprising an AAV vector including a nucleic acid sequence encoding a GSTA4 protein, a nucleic acid sequence encoding an Hb9 (MNX1) protein, and a nucleic acid sequence encoding an Lhx3 protein. Preferably, the AAV vector may be an AAV2 vector.

Subject to be Administered

The subject (patient) to whom the method for treating a spinal cord injury disease is applied may be a subject in whom the spinal cord is damaged, or a subject in whom motor neuron function is reduced or the number of motor neurons is decreased, but is not limited thereto.

The subject may be a mammal in need of treatment or prevention of a spinal cord injury disease. For example, the mammal may be a human, dog, horse, cat, mouse, rat, pig, rabbit, sheep, monkey, chimpanzee, or the like. Preferably, the subject is a human.

Method of Administration

The therapeutic agent for treating a spinal cord injury disease according to the present disclosure may be administered to a subject via various routes depending on clinical necessity.

The administration may be oral administration or parenteral administration. In this case, the parenteral administration may be injection administration.

In the case of injection administration, the administration site may include, but is not limited to, a damaged spinal cord, muscle, intradermal, subcutaneous, intravenous, intraperitoneal, intra-arterial, mucosal, bone marrow, intrathecal, or transdermal site. Preferably, the injection is directly administered to the damaged spinal cord.

For example, when the administration site is a damaged spinal cord, the site may be at L1 (lumbar 1), L2 (lumbar 2), L3 (lumbar 3), L4 (lumbar 4), L5 (lumbar 5), T1 (thoracic 1), T2 (thoracic 2), T3 (thoracic 3), T4 (thoracic 4), T5 (thoracic 5), T6 (thoracic 6), T7 (thoracic 7), T8 (thoracic 8), T9 (thoracic 9), T10 (thoracic 10), T11 (thoracic 11), T12 (thoracic 12), T13 (thoracic 13), C1 (cervical 1), C2 (cervical 2), C3 (cervical 3), C4 (cervical 4), C5 (cervical 5), C6 (cervical 6), C7 (cervical 7), C8 (cervical 8), S1 (sacrum 1), S2 (sacrum 2), S3 (sacrum 3), or S4 (sacrum 4).

The method may include administering, to the L4 region of the spinal cord, an AAV vector (AAV particle) comprising a nucleic acid sequence encoding a GSTA4 protein, or a pharmaceutical composition comprising the same, to a subject having damage at the L4 region of the spinal cord.

The method may include administering, to the L5 region of the spinal cord, an AAV vector (AAV particle) comprising a nucleic acid sequence encoding a GSTA4 protein, or a pharmaceutical composition comprising the same, to a subject having damage at the L5 region of the spinal cord.

The method may include administering, to an intervertebral region between L4 and L5 of the spinal cord, an AAV vector (AAV particle) comprising a nucleic acid sequence encoding a GSTA4 protein, or a pharmaceutical composition comprising the same, to a subject having damage in the region between L4 and L5 of the spinal cord.

In one specific example, a method of treating a spinal cord injury may include administering, to a subject having damage in a region between L4 and L5 of the spinal cord, an AAV vector (AAV particle) comprising a nucleic acid sequence encoding a GSTA4 protein, or a pharmaceutical composition comprising the same, into the region between L4 and L5 of the spinal cord.

In another specific example, a method of treating a spinal cord injury may include administering, to a subject having damage at L4-L5 of the spinal cord, an AAV vector (AAV particle) comprising a nucleic acid sequence encoding a GSTA4 protein and a nucleic acid sequence encoding an Lhx3 protein, or a pharmaceutical composition comprising the same, to L4-L5 of the spinal cord.

In another specific example, a method of treating a spinal cord injury may include administering, to a subject having damage at L4-L5 of the spinal cord, an AAV vector (AAV particle) comprising a nucleic acid sequence encoding a GSTA4 protein and a nucleic acid sequence encoding an MNX1 (Hb9) protein, or a pharmaceutical composition comprising the same, to L4-L5 of the spinal cord.

In another specific example, a method of treating a spinal cord injury may include administering, to a subject having damage at L4-L5 of the spinal cord, an AAV vector (AAV particle) comprising a nucleic acid sequence encoding a GSTA4 protein, a nucleic acid sequence encoding an MNX1 (Hb9) protein, and a nucleic acid sequence encoding an Lhx3 protein, or a pharmaceutical composition comprising the same, to L4-L5 of the spinal cord.

Administration Dose

The administration dose of the pharmaceutical composition of the present application may be determined in consideration of the type of disease of the subject, the site of administration, body weight, age, degree of disease progression, and the like, but is not limited thereto.

In addition, the administration dose may be determined within a range that does not cause excessive toxicity, irritation, allergic reactions, or other adverse effects, in accordance with a reasonable benefit/risk ratio and within the scope of sound medical judgment.

As a non-limiting example, when the pharmaceutical composition of the present disclosure is purified in the form of an AAV vector (AAV particle), the following doses may be included.

For example, when the subject is a human, an AAV particle may be administered at a dose within a range defined by any two selected values from $1 \times 10^5$ genome copies (GC)/kg, $1 \times 10^6$ GC/kg, $1 \times 10^7$ GC/kg, $1 \times 10^8$ GC/kg, $1 \times 10^5$ GC/kg, $1 \times 10^{10}$ GC/kg, $1 \times 10^{11}$ GC/kg, $1 \times 10^{12}$ GC/kg, $1 \times 10^{13}$ GC/kg, $1 \times 10^{14}$ GC/kg, $1 \times 10^{15}$ GC/kg, $1 \times 10^{16}$ GC/kg, $1 \times 10^{17}$ GC/kg, $1 \times 10^{18}$ GC/kg, $1 \times 10^{19}$ GC/kg, and $1 \times 10^{20}$ GC/kg.

The pharmaceutical composition may comprise an amount of AAV determined by a person skilled in the art in consideration of such AAV administration doses, such that the total AAV content included in the composition is appropriately adjusted.

In one specific embodiment, the method for treating a spinal cord injury disease according to the present application may comprise administering to a subject an AAV particle comprising a nucleic acid sequence encoding a GSTA4 protein at a dose of from $1 \times 10^5$ GC/kg to $1 \times 10^{15}$ GC/kg. Preferably, the AAV particle may be administered at a dose of from $1 \times 10^6$ GC/kg to $1 \times 10^{12}$ GC/kg. Most preferably, the AAV particle may be administered at a dose of from $6 \times 10^8$ GC/kg to $5 \times 10^{11}$ GC/kg. More preferably, the AAV particle may be administered at a dose of from $6.7 \times 10^8$ GC/kg to $3.3 \times 10^{11}$ GC/kg. In one embodiment, the AAV particle may be administered at a dose of $2.5 \times 10^9$ GC/kg. In another embodiment, the AAV particle may be administered at a dose of $6.7 \times 10^8$ GC/kg.

In another specific embodiment, the method for treating a spinal cord injury disease according to the present application may comprise administering to a subject an AAV particle comprising a nucleic acid sequence encoding a GSTA4 protein and a nucleic acid sequence encoding an Lhx3 protein at a dose of from $1\times10^5$ GC/kg to $1\times10^{15}$ GC/kg. Preferably, the AAV particle may be administered at a dose of from $1\times10^6$ GC/kg to $1\times10^{12}$ GC/kg. Most preferably, the AAV particle may be administered at a dose of from $6\times10^8$ GC/kg to $5\times10^{11}$ GC/kg. More preferably, the AAV particle may be administered at a dose of from $6.7\times10^8$ GC/kg to $3.3\times10^{11}$ GC/kg. In one embodiment, the AAV particle may be administered at a dose of $2.5\times10^9$ GC/kg. In another embodiment, the AAV particle may be administered at a dose of $6.7\times10^8$ GC/kg.

In another specific embodiment, the method for treating a spinal cord injury disease according to the present application may comprise administering to a subject an AAV particle comprising a nucleic acid sequence encoding a GSTA4 protein and a nucleic acid sequence encoding an Hb9 (MNX1) protein at a dose of from $1\times10^5$ GC/kg to $1\times10^{15}$ GC/kg. Preferably, the AAV particle may be administered at a dose of from $1\times10^6$ GC/kg to $1\times10^{12}$ GC/kg. Most preferably, the AAV particle may be administered at a dose of from $6\times10^8$ GC/kg to $5\times10^{11}$ GC/kg. More preferably, the AAV particle may be administered at a dose of from $6.7\times10^8$ GC/kg to $3.3\times10^{11}$ GC/kg. In one embodiment, the AAV particle may be administered at a dose of $2.5\times10^9$ GC/kg. In another embodiment, the AAV particle may be administered at a dose of $6.7\times10^8$ GC/kg.

In another specific embodiment, the method for treating a spinal cord injury disease according to the present application may comprise administering to a subject an AAV particle comprising a nucleic acid sequence encoding a GSTA4 protein, a nucleic acid sequence encoding an Hb9 (MNX1) protein, and a nucleic acid sequence encoding an Lhx3 protein at a dose of from $1\times10^5$ GC/kg to $1\times10^{15}$ GC/kg. Preferably, the AAV particle may be administered at a dose of from $1\times10^6$ GC/kg to $1\times10^{12}$ GC/kg. Most preferably, the AAV particle may be administered at a dose of from $6\times10^8$ GC/kg to $5\times10^{11}$ GC/kg. More preferably, the AAV particle may be administered at a dose of from $6.7\times10^8$ GC/kg to $3.3\times10^{11}$ GC/kg. In one embodiment, the AAV particle may be administered at a dose of $2.5\times10^9$ GC/kg. In another embodiment, the AAV particle may be administered at a dose of $6.7\times10^8$ GC/kg.

In another example, when the subject is a non-human animal, an AAV particle may be administered at a dose within a range selected from any two values among $1\times10^5$ GC (genome copies)/head, $1\times10^6$ GC/head, $1\times10^7$ GC/head, $1\times10^8$ GC/head, $1\times10^9$ GC/head, $1\times10^{10}$ GC/head, $1\times10^{11}$ GC/head, $1\times10^{12}$ GC/head, $1\times10^{13}$ GC/head, $1\times10^{14}$ GC/head, $1\times10^{15}$ GC/head, $1\times10^{16}$ GC/head, $1x10^{17}$ GC/head, $1\times10^{18}$ GC/head, $1\times10^{19}$ GC/head, and $1\times10^{20}$ GC/head. Herein, "GC/head" refers to the dose of genome copies (GC) administered per individual animal.

In one specific example, the method for treating a spinal cord injury according to the present application may comprise administering to a subject an AAV particle comprising a nucleic acid sequence encoding a GSTA4 protein at a dose of from $1\times10^5$ GC/head to $1\times10^{15}$ GC/head. Preferably, the AAV particle may be administered at a dose of from $1\times10^6$ GC/head to $1\times10^{12}$ GC/head. Most preferably, the AAV particle may be administered at a dose of from $2\times10^7$ GC/head to $1\times10^{10}$ GC/head.

In another specific example, the method for treating a spinal cord injury according to the present application may comprise administering to a subject an AAV particle comprising a nucleic acid sequence encoding a GSTA4 protein; and a nucleic acid sequence encoding an Lhx3 protein, at a dose of from $1\times10^5$ GC/head to $1\times10^{15}$ GC/head. Preferably, the AAV particle may be administered at a dose of from $1\times10^6$ GC/head to $1\times10^{12}$ GC/head. Most preferably, the AAV particle may be administered at a dose of from $2\times10^7$ GC/head to $1\times10^{10}$ GC/head.

In another specific example, the method for treating a spinal cord injury according to the present application may comprise administering to a subject an AAV particle comprising a nucleic acid sequence encoding a GSTA4 protein; and a nucleic acid sequence encoding an Hb9 (MNX1) protein, at a dose of from $1\times10^5$ GC/head to $1\times10^{15}$ GC/head. Preferably, the AAV particle may be administered at a dose of from $1\times10^6$ GC/head to $1\times10^{12}$ GC/head. Most preferably, the AAV particle may be administered at a dose of from $2\times10^7$ GC/head to $1\times10^{10}$ GC/head.

In another specific example, the method for treating a spinal cord injury according to the present application may comprise administering to a subject an AAV particle comprising a nucleic acid sequence encoding a GSTA4 protein; a nucleic acid sequence encoding an Hb9 (MNX1) protein; and a nucleic acid sequence encoding an Lhx3 protein, at a dose of from $1\times10^5$ GC/head to $1\times10^{15}$ GC/head. Preferably, the AAV particle may be administered at a dose of from $1\times10^6$ GC/head to $1\times10^{12}$ GC/head. Most preferably, the AAV particle may be administered at a dose of from $2\times10^7$ GC/head to $1\times10^{10}$ GC/head.

The administration dose may be determined based on data obtained from cell culture studies and/or animal studies and/or human clinical trials. In this regard, a dosage range for administration to humans may be determined based on an administration dose obtained from animal studies or preclinical studies using methods known in the art.

For example, the dosage range for administration to humans may be formulated with reference to the following equation:

$$\text{HED (Human Equivalent Dose)} = \text{animal NOAEL} \times (W_{\text{animal}}/W_{\text{human}})^{(1-b)}$$

wherein NOAEL (No Observed Adverse Effect Level) refers to a non-toxic dose.

In another example, the dosage range for administration to humans may be determined by selecting a value equal to or less than the MRSD (Maximum Recommended Starting Dose). In this case, the MRSD value may be calculated with reference to the following equation:

$$MRSD = HED / SF$$

In this case, the pharmaceutical composition may comprise an AAV at a concentration per 1 μL selected from a range defined by any two values selected from $1\times10^5$ genome copies (GC), $1\times10^6$ GC, $1\times10^7$ GC, $1\times10^8$ GC, $1\times10^9$ GC, $1\times10^{10}$ GC, $1\times10^{11}$ GC, $1\times10^{12}$ GC, $1\times10^{13}$ GC, $1\times10^{14}$ GC, $1\times10^{15}$ GC, $1\times10^{16}$ GC, $1\times10^{17}$ GC, and $1\times10^{18}$ GC.

By way of example, the pharmaceutical composition of the present application may comprise an AAV at a concentration of from $1\times10^5$ GC to $1\times10^{16}$ GC per 1 μL. Preferably, the pharmaceutical composition may comprise an AAV at a concentration of from $1\times10^5$ GC to $1\times10^{14}$ GC per 1 μL. Most preferably, the pharmaceutical composition may comprise an AAV at a concentration of from $2\times10^5$ GC to $3.0\times10^{13}$ GC per 1 μL.

In one specific embodiment, the pharmaceutical composition of the present application may comprise an AAV containing a nucleic acid sequence encoding a GSTA4 protein at an amount of from $1\times10^5$ GC to $1\times10^{16}$ GC per 1 μL. Preferably, the AAV may be present at an amount of from $1\times10^5$ GC to $1\times10^{14}$ GC per 1 μL. Most preferably, the AAV may be present at an amount of from $2\times10^5$ GC to $3.0\times10^{13}$ GC per 1 μL.

In another specific embodiment, the pharmaceutical composition of the present application may comprise, per 1 μL, an AAV comprising a nucleic acid sequence encoding a GSTA4 protein and a nucleic acid sequence encoding an Lhx3 protein at an amount of from $1\times10^5$ GC to $1\times10^{16}$ GC. Preferably, the AAV may be present at an amount of from $1\times10^5$ GC to $1\times10^{14}$ GC per 1 μL. Most preferably, the AAV may be present at an amount of from $2\times10^5$ GC to $3.0\times10^{13}$ GC per 1 μL.

In another specific embodiment, the pharmaceutical composition of the present application may comprise, per 1 μL, an AAV comprising a nucleic acid sequence encoding a GSTA4 protein and a nucleic acid sequence encoding an Hb9 (Mnx1) protein at an amount of from $1\times10^5$ GC to $1\times10^{16}$ GC. Preferably, the AAV may be present at an amount of from $1\times10^5$ GC to $1\times10^{14}$ GC per 1 μL. Most preferably, the AAV may be present at an amount of from $2\times10^5$ GC to $3.0\times10^{13}$ GC per 1 μL.

In another specific embodiment, the pharmaceutical composition of the present application may comprise, per 1 μL, an AAV comprising a nucleic acid sequence encoding a GSTA4 protein, a nucleic acid sequence encoding an Hb9 (Mnx1) protein, and a nucleic acid sequence encoding an Lhx3 protein at an amount of from $1\times10^5$ GC to $1\times10^{16}$ GC. Preferably, the AAV may be present at an amount of from $1\times10^5$ GC to $1\times10^{14}$ GC per 1 μL. Most preferably, the AAV may be present at an amount of from $2\times10^5$ GC to $3.0\times10^{13}$ GC per 1 μL.

The administration volume of the pharmaceutical composition of the present application may be appropriately selected in consideration of the administration route, the subject, and the like.

For example, the administration volume may range from 1 μL to 20 mL per administration, without being limited thereto.

By way of example, when the subject is a human, upon direct administration of the pharmaceutical composition of the present application to the injured spinal cord of the human, the administration may be performed in a volume per dose selected from within a numerical range defined by any two values selected from the group consisting of 1 μL, 2 μL, 3 μL, 4 μL, 5 μL, 6 μL, 7 μL, 8 μL, 9 μL, 10 μL, 11 μL, 12 μL, 13 μL, 14 μL, 15 μL, 16 μL, 17 μL, 18 μL, 19 μL, 20 μL, 21 μL, 22 pL, 23 μL, 24 μL, 25 μL, 26 μL, 27 μL, 28 μL, 29 μL, 30 μL, 31 μL, 32 μL, 33 μL, 34 μL, 35 μL, 36 μL, 37 μL, 38 μL, 39 μL, 40 μL, 41 μL, 42 μL, 43 μL, 44 μL, 45 μL, 46 μL, 47 μL, 48 μL, 49 μL, 50 μL, 51 μL, 52 μL, 53 μL, 54 μL, 55 μL, 56 μL, 57 μL, 58 μL, 59 μL, 60 μL, 61 μL, 62 μL, 63 μL, 64 μL, 65 μL, 66 μL, 67 μL, 68 μL, 69 μL, 70 μL, 71 μL, 72 μL, 73 μL, 74 μL, 75 μL, 76 μL, 77 μL, 78 μL, 79 μL, 80 μL, 81 μL, 82 μL, 83 μL, 84 μL, 85 μL, 86 μL, 87 μL, 88 μL, 89 μL, 90 μL, 91 μL, 92 μL, 93 μL, 94 μL, 95 μL, 96 μL, 97 μL, 98 μL, 99 μL, 100 μL, 110 μL, 120 μL, 130 μL, 140 μL, 150 μL, 200 μL, 250 μL, 300 μL, 350 μL, 400 μL, 450 μL, 500 μL, 600 μL, 700 μL, 800 μL, 900 μL, 950 μL, 1 mL, 2 mL, 3 mL, 4 mL, 5 mL, 6 mL, 7 mL, 8 mL, 9 mL, and 10 mL.

In one specific embodiment, when the pharmaceutical composition is directly administered to an injured spinal cord of a human, the administration may be performed in a volume of 10 μL to 200 μL per dose. In another specific embodiment, when the pharmaceutical composition is directly administered to an injured spinal cord of a human, the administration may be performed in a volume of 10 μL to 100 μL per dose. In another specific embodiment, when the pharmaceutical composition is directly administered to an injured spinal cord of a human, the administration may be performed in a volume of 10 μL to 90 μL per dose. In another specific embodiment, when the pharmaceutical composition is directly administered to an injured spinal cord of a human, the administration may be performed in a volume of 20 μL to 80 μL per dose. In another specific embodiment, when the pharmaceutical composition is directly administered to an injured spinal cord of a human, the administration may be performed in a volume of 20 μL to 70 μL per dose. In another specific embodiment, when the pharmaceutical composition is directly administered to an injured spinal cord of a human, the administration may be performed in a volume of 20 μL to 60 μL per dose. In another specific embodiment, when the pharmaceutical composition is directly administered to an injured spinal cord of a human, the administration may be performed in a volume of 30 μL to 60 μL per dose. In an optional embodiment, when the pharmaceutical composition is directly administered to an injured spinal cord of a human, the administration may be performed in a volume of 40 μL to 60 μL per dose. Most preferably, when the pharmaceutical composition is directly administered to an injured spinal cord of a human, the administration may be performed in a volume of 50 μL per dose.

In another example, when the subject is a non-human animal, the pharmaceutical composition of the present disclosure may be directly administered to an injured spinal cord of the non-human animal in a volume selected from within a range defined by any two of the following values per administration: 1 μL, 2 μL, 3 μL, 4 μL, 5 μL, 6 μL, 7 μL, 8 μL, 9 μL, 10 μL, 11 μL, 12 μL, 13 μL, 14 μL, 15 μL, 16 μL, 17 μL, 18 μL, 19 μL, 20 μL, 21 μL, 22 μL, 23 μL, 24 μL, 25 μL, 26 μL, 27 μL, 28 μL, 29 μL, 30 μL, 31 μL, 32 μL, 33 μL, 34 μL, 35 μL, 36 μL, 37 μL, 38 μL, 39 μL, 40 μL, 41 μL, 42 μL, 43 μL, 44 μL, 45 μL, 46 μL, 47 μL, 48 μL, 49 μL, 50 μL, 51 μL, 52 μL, 53 μL, 54 μL, 55 μL, 56 μL, 57 μL, 58 μL, 59 μL, 60 μL, 61 μL, 62 μL, 63 μL, 64 μL, 65 μL, 66 μL, 67 μL, 68 μL, 69 μL, 70 μL, 71 μL, 72 μL, 73 μL, 74 μL, 75 μL, 76 μL, 77 μL, 78 μL, 79 μL, 80 μL, 81 μL, 82 μL, 83 μL, 84 μL, 85 μL, 86 μL, 87 μL, 88 μL, 89 μL, 90 μL, 91 μL, 92 μL, 93 μL, 94 μL, 95 μL, 96 μL, 97 μL, 98 μL, 99 μL, 100 μL, 110 μL, 120 μL, 130 μL, 140 μL, 150 μL, 200 μL, 250 μL, 300 μL, 350 μL, 400 μL, 450 μL, 500 μL, 600 μL, 700 μL, 800 μL, 900 μL, 950 μL, 1 mL, 2 mL, 3 mL, 4 mL, 5 mL, 6 mL, 7 mL, 8 mL, 9 mL, and 10 mL. In this case, the non-human animal may be selected from mouse, rat, chimpanzee, monkey, dog, pig, or the like, without being limited thereto.

In one specific example, when the pharmaceutical composition is directly administered to an injured spinal cord of a non-human animal, the composition may be administered in a volume of 1 μL to 100 μL per administration. In another specific example, the pharmaceutical composition may be administered in a volume of 1 μL to 80 μL per administration. In another specific example, the pharmaceutical composition may be administered in a volume of 1 μL to 70 μL per administration. In another specific example, the pharmaceutical composition may be administered in a volume of 1 μL to 60 μL per administration. In another specific example, the pharmaceutical composition may be administered in a volume of 1 μL to 50 μL per administration. In another specific example, the pharmaceutical composition may be administered in a volume of 1 μL to 30 μL per administration. In another specific example, the pharmaceutical composition may be administered in a volume of 1 μL to 20 μL per administration. In another specific example, the pharmaceutical composition may be administered in a volume of 1 μL to 10 μL per administration. In an optional embodiment, the pharmaceutical composition may be administered in a volume of 1 μL to 5 μL per administration. Most preferably, the pharmaceutical composition may be administered in a volume of 2 μL per administration to the injured spinal cord of the non-human animal.

Administration Schedule

The administration schedule for administering the pharmaceutical composition to a subject may be determined according to the type of disease, the severity of onset, and the progression of the disease in the subject.

The number of administrations may be once per day or multiple times per day, or may be administered multiple times over a long-term period. In this case, the pharmaceutical composition may be administered at predetermined time intervals.

The administration interval may be, for example, from 1 day to 30 days. In this case, the pharmaceutical composition may be administered continuously or intermittently.

Confirmation of Therapeutic Effects

After applying the method for treating a spinal cord injury disorder to a subject, the alleviation, improvement, or treatment of the spinal cord injury disorder may be confirmed by various methods.

For example, the therapeutic effect may be confirmed through reduction of scar formation, changes in the expression of neuronal markers, BBB (Basso, Beattie, and Bresnahan) scores, action potentials, spontaneous responses, and the like.

In one example, by using the method for treating a spinal cord injury disorder, scar formation may be reduced by about 2-fold to 50-fold.

In another example, by using the method for treating a spinal cord injury disorder, the expression of neuronal markers may be increased by about 2-fold to 50-fold.

In another example, by using the method for treating a spinal cord injury disorder, the BBB score may be increased by about 2-fold to 50-fold.

In another example, by using the method for treating a spinal cord injury disorder, action potentials or spontaneous responses may be exhibited in a pattern similar to that of a normal subject.

The administration concentration, administration dose, administration schedule, and the like used in the above-described method for treating a spinal cord injury disorder may be readily determined and prescribed as a therapeutically effective amount by a physician, veterinarian, or the like skilled in the art.

For example, a physician or veterinarian may gradually increase or decrease the administered dose until a desired therapeutic effect is achieved. In addition, a physician or veterinarian may determine the therapeutically effective amount, administration period, and the like depending on the condition of the subject.

Further, the method for treating a spinal cord injury disorder according to the present application may be used in combination with adjunct therapies for rapid recovery and/or treatment of the spinal cord injury disorder. Such adjunct therapies may include physical therapy, rehabilitation therapy, administration of anticonvulsants, and/or administration of antidepressants, but are not limited thereto.

Meaning of Treatment or Recovery of an Injured Spinal Cord

As used herein, the treatment or recovery of an injured spinal cord refers to the treatment of a spinal cord injury disorder. In particular, since the composition for treating a spinal cord injury disorder according to the present application includes GSTA4, which is a direct reprogramming factor, the treatment of the spinal cord injury disorder refers to treatment resulting from the generation of motor neurons.

Specifically, the generation of motor neurons induced by GSTA4 may be hypothesized to occur through the following three mechanisms. However, in the present application, the treatment of an injured spinal cord is not limited to the three mechanisms described below, and includes all hypotheses that may be reasonably conceived by a person skilled in the art.

Generation of Motor Neurons Induced by GSTA4 (1): Direct Reprogramming of Abnormally Increased Astrocytes into Motor Neurons

A composition comprising GSTA4 may generate motor neurons by directly reprogramming somatic cells within an injured spinal cord into motor neurons. For example, in an injured spinal cord, astrocytes may abnormally proliferate while motor neurons are lost. In such a case, a composition comprising GSTA4 may act on the astrocytes to induce direct reprogramming into motor neurons, and the motor neurons thus generated may contribute to the treatment or recovery of the injured spinal cord.

Generation of motor neurons induced by GSTA4 (2): Restoration of apoptotic motor neurons

When the spinal cord is injured, motor neurons present within the spinal cord may undergo apoptosis. A composition comprising GSTA4 may act on the injured spinal cord to generate motor neurons by direct conversion of apoptotic motor neurons. For example, motor neurons within the injured spinal cord may undergo apoptosis as a result of a programmed cell death pathway but may remain present without undergoing degradation. In such a case, a composition comprising GSTA4 may act on the apoptotic motor neurons and induce direct reprogramming into normal motor neurons. As a result, the injured spinal cord may be treated or restored by the motor neurons generated through such direct conversion.

Generation of motor neurons induced by GSTA4 (3): Restoration of function in motor neurons that have not undergone apoptosis but exhibit reduced or degenerative function

When the spinal cord is injured, the function of motor neurons present within the spinal cord may be reduced. In other words, when the function of motor neurons is diminished, the muscles stimulated by such motor neurons may deteriorate and may fail to function properly. For example, when a composition comprising GSTA4 acts on an injured spinal cord, motor neurons exhibiting reduced function may be subjected to direct conversion into motor neurons having normal function. As a result of the generation of motor neurons with restored normal function, the injured spinal cord may be treated or restored.

[Modes for Carrying Out the Invention]

Hereinafter, the present invention will be described in more detail with reference to Experimental Examples.

These Experimental Examples are provided solely for the purpose of illustrating the present invention more specifically, and it will be apparent to those skilled in the art that the scope of the present invention is not limited by these Examples.

Experimental Materials

● Vector Design

In the experiments described below, an adeno-associated virus (AAV) vector (Cell Biolabs, Inc., VPK-402) was used, and a schematic diagram of the AAV vector for introduction of differentiation factors is illustrated in Fig. 1.

The DNA sequences of each vector used, corresponding to the vector schematics shown in Fig. 1, are as follows:

Fig. 1(a) corresponds to SEQ ID NO: 36; Fig. 1(b) corresponds to SEQ ID NO: 3; Fig. 1(c) corresponds to SEQ ID NO: 4; Fig. 1(d) corresponds to SEQ ID NO: 5; and Fig. 1(e) corresponds to SEQ ID NO: 29.

Fig. 1(a) comprises a nucleic acid sequence encoding human-derived GSTA4;

Fig. 1(b) comprises nucleic acid sequences encoding human-derived ASCL1, human-derived NEUROG2, human-derived ISL1, and human-derived LHX3;

Fig. 1(c) comprises nucleic acid sequences encoding human-derived BRN2, human-derived MNX1 (Motor neuron and pancreas homeobox 1; also referred to as Hb9), and human-derived NeuroD1;

Fig. 1(d) comprises nucleic acid sequences encoding human-derived GSTA4 and human-derived MYTL1; and

Fig. 1(e) comprises nucleic acid sequences encoding human-derived GSTA4, human-derived MNX1, and human-derived LHX3.

● AAV Production

For production of AAV, a 293T cell line was used. The 293T cells were cultured in Dulbecco's Modified Eagle Medium (DMEM; Thermo Fisher Scientific, #12430112) supplemented with 10% fetal bovine serum (FBS; Thermo Fisher Scientific, #26140079) and 1% antibiotic-antimycotic (Thermo Fisher Scientific, #15240096) under conditions of 37°C and 5% $CO_2$. The 293T cells were transfected with the AAV vector shown in Fig. 1, together with pHelper and pRepCap, and then cultured for 48 hours. Thereafter, AAV virus particles were isolated through an AAV virus particle extraction process, thereby obtaining AAV virus particles comprising differentiation factors. The produced AAV virus particles may hereinafter also be referred to as "AAV" or "AAV particles."

• Cell culture and intracellular delivery of AAV

Culture of fibroblasts and introduction of AAV comprising differentiation factors

Human dermal fibroblasts (Sigma, 106-05A) were cultured in fibroblast growth medium (Sigma, 116-500) supplemented with 10% fetal bovine serum (FBS; Thermo Fisher Scientific, #26140079) and 1% antibiotic-antimycotic (Thermo Fisher Scientific, #15240096) under conditions of 37°C and 5% $CO_2$.

Human dermal fibroblasts were prepared by seeding $0.025 \times 10^6$ cells per well in 24-well plates. Twenty-four hours after cell seeding, AAV comprising differentiation factors was applied to the cells (hereinafter, for convenience, the "AAV comprising differentiation factors" may also be referred to simply as "differentiation factors").

24 hours after treatment with the differentiation factors, the medium was replaced with fibroblast growth medium. After an additional 24 hours, the medium was replaced with neural induction medium. Thereafter, the medium was changed once every two days. On days 14 to 17, the cells were fixed, and immunofluorescence staining was performed.

Mouse embryonic fibroblasts were cultured in Dulbecco's Modified Eagle Medium (DMEM; Gibco) supplemented with 10% fetal bovine serum (Thermo Fisher Scientific, #26140079) and 1% penicillin/streptomycin (Gibco) at 37°C under 5% $CO_2$.

Mouse embryonic fibroblasts were seeded in 24-well plates at a density of $0.025 \times 10^6$ cells per well. Twenty-four hours after cell seeding, differentiation factors were administered. 24 hours after treatment with the differentiation factors, the medium was replaced with DMEM. After an additional 24 hours, the medium was replaced with neural induction medium. The medium was changed every two days thereafter. On days 14-17, the cells were fixed and subjected to immunofluorescence staining.

Culture of mouse-derived primary astrocytes and introduction of AAV comprising differentiation factors

The meninges were carefully removed from P1 pups of ICR mice, and the remaining tissue was finely minced using sterilized scissors or a razor blade. The minced tissue was transferred to a 50 mL conical tube, followed by the addition of 3 mL of TrypLE™ Express Enzyme (Gibco) and 20 μL of 0.5 M ascorbic acid (Sigma). The 50 mL conical tube was gently tapped to mix the contents thoroughly and incubated at 37°C for 20 minutes. Thereafter, the suspension was diluted with 10 mL of glial medium (DMEM supplemented with 1% L-glutamine (Gibco) and 1% penicillin/streptomycin). The medium was replaced with fresh glial medium every two days. On day 9, when the glial cells reached confluence in a T75 flask, the flask was placed on an orbital shaker in a 37°C incubator and shaken at 60 rpm for 30 minutes. The flask was then allowed to equilibrate in a 37°C incubator for 4 hours. Subsequently, the T75 flask was shaken on an orbital shaker in a 37°C incubator at 245 rpm for 18-20 hours. The cells were then passaged and aliquoted in amounts appropriate for subsequent use.

Mouse-derived primary astrocytes were prepared by seeding 0.2-0.6 × $10^6$ cells per well in 24-well plates. 24 hours after cell seeding, differentiation factors were applied. After 24 hours of treatment with the differentiation factors, the medium was replaced with DMEM. 24 hours thereafter, the medium was replaced with neural induction medium. The medium was changed every two days. On days 14-17, the cells were fixed, and immunofluorescence staining was performed.

• Immunofluorescence staining

For cell fixation, the cells were washed twice with PBS (Gibco) for 5 minutes each. Subsequently, 4% paraformaldehyde (Thermo Fisher) was added, and the cells were incubated at room temperature for 10 minutes.
For permeabilization, PBS containing 0.1% Triton-X was added, and the cells were incubated at room temperature for 10 minutes. The cells were then washed three times with PBS for 5 minutes each.
For blocking and immunostaining, cells were incubated with 1% BSA in PBST (PBS + 0.1% Tween 20) at room temperature for 30 minutes. Primary antibodies against ChAT (Invitrogen, 1:1000), Map2 (Millipore, 1:200), and Tuj1 (Beta III Tubulin) (Abcam, 1:1000) were used. The primary antibodies were diluted in 1% BSA + PBST and incubated with the cells overnight at 4°C. After washing three times with PBS for 5 minutes each, secondary antibodies Alexa Fluor™ 488/594 (Thermo Fisher, 1:1000) diluted in 1% BSA + PBST were added and incubated for 1 hour at room temperature in the dark. The cells were then washed three times with PBS for 5 minutes each, followed by DAPI staining.

• RNA extraction and cDNA synthesis

At approximately 14-17 days after seeding, cells treated with AAV containing differentiation factors were harvested. A total of 2 × $10^6$ cells were detached using trypsin (Thermo Fisher) and centrifuged at 13,000 rpm for 10 seconds. After removal of the supernatant, 1 mL of easy-Blue™ (iNtRON) was added, followed by vortexing for 10 seconds. Subsequently, 200 μL of chloroform was added and the mixture was vortexed. After centrifugation at 13,000 rpm for 10 minutes, 400 μL of the upper aqueous phase was transferred to a new 1.5 mL tube. Then, 400 μL of 2-propanol (Sigma) was added, gently mixed by inverting 2-3 times, and incubated at room temperature for 10 minutes. The mixture was centrifuged at 13,000 rpm for 5 minutes, and the upper layer was removed. Next, 1 mL of 75% ethanol (Sigma) was added and mixed by inverting 2-3 times. After centrifugation at 10,000 rpm at 4°C for 5 minutes, the upper layer was removed. The RNA pellet was air-dried at room temperature for 5 minutes and then dissolved in 20 μL of distilled water. The isolated RNA was stored at -70°C until use.
cDNA synthesis was performed using AccuPower® CycleScript RT PreMix & Master Mix (Bioneer) according to the manufacturer's instructions.

● Real-time quantitative reverse transcription PCR (qRT-PCR)

To determine whether fibroblasts or astrocytes were differentiated into motor neurons, qRT-PCR analysis was performed using SYBR Green Real-time PCR Master Mix (TOYOBO). To verify whether direct conversion and direct reprogramming from fibroblasts or astrocytes into motor neurons had occurred, the expression levels of Synapsin, Map2, and Hb9 in the cells were analyzed by qRT-PCR. It was confirmed that cells in which differentiation into motor neurons was induced from fibroblasts or astrocytes exhibited high expression levels of Synapsin, Map2, and Hb9.
The primers used in this analysis are listed in the table below.

[Table 1]

| Name | Sequence | Sequence No. |
|---|---|---|
| hSYN1_F | GTG GCA CTG ACC AAG ACG TA | SEQ ID NO: 6 |
| hSYN1_R | CTG ACG TCC TCA TGT AGG CC | SEQ ID NO: 7 |
| hMAP2_F | TCC TCC ATT CTC CCT CCT CG | SEQ ID NO: 8 |

(continued)

| Name | Sequence | Sequence No. |
|---|---|---|
| hMAP2_R | CGA ATT GGC TCT GAC CTG GT | SEQ ID NO: 9 |
| hHb9_F | GAG CTT GAA CTG GTG CTC CA | SEQ ID NO: 10 |
| hHb9_R | TCT CAA CAG TAG GTG CCT GC | SEQ ID NO: 11 |
| mMap2-F | ACC TTC CTC CAT CCT CCC TC | SEQ ID NO: 30 |
| mMap2-R | TCC TGC TCT GCG AAT TGG TT | SEQ ID NO: 31 |
| mHb9-F | TCC AGA ACC GCC GAA TGA AA | SEQ ID NO: 32 |
| mHb9-R | CCA TCA GCT CCT CTT CCG TC | SEQ ID NO: 33 |
| mSynapsin-F | TCC TCC TGC TCA ACA ACG AC | SEQ ID NO: 34 |
| mSynapsin-R | CGG TCC AAG GCC AGA AAG AT | SEQ ID NO: 35 |

● Cell viability test

Mouse-derived primary astrocytes were seeded into a 96-well plate at a density of $0.2 \times 10^5$ cells per well. After treatment with the test substance, the cells were incubated at 37°C for 20 hours. Thereafter, an MTS reagent (Promega, G3582) was added to the cells prepared in the 96-well plate, followed by incubation at 37°C for 30 minutes. After brief shaking, absorbance was measured at 490 nm using a microplate reader (Allsheng, AMR-100).

● SCI mouse model for spinal cord injury - Establishment of an SCI Mouse Model

To establish an SCI (spinal cord injury) mouse model representing paraplegia caused by spinal cord injury, ICR mice were anesthetized by intraperitoneal injection of avertin (125-250 mg/kg). After confirmation of anesthesia, the animals were fixed on a surgical table. The surgical site (dorsal region) was disinfected with povidone and 70% ethanol and sufficiently dried. Following confirmation of anesthesia, the skin over the vertebral column was disinfected with povidone solution and incised. After removal of the T13 vertebra, the spinal cord bundle was injured by applying compression with forceps equipped with a digital caliper (Cixi Duxan) to a thickness of 0.35 mm for 30 seconds. After induction of the spinal cord injury, the surgical site was sutured using surgical sutures. Following surgery, the animals were placed on a heating pad pre-warmed to 37°C and monitored until recovery of consciousness. After awakening from anesthesia, an analgesic (ketoprofen, 1 mg/kg) was administered subcutaneously to alleviate severe pain. After recovery, the mice were housed in an animal facility and their condition was monitored for up to three weeks. For a certain period after completion of surgery, a warm environment was maintained using an infrared lamp to prevent a drop in body temperature. The SCI mouse model was generated by inducing injury at the lumbar 4-5 region of the spinal cord (hereinafter referred to as L4-5; corresponding to vertebral levels T13-L1), and the animals were used for experiments five weeks after injury.

● Administration of Test Substances to the SCI Mouse Model

All surgical procedures were performed for each experimental group, and sterilized and disinfected instruments were used to prevent contamination during surgery. Prior to administration, anesthesia was induced by intraperitoneal injection of avertin (125-250 mg/kg). Once the animals were fully anesthetized, they were fixed on a surgical platform. The surgical site (dorsal area) was disinfected with povidone and 70% ethanol and allowed to dry sufficiently. To expose the vertebral column, the muscle adjacent to the administration site was vertically incised using a scalpel blade, taking care to avoid damage to nerves and blood vessels. After confirming the administration site under a stereomicroscope, a Hamilton syringe (33-gauge needle) was oriented toward the lumbar direction and inserted at an angle of approximately 20-30° into the injured region between L4 and L5 of the SCI mouse model. The prepared test substance (2 μL) was then microinjected using an infusion pump at a rate of approximately 1 μL/min. After completion of the injection, the needle was left in place for approximately 2 minutes and then slowly withdrawn from the spinal cord. Following administration, the incised muscle and skin were sutured using surgical sutures.

● Behavioral Assessment

Among behavioral assessments, the Basso, Beattie, and Bresnahan (hereinafter referred to as the "BBB") score test, which enables evaluation of motor function, was performed. The BBB score test was conducted in accordance with the standard operating procedure EXP-SOP-012 (Basso, Beattie, and Bresnahan (BBB) score test).

The BBB score was measured before administration of the prepared AAV, immediately after administration, and then weekly from 1 week to 8 weeks after administration. The prepared AAV was a composition comprising Gsta4, Mnx1, and Lhx3 (hereinafter referred to as "STUP-001").

If the score increased in a statistically significant manner compared to the control group, the treated active substance (denoted as STUP-001 in this experimental example) was determined to have effective efficacy. The analysis criteria were as follows. The BBB scores for all animals were recorded as measured values in the table below, and figures were generated using corrected BBB scores. To normalize inter-individual variability, the evaluation was performed by applying the following equation, wherein the reference point was defined as the average BBB score for each animal during weeks 1 to 4 after surgery.

$$\text{Corrected BBB score} = \text{BBB score (week n)} - \text{BBB score (reference point)}$$

● Physiological Assessment

Electrophysiological recordings of motor neurons using patch-clamp techniques in ex vivo spinal cord slices were performed according to the method described by Shim HS et al. (2019). The lumbar region of the dissected spinal cord was transversely sectioned at a thickness of 350 $\mu$m in cold (4°C) NMDG (N-methyl-D-glucamine)-based solution saturated with a mixed gas (95% oxygen and 5% carbon dioxide) (in mM: 93 NMDG, 2.5 KCl, 1.2 NaH$_2$PO$_4$, 30 NaHCO$_3$, 20 HEPES, 25 glucose, 5 sodium ascorbate, 2 thiourea, 3 sodium pyruvate, 10 MgSO$_4$, and 0.5 CaCl$_2$; pH 7.4). Whole-cell electrophysiological recordings of motor neurons located in the ventral horn of the spinal cord were obtained in artificial cerebrospinal fluid (aCSF; in mM: 124 NaCl, 2.5 KCl, 1.2 NaH$_2$PO$_4$, 24 NaHCO$_3$, 5 HEPES, 12.5 glucose, 2 MgSO$_4$, and 2 CaCl$_2$; pH 7.4). Electrophysiological signals were acquired using a Multiclamp 700B amplifier (Molecular Devices, Sunnyvale, CA), DigiDATA (Molecular Devices, Sunnyvale, CA), and pClamp software (version 10.6, Molecular Devices). Patch pipettes with a resistance of 4-8 M$\Omega$ were used and filled with an internal solution (in mM: 120 K-gluconate, 10 KCl, 2 Mg-ATP, 0.5 Na-GTP, 0.5 EGTA, 20 HEPES, and 10 phosphocreatine; pH 7.3). Action potentials of motor neurons were measured using current-step injections ranging from 0 pA to 140 pA in increments of 20 pA.

● Protein Quantification Assay

Protein quantification at the administration site was performed using ELISA kits. For each experimental animal, the levels of neuronal marker proteins MAP2 and ChAT at the administration site were measured, and the results were presented in tables and graphs. Information on the ELISA kits used is provided in the table below (Table 2).

[Table 2]

| ELISA kit | Company | Catalog Number |
|---|---|---|
| Mouse choline acetyltransterase (CHAT)ELISA Kit | CUSABIO | CSB-E17524m |
| Mouse Microtubule-associated protein 2(MAP2) ELISA Kit | Abcam | ab253229 |

● Body Weight Measurement

The body weight of the mouse model was measured prior to administration of the test substance, and thereafter measured once weekly and on the day of necropsy. On the day of necropsy, body weight was measured after fasting.

● Functional Observation Tests

Functional observation tests were performed on all surviving animals during the final week of observation (approximately 13 weeks after administration). The following items were evaluated. Stimulus response tests included auditory response, righting reflex, nociceptive response, ear twitch response, and threat (menace) reflex. Muscle strength was assessed by a grip strength test to evaluate neurological and muscular condition. Locomotor activity was examined outside the home cage to assess general motor activity.

**Experimental Example 1: Confirmation of the Effect of Gsta4 on Direct Reprogramming into Motor Neurons in Cells**

Experimental Example 1-1: Introduction of GSTA4 into Mouse-Derived Fibroblasts

An AAV containing GSTA4 (Fig. 1(a)) was introduced into mouse-derived fibroblasts. At approximately 14 to 17 days after AAV introduction, direct reprogramming of fibroblasts into neuronal cells induced by Gsta4 was confirmed by immunofluorescence staining and qPCR analysis (Fig. 3).

In the differentiated cells, high expression of ChAT, a motor neuron marker, and Map2, a marker of mature neurons, was confirmed by immunofluorescence staining. In addition, changes in the expression of Synapsin, a synapse formation marker, Map2, a mature neuronal marker, and Hb9, a mature neuronal marker, were examined by qPCR, thereby confirming the effect of direct reprogramming and direct conversion of fibroblasts into neuronal cells.

As a result, when Gsta4 alone was administered, the expression level of synapsin was increased by approximately four-fold compared to the control, the expression level of Map2 was increased by approximately five-fold compared to the control, and the expression level of Hb9 was increased by approximately two-fold compared to the control.

These results indicate that the Gsta4 factor has the ability to induce direct reprogramming and direct conversion of fibroblasts into neuronal cells.

Experimental Example 1-2: Introduction of GSTA4 into mouse-derived primary astrocytes

An AAV comprising GSTA4, as used in Experimental Example 1-1, was introduced into mouse-derived primary astrocytes. Differentiation of astrocytes into neuronal cells induced by Gsta4 was examined by immunofluorescence staining (Figs. 5 and 6).

Immunofluorescence staining confirmed high expression of the neuronal marker Tuj1 and the mature neuronal marker Map2 in the differentiated cells.

In the control group (denoted as control), neither Tuj1 nor Map2 expression was detected. In contrast, upon treatment with Gsta4 alone, the number of cells immunostained for neuronal markers was increased by approximately 40-50 fold for Tuj1 and approximately 20-30 fold for Map2 compared to the control group.

These results indicate that the Gsta4 factor has the capability to induce direct reprogramming and direct conversion of mouse-derived primary astrocytes into neuronal cells.

From the results of Experimental Example 1, it can be understood that Gsta4 alone possesses the capability to effectively induce the conversion of somatic cells into motor neurons through direct conversion and direct reprogramming.

## Experimental Example 2: Verification of direct conversion of known differentiation factors into motor neurons in cells

In Experimental Example 1, it was confirmed that Gsta4 alone is capable of inducing direct conversion of somatic cells into motor neurons. Furthermore, the direct conversion efficiency of Gsta4 was compared with that of conventionally known direct conversion factors. For this purpose, known direct conversion factors were introduced into cells, and the extent of direct conversion into motor neurons was evaluated.

Experimental Example 2-1: Comparison of effects with known direct conversion factors

Conventional differentiation factors were introduced into mouse-derived fibroblasts, and the extent of differentiation into motor neurons was evaluated by qPCR by examining the expression of neuronal markers Synapsin and Map2 (see Fig. 4(a)).

When representative conventional differentiation factors Ascl1 and Brn2 were each treated alone, the expression level of Synapsin showed an increase of approximately 1.5- to 2-fold compared to the control, whereas the expression level of Map2 was almost similar to that of the control and showed little to no increase.

This result was compared with the case in which Gsta4 of the present application was treated alone (Experimental Example 1). As described above, Gsta4 of the present application exhibited an approximately 4-fold increase in Synapsin expression and an approximately 5-fold increase in Map2 expression compared to the control. Accordingly, when compared with conventionally known differentiation factors, it was confirmed that Gsta4 of the present application shows a markedly higher efficiency of direct reprogramming into motor neurons.

In addition, conventionally known differentiation factors MNX1 and Lhx3 were each introduced into mouse-derived primary astrocytes, and the expression of the neuronal marker Tuj1 and the mature neuronal marker Map2 was examined by immunofluorescence staining, and the results were compared with those obtained for Gsta4 of the present application.

As can be seen from Figs. 5 and 6, when the conventional differentiation factors MNX1 and Lhx3 were each administered alone, the expression levels of Tuj1 and Map2 were found to be almost similar to those of the control group. In contrast, when GSTA4 was administered alone, the expression of Tuj1 and Map2 was markedly increased, such that the number of cells immunostained with neuronal markers was approximately 10- to 15-fold higher than in the cases where MNX1 or Lhx3 was administered alone.

From these experimental results, it can be seen that the direct reprogramming capability of GSTA4 toward motor neurons according to the present invention is significantly higher than the direct reprogramming capability toward motor neurons of conventionally known differentiation factors (Ascl1, Brn2, MNX1, and Lhx3).

This suggests that GSTA4 of the present invention can be highly useful for the regeneration and generation of motor neurons.

Experimental Example 2-2: Combination of Conventional Direct Reprogramming Factors

Through Experimental Example 2-1, it was confirmed that GSTA4 alone exhibits an excellent direct reprogramming effect toward motor neurons. In addition, the present inventors further sought to compare this effect with the differentiation effects obtained when two or more conventionally known differentiation factors were combined.

To this end, various combinations of conventional differentiation factors were applied to human-derived fibroblasts, and the extent of differentiation into motor neurons was evaluated by qPCR. In this analysis, the expression levels of neuronal markers Synapsin, Map2, and Hb9 were examined (Fig. 4(b)).

Specifically, the following combinations were each introduced into human-derived fibroblasts:

Combination 1: a combination of Ascl1, Brn2, and Myt1I (designated as ABM); and

Combination 2: a combination of Hb9, Isl1, and Lhx3 (designated as HIL).

In this case, for both Combination 1 and Combination 2, the expression level of synapsin was increased by approximately 1.5- to 3-fold compared with the control group (indicated as control), the expression level of Map2 was increased by approximately 1- to 4-fold compared with the control group, and the expression level of Hb9 was increased by approximately 1- to 1.5-fold compared with the control group.

In Example 1, when Gsta4 was administered alone, the expression of synapsin was increased by approximately 4-fold and the expression of Map2 was increased by approximately 5-fold compared with the respective control groups. In contrast, even when three conventional differentiation factors were administered in combination, the efficiency of direct reprogramming into motor neurons was lower than that observed with administration of Gsta4 alone according to the present invention. These results indicate that Gsta4 of the present invention exhibits a superior direct reprogramming effect compared to cases in which combinations of conventional differentiation factors are used.

Through the results of Experimental Example 2, it was confirmed that Gsta4, as compared with conventionally known factors, exhibits a remarkably high efficiency of direct reprogramming and thus can be regarded as a novel "direct reprogramming factor for conversion into motor neurons".

**Experimental Example 3: Confirmation of enhanced direct reprogramming effects by combination use of Gsta4**

The present inventors further sought to examine the effects obtained when Gsta4 is used in combination with conventionally known direct reprogramming factors.

Experimental Example 3-1: Comparison between MNX1 alone and Gsta4 + MNX1

In mouse-derived primary astrocytes, direct reprogramming effects were compared between a case in which MNX1 alone was introduced using an AAV vector and a case in which Gsta4 and MNX1 were introduced together.

The degree of differentiation was evaluated by immunofluorescence staining, and the expression of Tuj1, a neuronal marker, and Map2, a marker of mature neurons, was examined (Figs. 5 and 6).

As a result, the number of Tuj1-expressing cells was only slightly increased compared to the control (indicated as control) when the conventional differentiation factor MNX1 was administered alone. In contrast, when the combination of Gsta4 and MNX1 (indicated as Gsta4+MNX1) was administered, the number of Tuj1-expressing cells was increased by approximately 8- to 20-fold or more compared to the control.

The number of Map2-expressing cells was almost negligible when the conventional differentiation factor MNX1 was administered alone, whereas treatment with the combination of Gsta4 and MNX1 (indicated as Gsta4+MNX1) resulted in an increase of approximately 50-fold or more in Map2-expressing cells.

From these results, it was confirmed that when GSTA4, which exhibits a high efficiency of direct reprogramming from somatic cells to motor neurons according to the present application, is used in combination with the conventional differentiation factor MNX1, the efficiency of direct reprogramming is further enhanced.

That is, treatment with the combination of Gsta4 and MNX1 resulted in an unexpectedly remarkable direct reprogramming effect.

Experimental Example 3-2: Comparison of Lhx3 vs. Gsta4 + Lhx3

The direct reprogramming effects were compared between a case in which only Lhx3 was introduced via AAV into mouse-derived primary astrocytes and a case in which both Gsta4 and Lhx3 were introduced.
The extent of differentiation was evaluated by immunofluorescence staining, by examining the expression of the neuronal marker Tuj1 and the mature neuronal marker Map2 (Figs. 5 and 6).
As a result, the number of cells expressing Tuj1 was only slightly increased compared to the control group (indicated as control) when the conventional differentiation factor Lhx3 was treated alone. In contrast, when Gsta4 and Lhx3 were treated in combination (indicated as Gsta4+Lhx3), the number of Tuj1-positive cells was confirmed to increase by approximately 8- to 20-fold or more.
The number of cells expressing Map2 was almost negligible when the conventional differentiation factor Lhx3 was treated alone, whereas when Gsta4 and Lhx3 were treated in combination (indicated as Gsta4+Lhx3), the number of Map2-positive cells was confirmed to increase by approximately 20-fold or more.
From these results, it was confirmed that when GSTA4, which exhibits a high efficiency of direct reprogramming from somatic cells into motor neurons according to the present application, is used in combination with the conventional differentiation factor Lhx3, the efficiency of direct reprogramming is further increased.
That is, when Gsta4 and Lhx3 were administered in combination, an unexpected and remarkably enhanced direct reprogramming effect was observed.

Experimental Example 3-3: Comparison of MNX1 +Lhx3 vs. Gsta4+MNX1 +Lhx3

3-3-1. Direct reprogramming of mouse-derived fibroblasts

The direct reprogramming efficiency was compared between a case in which MNX1 and Lhx3 were introduced into mouse-derived fibroblasts using AAV and a case in which Gsta4, MNX1, and Lhx3 were introduced in combination using AAV.
To assess and compare the degree of differentiation, the expression levels of neuronal markers were analyzed using immunofluorescence staining and qPCR (Figs. 7 and 8). For reference, the results obtained with Gsta4 alone (indicated as Gsta4) were also included and shown together in the graphs.
First, as a result of analyzing the immunofluorescence-stained cells, it was confirmed that the number of cells positive for neuronal markers was increased by approximately 40-fold or more when Gsta4 was combined with Mnx1 and Lhx3 (indicated as Gsta4+Mnx1+Lhx3), compared to the case in which the conventional differentiation factors Mnx1 and Lhx3 were used in combination alone (indicated as Mnx1+Lhx3) (Fig. 7).
In addition, the expression levels of neuronal markers (Synapsin and Map2) were analyzed by qPCR (Fig. 8).
Compared with treatment using the conventional differentiation factors Mnx1 and Lhx3 in combination (denoted as Mnx1+Lhx3), treatment using the combination of Gsta4, Mnx1, and Lhx3 (denoted as Gsta4+Mnx1+Lhx3) resulted in an approximately 2- to 4-fold or greater increase in the mRNA expression levels of neuronal markers.
Specifically, in the case of Gsta4+Mnx1+Lhx3, the expression levels of Synapsin, Map2, and Hb9 were increased by approximately 8-fold, 7-fold, and 4-fold, respectively, compared with the control group. In addition, compared with the Mnx1+Lhx3 group, the expression levels of Synapsin, Map2, and Hb9 were increased by approximately 4-fold, 4-fold, and 2.5-fold, respectively.
In other words, compared with the combination of the conventional differentiation factors Mnx1+Lhx3 alone, the addition of Gsta4, that is, treatment with "Gsta4+Mnx1+Lhx3," resulted in an even more pronounced direct reprogramming effect.
As observed in the foregoing experiments, administration of Gsta4 alone (denoted as Gsta4) exhibited a superior direct reprogramming effect compared with the conventional factor combination (Mnx1+Lhx3). Moreover, when Gsta4 was additionally used in combination with such conventional factors, it was found that a highly pronounced direct reprogramming of cells into motor neurons occurred.

3-3-2. Direct conversion of mouse-derived primary astrocytes

In addition, the inventors evaluated the effect of the "Gsta4+Mnx1 +Lhx3" combination on mouse-derived primary astrocytes. Specifically, a composition containing Gsta4, Mnx1, and Lhx3 (referred to as STUP-001) was administered to mouse-derived primary astrocytes at different concentrations, and the degree of differentiation into motor neurons was assessed by immunofluorescence staining to examine the expression of neuronal markers (Tuj1 and Map2) (Fig. 9).

At this time, the concentration of Gsta4+Mnx1+Lhx3 (designated as STUP-001) was set to $1 \times 10^{10}$ GC/well. "Naive" refers to a control group that was not treated with any substance, while "control" refers to a vehicle control group treated with an AAV2 buffer containing sodium chloride (13.84 g/L), potassium chloride (0.2 g/L), potassium dihydrogen phosphate (0.24 g/L), dibasic sodium phosphate (1.44 g/L), and Poloxamer 188 (0.01 g/L).

As shown in Fig. 9, in the group treated with Gsta4+Mnx1+Lhx3 (designated as STUP-001) in mouse-derived astrocytes, the expression intensity of neuronal markers was confirmed to be increased by approximately 3- to 5-fold or more.

From the results of Experimental Examples 1 to 3, it was confirmed that Gsta4 of the present application is a differentiation factor having the capability to directly convert somatic cells, such as fibroblasts and astrocytes, into induced motor neurons. In addition, Gsta4 exhibits a higher direct conversion efficiency than conventionally known direct conversion factors, and furthermore, when used in combination with such conventional factors, Gsta4 was found to markedly enhance the relatively low differentiation efficiency of the conventional differentiation factors.

Furthermore, the inventors sought to verify, through in vivo animal experiments, the direct conversion effect of somatic cells into motor neurons by the use of Gsta4 of the present invention or the combination of Gsta4+Mnx1+Lhx3, as well as the therapeutic effect on spinal cord injury resulting therefrom.

**Experimental Example 4: Mouse experiments to evaluate the therapeutic effect of Gsta4 on spinal cord injury**

The SCI mouse model prepared according to the methods described above was administered with a composition comprising GSTA4. The administration site and the administered dose were as described in the section entitled "Experimental Materials."

Immunofluorescence staining was performed to examine changes in the expression of motor neuron markers at the spinal cord injury site (between L4 and L5) in a group administered GSTA4 alone as a differentiation factor (Fig. 10).

As shown in Fig. 10, the intensities of ChAT and Map2 in the group treated with GSTA4 alone (denoted as SCI+GSTA4) were approximately fourfold higher than those in the spinal cord injury model group (denoted as SCI).

These results indicate that, when GSTA4 is administered alone to the SCI mouse model, cells within the injured spinal cord region undergo direct reprogramming (direct conversion) into motor neurons, thereby increasing the number of motor neurons at the site of spinal cord injury. In other words, it was indirectly confirmed that administration of GSTA4 alone can exert a therapeutic effect on spinal cord injury in the SCI mouse model.

**Experimental Example 5: Mouse experiment for confirming the therapeutic effect of Gsta4+Mnx1+Lhx3 on spinal cord injury**

In order to evaluate the therapeutic effect on spinal cord injury upon treatment with a composition comprising Gsta4, Mnx1, and Lhx3 (hereinafter referred to as STUP-001), cytotoxicity assessment was performed, followed by administration of the composition to mice and subsequent analyses using various evaluation methods.

Experimental Example 5-1: Evaluation of cell viability

In Experimental Example 4, it was confirmed that the composition comprising the combination of Gsta4, Mnx1, and Lhx3 (STUP-001) induces direct reprogramming of fibroblasts or astrocytes into motor neurons (Figs. 7 and 9). Furthermore, in order to evaluate the cytotoxicity of STUP-001, cell viability was additionally measured (Fig. 11).

Cell viability of cells directly converted into motor neurons was measured after treating mouse-derived primary astrocytes with STUP-001 at different concentrations.

In this experiment, STUP-001 was administered at concentrations of $1 \times 10^6$ GC/well, $1 \times 10^7$ GC/well, $1 \times 10^1$ GC/well, $1 \times 10^9$ GC/well, $1 \times 10^{10}$ GC/well, and $1 \times 10^{11}$ GC/well. Herein, "Naive" refers to a control group to which no substance was administered, and "control" refers to a vehicle control group treated with an AAV2 buffer.

As a result, as shown in Fig. 11, it was confirmed that even as the concentration of STUP-001 administered to mouse-derived primary astrocytes increased, the cell viability exhibited a pattern similar to that of the control group and the vehicle control group. This indicates that STUP-001 exhibits little to no cytotoxicity.

After confirming the absence of toxicity of STUP-001 in vitro, the inventors administered STUP-001 to an SCI mouse model, which is an animal model of spinal cord injury, at various concentrations, and then performed behavioral assessments, electrophysiological analyses, and protein quantification analyses.

At this time, groups E0 to E5 each consisted of three female mice and three male mice, and measurements were conducted for 8 weeks after administration.

E0 to E5 refer to the following groups:

E0 refers to a control group of normal ICR mice;

E1 refers to a vehicle control group in which an AAV2 buffer was administered to an SCI mouse model;

E2 refers to a group in which STUP-001 was administered to an SCI mouse model at a dose of $2.0 \times 10^8$ GC/head;

E3 refers to a group in which STUP-001 was administered to an SCI mouse model at a dose of $2.0 \times 10^7$ GC/head;

E4 refers to a group in which STUP-001 was administered to an SCI mouse model at a dose of $2.0 \times 10^6$ GC/head; and

E5 refers to a group in which STUP-001 was administered to an SCI mouse model at a dose of $2.0 \times 10^5$ GC/head.

## Experimental Example 5-2: Evaluation of therapeutic effects on spinal cord injury through behavioral analysis

Behavioral analysis was performed by measuring BBB scores after administering STUP-001 to SCI mouse models at different doses (Fig. 12, Tables 3-7).

Fig. 12 illustrates graphs generated from the BBB scores shown in Tables 3-7.

Table 3 shows the BBB scores of the E1 group, Table 4 shows the BBB scores of the E2 group, Table 5 shows the BBB scores of the E3 group, Table 6 shows the BBB scores of the E4 group, and Table 7 shows the BBB scores of the E5 group.

【Table 3】

| Group | Injection | User | Animal ID | Acc[1] | SCI | 1w | 2w | 3w | 4w[2] | 5w | 6w | 7w | 8w | 9w | 10w | 11w | 12w |
|---|---|---|---|---|---|---|---|---|---|---|---|---|---|---|---|---|---|
| E | 1 | | 7 | 21 | 0 | 1 | 1 | 2 | 2 | 2 | 1 | 2 | 2 | 3 | 3 | 3 | 3 |
| | | | 8 | 21 | 0 | 0 | 1 | 0 | 0 | 0 | 0 | 0 | 0 | 1 | 0 | 0 | 2 |
| | | User 1 | 9 | 21 | 0 | 0 | 1 | 0 | 0 | 1 | 2 | 3 | 3 | 3 | 1 | 3 | 2 |
| | | | 10 | 21 | 0 | 0 | 1 | 0 | 0 | 1 | 0 | 0 | 0 | 1 | 0 | 0 | 0 |
| | | | 11 | 21 | 0 | 2 | 0 | 0 | 0 | 0 | 0 | 0 | 0 | 2 | 2 | 3 | 1 |
| | | | 12 | 21 | 0 | 2 | 0 | 1 | 0 | 5 | 4 | 3 | 3 | 1 | 2 | 2 | 2 |
| | | | 7 | 21 | 0 | 1 | 2 | 2 | 2 | 3 | 2 | 3 | 3 | 4 | 4 | 3 | 3 |
| | | | 8 | 21 | 0 | 1 | 0 | 0 | 1 | 1 | 1 | 1 | 0 | 2 | 0 | 0 | 2 |
| | | User 2 | 9 | 21 | 0 | 0 | 1 | 2 | 2 | 2 | 3 | 3 | 3 | 2 | 4 | 2 | 3 |
| | | | 10 | 21 | 0 | 1 | 0 | 1 | 1 | 0 | 0 | 1 | 1 | 1 | 1 | 0 | 1 |
| | | | 11 | 21 | 0 | 0 | 0 | 1 | 1 | 2 | 0 | 0 | 0 | 1 | 1 | 1 | 1 |
| | | | 12 | 21 | 0 | 2 | 0 | 0 | 1 | 0 | 2 | 0 | 1 | 1 | 1 | 1 | 2 |
| | | | 7 | 21 | 0 | 2 | 1 | 2 | 2 | 3 | 3 | 3 | 4 | 3 | 4 | 3 | 3 |
| | | | 8 | 21 | 0 | 1 | 0 | 0 | 1 | 1 | 0 | 1 | 2 | 1 | 1 | 1 | 1 |
| | | User 3 | 9 | 21 | 0 | 0 | 1 | 1 | 2 | 3 | 3 | 2 | 1 | 1 | 3 | 2 | 3 |
| | | | 10 | 21 | 0 | 2 | 0 | 1 | 1 | 1 | 1 | 1 | 1 | 0 | 1 | 1 | 1 |
| | | | 11 | 21 | 0 | 0 | 0 | 0 | 0 | 1 | 1 | 1 | 1 | 1 | 1 | 1 | 0 |
| | | | 12 | 21 | 0 | 0 | 1 | 1 | 1 | 1 | 2 | 0 | 0 | 1 | 1 | 1 | 0 |

(1) Acc: Acclimation
(2) Day of Injection
w: week

【Table 4】

| Group | Injection | User | Animal ID | Acc[1] | SCI | 1w | 2w | 3w | 4w[2] | 5w | 6w | 7w | 8w | 9w | 10w | 11w | 12w |
|---|---|---|---|---|---|---|---|---|---|---|---|---|---|---|---|---|---|
| E | 2 | User 1 | 13 | 21 | 0 | 1 | 0 | 1 | 3 | 5 | 2 | 1 | 3 | 3 | 5 | 3 | 5 |
| | | | 14 | 21 | 0 | 0 | 1 | 2 | 2 | 6 | 5 | 5 | 6 | 7 | 8 | 9 | 9 |
| | | | 15 | 21 | 0 | 2 | 3 | 3 | 4 | 14 | 12 | 12 | 12 | 11 | 12 | 11 | 13 |
| | | | 16 | 21 | 0 | 0 | 0 | 1 | 3 | 8 | 3 | 2 | 2 | 2 | 3 | 4 | 5 |
| | | | 17 | 21 | 0 | 0 | 1 | 1 | 1 | 4 | 1 | 2 | 2 | 3 | 5 | 6 | 6 |
| | | | 18 | 21 | 0 | 4 | 4 | 5 | 5 | 7 | 7 | 8 | 9 | 9 | 14 | 16 | 17 |
| | | User 2 | 13 | 21 | 0 | 1 | 1 | 1 | 2 | 2 | 5 | 6 | 7 | 7 | 6 | 7 | 7 |
| | | | 14 | 21 | 0 | 1 | 2 | 0 | 3 | 7 | 5 | 5 | 8 | 7 | 7 | 10 | 11 |
| | | | 15 | 21 | 0 | 1 | 2 | 4 | 5 | 12 | 11 | 11 | 12 | 12 | 12 | 12 | 15 |
| | | | 16 | 21 | 0 | 0 | 0 | 1 | 1 | 2 | 5 | 6 | 4 | 6 | 5 | 5 | 6 |
| | | | 17 | 21 | 0 | 1 | 1 | 0 | 0 | 0 | 2 | 3 | 4 | 5 | 6 | 6 | 7 |
| | | | 18 | 21 | 0 | 1 | 4 | 4 | 5 | 7 | 11 | 15 | 15 | 15 | 14 | 17 | 17 |
| | | User 3 | 13 | 21 | 0 | 1 | 1 | 0 | 1 | 3 | 2 | 2 | 4 | 4 | 5 | 5 | 5 |
| | | | 14 | 21 | 0 | 0 | 1 | 2 | 2 | 6 | 5 | 5 | 6 | 7 | 7 | 7 | 7 |
| | | | 15 | 21 | 0 | 0 | 0 | 1 | 4 | 14 | 12 | 12 | 12 | 11 | 12 | 11 | 12 |
| | | | 16 | 21 | 0 | 0 | 0 | 1 | 1 | 5 | 2 | 2 | 3 | 1 | 3 | 2 | 8 |
| | | | 17 | 21 | 0 | 0 | 1 | 0 | 1 | 2 | 1 | 2 | 1 | 5 | 5 | 4 | 7 |
| | | | 18 | 21 | 0 | 2 | 4 | 4 | 6 | 7 | 11 | 16 | 17 | 17 | 14 | 17 | 19 |

(1) Acc: Acclimation
(2) Day of Injection
w: week

【Table 5】

| Group | Injection | User | Animal ID | Acc[1] | SCI | 1w | 2w | 3w | 4w[2] | 5w | 6w | 7w | 8w | 9w | 10w | 11w | 12w |
|---|---|---|---|---|---|---|---|---|---|---|---|---|---|---|---|---|---|
| E | 3 | User 1 | 19 | 21 | 0 | 1 | 1 | 0 | 2 | 3 | 5 | 4 | 3 | 8 | 8 | 9 | 9 |
| | | | 20 | 21 | 0 | 1 | 1 | 1 | 2 | 4 | 5 | 2 | 4 | 3 | 6 | 11 | 10 |
| | | | 21 | 21 | 0 | 1 | 0 | 1 | 3 | 4 | 6 | 8 | 8 | 9 | 9 | 10 | 10 |
| | | | 22 | 21 | 0 | 1 | 1 | 1 | 2 | 4 | 4 | 6 | 5 | 4 | 6 | 10 | 9 |
| | | | 23 | 21 | 0 | 1 | 0 | 0 | 1 | 1 | 2 | 2 | 4 | 6 | 6 | 6 | 7 |
| | | | 24 | 21 | 0 | 0 | 0 | 0 | 0 | 2 | 2 | 2 | 2 | 3 | 3 | 7 | 7 |
| | | User 2 | 19 | 21 | 0 | 1 | 0 | 0 | 1 | 2 | 3 | 3 | 4 | 4 | 6 | 6 | 8 |
| | | | 20 | 21 | 0 | 1 | 1 | 1 | 1 | 2 | 4 | 3 | 4 | 4 | 5 | 7 | 7 |
| | | | 21 | 21 | 0 | 2 | 1 | 1 | 3 | 5 | 6 | 8 | 9 | 9 | 9 | 11 | 12 |
| | | | 22 | 21 | 0 | 1 | 0 | 1 | 0 | 2 | 2 | 4 | 4 | 6 | 6 | 5 | 7 |
| | | | 23 | 21 | 0 | 0 | 0 | 1 | 1 | 1 | 2 | 3 | 5 | 5 | 4 | 4 | 7 |
| | | | 24 | 21 | 0 | 0 | 0 | 0 | 0 | 0 | 2 | 4 | 4 | 3 | 4 | 5 | 8 |
| | | User 3 | 19 | 21 | 0 | 0 | 1 | 0 | 2 | 6 | 7 | 3 | 3 | 3 | 5 | 6 | 9 |
| | | | 20 | 21 | 0 | 1 | 1 | 1 | 2 | 5 | 3 | 3 | 3 | 8 | 5 | 8 | 10 |
| | | | 21 | 21 | 0 | 1 | 0 | 1 | 3 | 4 | 6 | 8 | 8 | 9 | 9 | 9 | 11 |
| | | | 22 | 21 | 0 | 0 | 0 | 1 | 1 | 2 | 3 | 3 | 2 | 3 | 4 | 4 | 4 |
| | | | 23 | 21 | 0 | 0 | 0 | 1 | 1 | 0 | 0 | 4 | 5 | 3 | 4 | 3 | 7 |
| | | | 24 | 21 | 0 | 0 | 0 | 0 | 1 | 1 | 0 | 2 | 2 | 2 | 2 | 4 | 8 |

(1) Acc: Acclimation
(2) Day of Injection
w: week

【Table 6】

| Group | Injection | User | Animal ID | Acc[1] | SCI | 1w | 2w | 3w | 4w[2] | 5w | 6w | 7w | 8w | 9w | 10w | 11w | 12w |
|---|---|---|---|---|---|---|---|---|---|---|---|---|---|---|---|---|---|
| E | 4 | | 25 | 21 | 0 | 3 | 1 | 4 | 0 | 1 | 6 | 6 | 6 | 7 | 7 | 6 | 6 |
| | | | 26 | 21 | 0 | 1 | 1 | 1 | 2 | 3 | 4 | 3 | 0 | 4 | 2 | 1 | 1 |
| | | User 1 | 27 | 21 | 0 | 1 | 2 | 1 | 2 | 2 | 0 | 5 | 4 | 5 | 4 | 3 | 4 |
| | | | 28 | 21 | 0 | 1 | 0 | 1 | 0 | 0 | 0 | 1 | 1 | 3 | 3 | 2 | 3 |
| | | | 29 | 21 | 0 | 1 | 0 | 2 | 4 | 3 | 2 | 5 | 6 | 3 | 4 | 5 | 6 |
| | | | 30 | 21 | 0 | 3 | 4 | 3 | 5 | 4 | 6 | 6 | 6 | 6 | 7 | 8 | 8 |
| | | | 25 | 21 | 0 | 3 | 2 | 2 | 3 | 3 | 3 | 5 | 5 | 5 | 6 | 6 | 6 |
| | | | 26 | 21 | 0 | 4 | 3 | 4 | 4 | 6 | 5 | 2 | 7 | 6 | 3 | 3 | 4 |
| | | User 2 | 27 | 21 | 0 | 2 | 1 | 1 | 0 | 0 | 2 | 3 | 3 | 3 | 3 | 3 | 3 |
| | | | 28 | 21 | 0 | 1 | 2 | 1 | 3 | 3 | 1 | 1 | 1 | 4 | 3 | 3 | 3 |
| | | | 29 | 21 | 0 | 0 | 0 | 1 | 1 | 1 | 1 | 3 | 3 | 1 | 3 | 3 | 1 |
| | | | 30 | 21 | 0 | 0 | 0 | 0 | 2 | 2 | 3 | 3 | 3 | 3 | 4 | 4 | 1 |
| | | | 25 | 21 | 0 | 2 | 1 | 2 | 2 | 3 | 4 | 3 | 3 | 3 | 4 | 5 | 5 |
| | | | 26 | 21 | 0 | 2 | 2 | 1 | 0 | 4 | 1 | 1 | 3 | 3 | 3 | 1 | 0 |
| | | User 3 | 27 | 21 | 0 | 2 | 3 | 4 | 3 | 0 | 5 | 5 | 4 | 5 | 5 | 6 | 6 |
| | | | 28 | 21 | 0 | 2 | 2 | 1 | 2 | 2 | 2 | 0 | 1 | 5 | 1 | 4 | 4 |
| | | | 29 | 21 | 0 | 4 | 3 | 1 | 4 | 1 | 2 | 1 | 1 | 3 | 1 | 3 | 6 |
| | | | 30 | 21 | 0 | 3 | 4 | 3 | 5 | 1 | 1 | 1 | 1 | 6 | 3 | 3 | 8 |

(1) Acc: Acclimation
(2) Day of Injection
w: week

【Table 7】

| Group | Injection | User | Animal ID | Acc[1] | SCI | 1w | 2w | 3w | 4w[2] | 5w | 6w | 7w | 8w | 9w | 10w | 11w | 12w |
|---|---|---|---|---|---|---|---|---|---|---|---|---|---|---|---|---|---|
| E | 5 | | 31 | 21 | 0 | 3 | 2 | 4 | 3 | 4 | 4 | 4 | 5 | 5 | 5 | 5 | 5 |
| | | | 32 | 21 | 0 | 2 | 3 | 3 | 4 | 5 | 5 | 6 | 4 | 6 | 5 | 5 | 6 |
| | | User 1 | 33 | 21 | 0 | 1 | 2 | 1 | 3 | 4 | 6 | 6 | 4 | 4 | 3 | 3 | 6 |
| | | | 34 | 21 | 0 | 1 | 0 | 1 | 1 | 1 | 1 | 0 | 0 | 1 | 0 | 1 | 1 |
| | | | 35 | 21 | 0 | 1 | 4 | 2 | 4 | 3 | 5 | 5 | 4 | 4 | 3 | 3 | 6 |
| | | | 36 | 21 | 0 | 3 | 4 | 3 | 4 | 7 | 7 | 5 | 7 | 7 | 5 | 7 | 5 |
| | | | 31 | 21 | 0 | 4 | 1 | 3 | 2 | 3 | 4 | 4 | 3 | 4 | 4 | 4 | 3 |
| | | | 32 | 21 | 0 | 4 | 3 | 4 | 4 | 3 | 4 | 4 | 3 | 4 | 4 | 4 | 1 |
| | | User 2 | 33 | 21 | 0 | 2 | 1 | 1 | 4 | 4 | 4 | 3 | 4 | 6 | 7 | 7 | 13 |
| | | | 34 | 21 | 0 | 1 | 2 | 1 | 3 | 3 | 3 | 3 | 2 | 3 | 3 | 3 | 0 |
| | | | 35 | 21 | 0 | 0 | 0 | 1 | 1 | 1 | 1 | 1 | 0 | 1 | 1 | 1 | 1 |
| | | | 36 | 21 | 0 | 0 | 0 | 0 | 7 | 6 | 8 | 8 | 7 | 8 | 8 | 8 | 6 |
| | | | 31 | 21 | 0 | 2 | 1 | 2 | 2 | 3 | 2 | 4 | 2 | 4 | 4 | 4 | 4 |
| | | | 32 | 21 | 0 | 2 | 2 | 1 | 4 | 4 | 4 | 4 | 2 | 6 | 2 | 3 | 1 |
| | | User 3 | 33 | 21 | 0 | 2 | 3 | 4 | 4 | 4 | 6 | 6 | 7 | 9 | 7 | 7 | 8 |
| | | | 34 | 21 | 0 | 2 | 2 | 1 | 2 | 2 | 0 | 1 | 2 | 2 | 2 | 2 | 2 |
| | | | 35 | 21 | 0 | 4 | 3 | 1 | 1 | 1 | 3 | 3 | 2 | 4 | 3 | 2 | 3 |
| | | | 36 | 21 | 0 | 3 | 4 | 3 | 4 | 9 | 10 | 9 | 9 | 9 | 11 | 11 | 12 |

(1) Acc: Acclimation
(2) Day of Injection
w: week

As shown in Fig. 12, at 0 weeks prior to administration, the BBB scores of the E0-E5 groups were identical, with a maximum score of 21. In the spinal cord injury model group(E1), the BBB score was markedly reduced due to injury between lumbar levels L4-L5, and a consistently low BBB score of approximately 4 or less was recorded throughout the BBB score measurement period. This indicates that the spinal cord was injured.

Meanwhile, E2-E5 groups administered with STUP-001 of the present invention exhibited an overall tendency toward increased BBB scores compared with the vehicle control group after administration.

In the E2 group, BBB scores were statistically significantly increased compared with the vehicle control group (designated as E1) from approximately weeks 3 to 8 after administration. In particular, in the E2 group, the BBB score was measured to be approximately 5 points or higher from about week 6 onward.

In the E3 group, BBB scores were significantly increased compared with the vehicle control group from approximately weeks 4 to 8. In addition, at approximately week 8, the E3 group showed a statistically significant increase in BBB scores compared with the E4 and E5 groups.

The E4 and E5 groups also exhibited a tendency toward increased BBB scores.

These results indicate that administration of STUP-001 according to the present invention leads to recovery of spinal cord injury in mice, as reflected by increased BBB scores. In particular, compared with the vehicle control group (E1), the E2 and E3 groups showed a greater tendency toward higher BBB scores, with the E2 group exhibiting the highest overall BBB score trend among the treatment groups.

**Experiment Example 5-3: Confirmation of therapeutic effects on spinal cord injury through electrophysiological analysis**

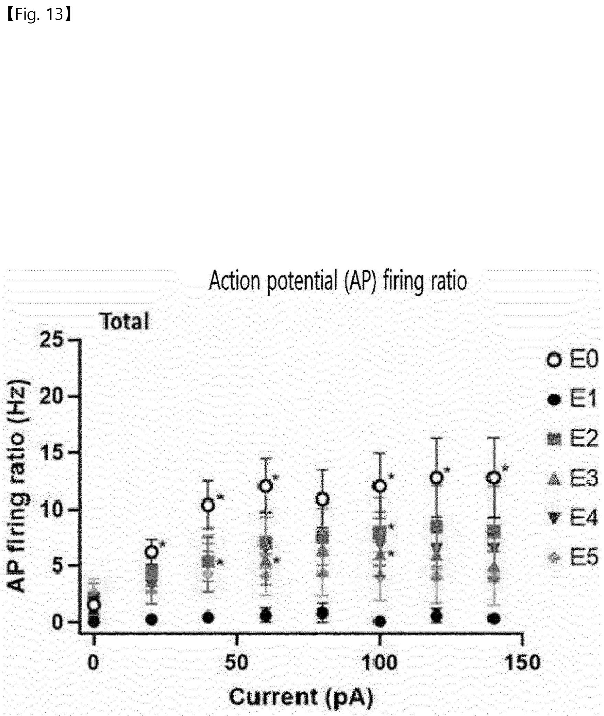

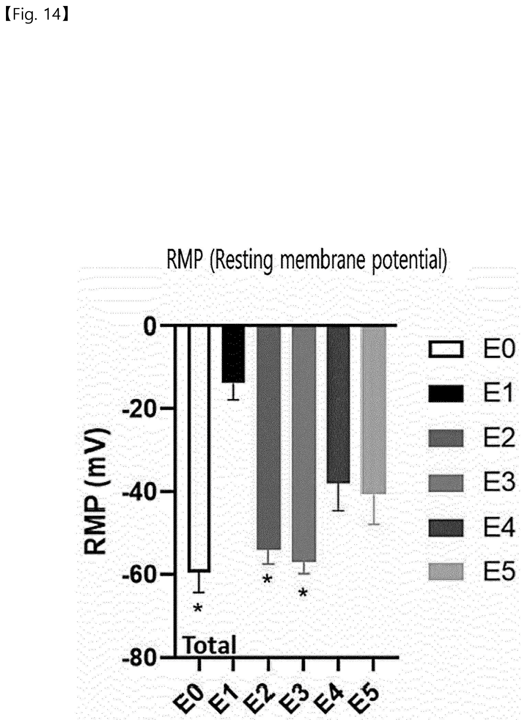

Electrophysiological analysis was performed by measuring the action potential (AP) firing ratio and the resting membrane potential (RMP) after administering STUP-001 at different concentrations to an SCI mouse model (Figs. 13 and 14).

Fig. 13 shows the results of measuring the action potential (AP) firing ratio. As a result, the vehicle control group E1 exhibited a statistically significant decrease in the frequency of AP firing compared with the normal group E0. In contrast, the groups of the present invention, E2 and E3, showed a highly significant increase in the AP firing ratio at current injections of 40, 60, and 100 pA compared with the E1 group. In addition, the E4 and E5 groups also exhibited a tendency toward increased AP firing ratios. That is, administration of STUP-001 according to the present invention was confirmed to show an overall tendency to improve the AP firing ratio.

Fig. 14 shows the results of measuring the resting membrane potential (RMP).

As a result, the normal group E0 exhibited an average resting membrane potential of approximately $-59.442 \pm 4.948$ mV, whereas the vehicle control group E1 exhibited an average resting membrane potential of approximately $-13.672 \pm 9.995$ mV. The resting membrane potential of the E1 group was statistically significantly higher than that of the E0 group, indicating that spinal cord injury results in an elevation of the resting membrane potential.

When STUP-001 of the present invention was administered to the SCI mouse model, all of groups E2 to E5 exhibited substantially restored resting membrane potentials compared with the vehicle control group E1. In particular, the E2 group showed an average resting membrane potential of approximately $-54.690 \pm 9.147$ mV, and the E3 group showed an average of approximately $-57.89 \pm 7.382$ mV, indicating recovery to levels comparable to those of the normal group E0.

**Experimental Example 5-4: Measurement of Protein Expression Levels Related to Differentiation Efficacy**

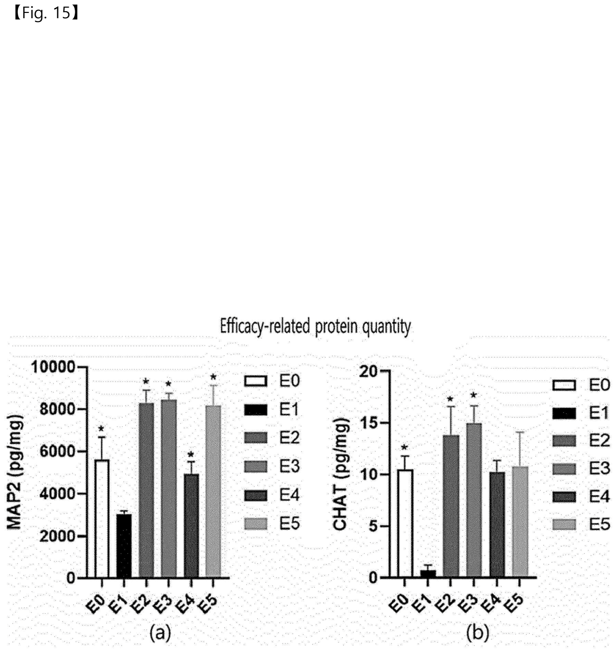

In groups E0 to E5, the expression levels of MAP2 and ChAT in spinal cord tissues at the STUP-001 administration sites of sacrificed mice were measured using ELISA, and the results are shown in Fig. 15.

MAP2 is a marker expressed by mature neurons, and ChAT is a marker expressed by motor neurons.

As confirmed in the preceding experimental examples (Experimental Examples 3 and 4), STUP-001 of the present invention induces direct reprogramming of astrocytes or fibroblasts into motor neurons. Accordingly, in the E2-E5 groups in which STUP-001 of the present invention was administered to the SCI mouse model, the number of motor neurons increases at the administration site (the spinal cord injury site). As a result, the expression levels of MAP2 and ChAT at the corresponding site are also increased.

It was confirmed that, compared with the normal group E0, the vehicle control group E1 exhibited a statistically significant decrease in the expression levels of MAP2 protein and ChAT protein. That is, when the spinal cord is injured, the expression of MAP2 and ChAT is reduced.

In contrast, it was confirmed that, in the groups E2 to E5 administered with STUP-001 of the present invention, the expression levels of MAP2 protein and ChAT protein were statistically significantly increased compared with those in the E1 group (Fig. 15).

Thus, through Example 5, it was confirmed that, when STUP-001 was administered to the SCI mouse model, the BBB score, action potential firing rate, resting membrane potential, and efficacy-related protein levels exhibited values close to those of the normal group. These results suggest that STUP-001 of the present invention effectively induces direct reprogramming of somatic cells at the spinal cord injury site into motor neurons, thereby markedly increasing the number of motor neurons at the injured site (regeneration and generation of motor neurons), and, as a result, enabling treatment of spinal cord injury.

In particular, the E2 group treated with STUP-001 at a dose of $2.0 \times 10^8$ GC/head and the E3 group treated with STUP-001 at a dose of $2.0 \times 10^7$ GC/head exhibited exceptionally superior therapeutic effects.

**Experimental Example 6: Toxicity assessment of a therapeutic agent for spinal cord injury from multiple perspectives**

Thereafter, in order to investigate the toxicity of STUP-001 as a therapeutic agent for spinal cord injury and to evaluate it from more diverse perspectives, the experiments were designed and conducted as shown in Table 8 below.

Hereinafter, groups G1 to G4 refer to the following:

G1 is a vehicle control group in which an AAV2 buffer was administered to ICR mice;

G2 is a group in which STUP-001 was administered to ICR mice at a dose of $2.5 \times 10^9$ GC/head;

G3 is a group in which STUP-001 was administered to ICR mice at a dose of $5.0 \times 10^9$ GC/head; and

G4 is a group in which STUP-001 was administered to ICR mice at a dose of $1.0 \times 10^{10}$ GC/head.

【Table 8】

| Group | Sex | Number of Animals | Animal ID No. | Dose Volume (μL/head) | Dose (GC/head) |
|---|---|---|---|---|---|
| G1 | M / F | 10 / 10 | 1–10 / 41–50 | 2 | 0 |
| G2 | M / F | 10 / 10 | 11–20 / 51–60 | 2 | $2.5 \times 10^9$ |
| G3 | M / F | 10 / 10 | 21–30 / 61–70 | 2 | $5.0 \times 10^9$ |
| G4 | M / F | 10 / 10 | 31–40 / 71–80 | 2 | $1.0 \times 10^{10}$ |

Notes:

- G1: Vehicle control group
- G2–G4: Test article–treated groups

**Experimental Example 6-1: Body weight measurement**

Tables 9 and 10 illustrate the results of body weight measurements for groups G1 to G4.
Table 9 shows the body weights of male mice, and Table 10 shows the body weights of female mice.

【Table 9】

| Sex: Male | | | G1 0 GC/head | G2 2.5x10^9 GC/head | G3 5.0x10^9 GC/head | G4 1.0x10^10 GC/head |
|---|---|---|---|---|---|---|
| | Day(s) Relative to Start Date | | | | | |
| Body wt. (g) | 1 [a] | Mean | 31.07 | 31.16 | 31.15 | 31.19 |
| | | SD | 1.24 | 1.07 | 1.49 | 1.38 |
| | | N | 10 | 10 | 10 | 10 |
| | | %Diff | - | 0.3 | 0.2 | 0.4 |
| | 7 [a] | Mean | 32.64 | 32.54 | 32.83 | 32.88 |
| | | SD | 1.44 | 1.44 | 1.90 | 2.06 |
| | | N | 10 | 10 | 10 | 10 |
| | | %Diff | - | -0.3 | 0.6 | 0.7 |
| | 14 [a] | Mean | 33.20 | 33.18 | 33.38 | 34.01 |
| | | SD | 1.65 | 1.43 | 2.13 | 1.99 |
| | | N | 10 | 10 | 10 | 10 |
| | | %Diff | - | -0.1 | 0.5 | 2.4 |
| | 21 [a] | Mean | 34.79 | 34.98 | 34.64 | 35.71 |
| | | SD | 1.76 | 1.55 | 2.56 | 2.27 |
| | | N | 10 | 10 | 10 | 10 |
| | | %Diff | - | 0.5 | -0.4 | 2.6 |
| | 28 [a] | Mean | 36.07 | 36.28 | 35.59 | 36.69 |
| | | SD | 1.88 | 1.54 | 2.54 | 2.47 |
| | | N | 10 | 10 | 10 | 10 |
| | | %Diff | - | 0.6 | -1.3 | 1.7 |
| | 35 [a] | Mean | 36.70 | 37.21 | 36.70 | 37.67 |
| | | SD | 1.96 | 1.45 | 2.55 | 3.17 |
| | | N | 10 | 10 | 10 | 10 |
| | | %Diff | - | 1.4 | 0.0 | 2.6 |
| | 42 [a] | Mean | 37.15 | 37.56 | 37.27 | 38.47 |
| | | SD | 1.99 | 1.70 | 2.84 | 3.13 |
| | | N | 10 | 10 | 10 | 10 |
| | | %Diff | - | 1.1 | 0.3 | 3.6 |
| | 49 [a] | Mean | 37.80 | 37.94 | 38.22 | 39.38 |
| | | SD | 1.98 | 2.05 | 2.61 | 3.71 |
| | | N | 10 | 10 | 10 | 10 |
| | | %Diff | - | 0.4 | 1.1 | 4.2 |
| | 56 [a] | Mean | 38.57 | 39.12 | 38.76 | 40.58 |
| | | SD | 1.94 | 2.31 | 2.92 | 4.10 |
| | | N | 10 | 10 | 10 | 10 |
| | | %Diff | - | 1.4 | 0.5 | 5.2 |
| | 63 [a] | Mean | 38.83 | 39.45 | 39.02 | 41.04 |
| | | SD | 2.01 | 2.15 | 3.09 | 4.03 |
| | | N | 10 | 10 | 10 | 10 |
| | | %Diff | - | 1.6 | 0.5 | 5.7 |
| | 70 [a] | Mean | 39.15 | 40.01 | 39.46 | 41.73 |
| | | SD | 1.99 | 2.41 | 3.40 | 4.29 |
| | | N | 10 | 10 | 10 | 10 |
| | | %Diff | - | 2.2 | 0.8 | 6.6 |

| Sex: Male | | | G1 0 GC/head | G2 2.5x10^9 GC/head | G3 5.0x10^9 GC/head | G4 1.0x10^10 GC/head |
|---|---|---|---|---|---|---|
| Day(s) Relative to Start Date | | | | | | |
| Body wt. (g) | 77 [a] | Mean | 39.05 | 40.49 | 39.69 | 42.00 |
| | | SD | 2.26 | 2.28 | 3.52 | 4.87 |
| | | N | 10 | 10 | 10 | 10 |
| | | %Diff | - | 3.7 | 1.6 | 7.5 |
| | 84 [a] | Mean | 40.42 | 41.65 | 41.02 | 42.60 |
| | | SD | 2.75 | 2.87 | 3.90 | 5.36 |
| | | N | 10 | 10 | 10 | 10 |
| | | %Diff | - | 3.1 | 1.5 | 5.4 |
| | 91 [a] | Mean | 39.50 | 40.85 | 40.61 | 42.01 |
| | | SD | 2.26 | 2.58 | 4.05 | 5.29 |
| | | N | 10 | 10 | 10 | 10 |
| | | %Diff | - | 3.4 | 2.8 | 6.4 |
| Gain | 1 → 91 [a] | Mean | 8.42 | 9.69 | 9.46 | 10.82 |
| | | SD | 1.74 | 1.97 | 3.41 | 4.47 |
| | | N | 10 | 10 | 10 | 10 |
| | | %Diff | - | 15.1 | 12.3 | 28.5 |

【Table 10】

| Sex: Female | | | G1<br>0<br>GC/head | G2<br>2.5x10^9<br>GC/head | G3<br>5.0x10^9<br>GC/head | G4<br>1.0x10^10<br>GC/head |
|---|---|---|---|---|---|---|
| | Day(s) Relative to Start Date | | | | | |
| Body wt.<br>(g) | 1 [a] | Mean | 27.01 | 26.87 | 26.52 | 26.60 |
| | | SD | 1.92 | 1.76 | 1.99 | 1.60 |
| | | N | 10 | 10 | 10 | 10 |
| | | %Diff | - | -0.5 | -1.8 | -1.5 |
| | 7 [a] | Mean | 28.61 | 28.64 | 27.31 | 28.32 |
| | | SD | 2.15 | 1.49 | 1.93 | 1.43 |
| | | N | 10 | 10 | 10 | 10 |
| | | %Diff | - | 0.1 | -4.5 | -1.0 |
| | 14 [a] | Mean | 29.47 | 29.88 | 28.20 | 29.96 |
| | | SD | 2.06 | 1.20 | 1.64 | 1.98 |
| | | N | 10 | 10 | 10 | 10 |
| | | %Diff | - | 1.4 | -4.3 | 1.7 |
| | 21 [a] | Mean | 31.25 | 30.86 | 29.28 | 31.49 |
| | | SD | 2.71 | 1.61 | 1.80 | 1.82 |
| | | N | 10 | 10 | 10 | 10 |
| | | %Diff | - | -1.3 | -6.3 | 0.8 |
| | 28 [a] | Mean | 32.22 | 32.21 | 30.37 | 32.63 |
| | | SD | 3.62 | 2.07 | 1.98 | 2.33 |
| | | N | 10 | 10 | 10 | 10 |
| | | %Diff | - | 0.0 | -5.8 | 1.3 |
| | 35 [a] | Mean | 33.07 | 32.61 | 30.45 | 32.73 |
| | | SD | 3.55 | 2.35 | 1.99 | 2.31 |
| | | N | 10 | 10 | 10 | 10 |
| | | %Diff | - | -1.4 | -7.9 | -1.0 |
| | 42 [a] | Mean | 32.95 | 32.92 | 31.41 | 34.19 |
| | | SD | 3.50 | 2.38 | 2.50 | 2.91 |
| | | N | 10 | 10 | 10 | 10 |
| | | %Diff | - | -0.1 | -4.7 | 3.8 |
| | 49 [a] | Mean | 34.03 | 33.98 | 32.50 | 35.21 |
| | | SD | 4.17 | 2.62 | 2.50 | 3.51 |
| | | N | 10 | 10 | 10 | 10 |
| | | %Diff | - | -0.1 | -4.5 | 3.5 |
| | 56 [a] | Mean | 34.71 | 34.55 | 33.48 | 36.40 |
| | | SD | 3.91 | 2.94 | 3.25 | 4.04 |
| | | N | 10 | 10 | 10 | 10 |
| | | %Diff | - | -0.4 | -3.5 | 4.9 |
| | 63 [a] | Mean | 34.97 | 35.59 | 33.47 | 36.65 |
| | | SD | 3.64 | 3.41 | 3.01 | 4.21 |
| | | N | 10 | 10 | 10 | 10 |
| | | %Diff | - | 1.8 | -4.3 | 4.8 |
| | 70 [a] | Mean | 36.29 | 35.77 | 34.20 | 38.14 |
| | | SD | 4.80 | 2.88 | 3.46 | 4.88 |
| | | N | 10 | 10 | 10 | 10 |
| | | %Diff | - | -1.5 | -5.8 | 5.1 |

| Sex: Female | | | G1<br>0<br>GC/head | G2<br>2.5x10^9<br>GC/head | G3<br>5.0x10^9<br>GC/head | G4<br>1.0x10^10<br>GC/head |
|---|---|---|---|---|---|---|
| Day(s) Relative to Start Date | | | | | | |
| Body wt.<br>(g) | 77 [a] | Mean | 35.34 | 35.99 | 34.55 | 37.71 |
| | | SD | 3.89 | 3.39 | 3.55 | 5.19 |
| | | N | 10 | 10 | 10 | 10 |
| | | %Diff | - | 1.9 | -2.2 | 6.7 |
| | 84 [a] | Mean | 36.33 | 36.96 | 34.84 | 38.83 |
| | | SD | 4.71 | 3.28 | 3.75 | 5.93 |
| | | N | 10 | 10 | 10 | 10 |
| | | %Diff | - | 1.7 | -4.1 | 6.9 |
| | 91 [a] | Mean | 35.90 | 36.16 | 34.29 | 38.05 |
| | | SD | 5.55 | 3.20 | 4.44 | 6.00 |
| | | N | 10 | 10 | 10 | 10 |
| | | %Diff | - | 0.7 | -4.5 | 6.0 |
| Gain | 1 → 91 [a] | Mean | 8.90 | 9.29 | 7.77 | 11.45 |
| | | SD | 4.78 | 3.34 | 3.48 | 4.65 |
| | | N | 10 | 10 | 10 | 10 |
| | | %Diff | - | 4.4 | -12.7 | 28.7 |

Based on the measurement results, it can be seen that both male and female mice in groups G2 to G4 showed no difference in body weight compared with male and female mice in group G1. That is, STUP-001 of the present invention is expected not to cause toxicologically harmful changes, such as body weight loss, during follow-up observation after administration.

## Experimental Example 6-2: Functional Observation Test

Tables 11 (functional observation assessment in males) and 12 (functional observation assessment in females) show the results of functional observation tests for groups G1 to G4. This experiment was conducted to evaluate safety pharmacology in the central nervous system. As functional observation tests, stimulus responsiveness tests were performed, including auditory response, righting reflex, pain response, ear reflex, and menace reflex. In addition, grip strength was measured to assess neurological and muscular conditions. Locomotor activity was evaluated to assess movement outside the home cage.

Based on the measurement results, it was confirmed that male and female animals in groups G2 to G4 showed no differences in any of the functional observation parameters compared with male and female animals in group G1. That is, it is expected that STUP-001 of the present invention does not cause adverse effects on central nervous system functions, such as impairment or weakening of various physiological functions in subjects.

【Table 11】

| Sex: Male | | G1 0 GC/head | G2 2.5x10^9 GC/head | G3 5.0x10^9 GC/head | G4 1.0x10^10 GC/head |
|---|---|---|---|---|---|
| GS [a] | Mean SD N | 256.8 11.7 10 | 261.5 19.9 10 | 286.4** 23.1 10 | 278.1 24.1 10 |
| LA [a] | Mean SD N | 305 67 10 | 368 96 10 | 344 57 10 | 348 81 10 |
| ASR Normal | Count+ | 10 | 10 | 10 | 10 |
| ASR NotApply | Count+ | 0 | 0 | 0 | 0 |
| MER Normal | Count+ | 10 | 10 | 10 | 10 |
| MER NotApply | Count+ | 0 | 0 | 0 | 0 |
| ARR Normal | Count+ | 10 | 10 | 10 | 10 |
| ARR NotApply | Count+ | 0 | 0 | 0 | 0 |
| AR Normal | Count+ | 10 | 10 | 10 | 10 |
| AR NotApply | Count+ | 0 | 0 | 0 | 0 |
| NR Normal | Count+ | 10 | 10 | 10 | 10 |
| NR NotApply | Count+ | 0 | 0 | 0 | 0 |

[a] - Anova & Dunnett: ** = p < 0.01
GS : Grip strength, LA : Locomotor activity, ASR : Auditory Startle Reflex
MER : Menace Reflex, ARR : Air Righting Reflex, AR : Auricle Reflex
NR : Nociceptive Reflex

【Table 12】

| Sex: Female | | G1<br>0<br>GC/head | G2<br>2.5x10^9<br>GC/head | G3<br>5.0x10^9<br>GC/head | G4<br>1.0x10^10<br>GC/head |
|---|---|---|---|---|---|
| GS [a] | Mean<br>SD<br>N | 236.4<br>12.6<br>10 | 225.2<br>9.7<br>10 | 220.4*<br>8.8<br>10 | 229.3<br>16.4<br>10 |
| LA [a] | Mean<br>SD<br>N | 448<br>288<br>10 | 416<br>255<br>10 | 311<br>50<br>10 | 321<br>62<br>10 |
| ASR Normal | Count+ | 10 | 10 | 10 | 10 |
| ASR NotApply | Count+ | 0 | 0 | 0 | 0 |
| MER Normal | Count+ | 10 | 10 | 10 | 10 |
| MER NotApply | Count+ | 0 | 0 | 0 | 0 |
| ARR Normal | Count+ | 10 | 10 | 10 | 10 |
| ARR NotApply | Count+ | 0 | 0 | 0 | 0 |
| AR Normal | Count+ | 10 | 10 | 10 | 10 |
| AR NotApply | Count+ | 0 | 0 | 0 | 0 |
| NR Normal | Count+ | 10 | 10 | 10 | 10 |
| NR NotApply | Count+ | 0 | 0 | 0 | 0 |

[a] - Anova & Dunnett: * = $p < 0.05$
GS : Grip strength, LA : Locomotor activity, ASR : Auditory Startle Reflex
MER : Menace Reflex, ARR : Air Righting Reflex, AR : Auricle Reflex
NR : Nociceptive Reflex

## Experimental Example 6-3: General toxicity evaluation

The general toxicity evaluation was conducted to investigate toxicity observed for 13 weeks after administration of STUP-001 to healthy mice, and to identify the maximum no-observed-adverse-effect level (NOAEL) and target organs. The general toxicity evaluation was assessed based on necropsy findings.

Table 13 presents the necropsy findings obtained for the toxicity evaluation. Based on the necropsy results, observations were examined across various tissues and organs in the body, and no findings indicative of toxicity were identified. That is, since STUP-001 of the present invention does not exhibit toxicity toward various biological tissues of the subject, it is expected to be usable as a therapeutic agent with high safety.

【Table 13】

| Organ | Gross Findings | Group (Animal No.) | Histopathological Diagnosis |
|---|---|---|---|
| Ear | Partial crust on both sides | G3 (#22) | Erosion/ulcer |
| Testis | Left side small | G3 (#26) | Atrophy |
| Epididymis | Left side small | G3 (#26) | Atrophy |
| Eye | Unilateral corneal opacity | G4 (#39) | No correlate |
| Jejunum | Diverticulum | G3 (#24), G4 (#37) | Diverticulum |
| Thyroid / Parathyroid gland | Bilateral enlargement | G2 (#51), G3 (#64) | Diffuse follicular dilatation (dilatation, follicle, diffuse) |
| | Left enlargement | G4 (#34) | No correlate |
| Kidney | Bilateral hydronephrosis | G1 (#50) | Pelvic dilatation (dilatation, pelvis) |
| Ureter | Bilateral dilatation | G1 (#50) | Dilatation |
| Urinary bladder | Abnormally filled with contents | G1 (#50) | Dilatation |
| Liver | Dark red focal contents in gallbladder region | G1 (#47) | No correlate |
| Lung | Focal nodule | G2 (#57) | Adenoma |
| Ovary | Abnormal shape (right) | G3 (#63) | No correlate |
| | Soft consistency (right) | | |
| Pituitary gland | Partial abnormal shape | G1 (#43) | Cyst |
| | Partial discoloration | G4 (#75) | No correlate |
| Uterus | Clear fluid retention | G1 (#41, 44, 46), G2 (#54), G3 (#61, 67), G4 (#72, 75, 80) | Luminal dilatation |
| Tail | Partial cut | G4 (#72) | No correlate |

Through Experimental Example 6, it was confirmed that even in Group G4, which was treated with a very high dose of STUP-001 at $1.0 \times 10^{10}$ GC/head, no changes in body weight were observed and no toxic findings were identified.

These results demonstrate that STUP-001 of the present invention has very high suitability as a therapeutic agent for the treatment of spinal cord injury-related diseases.

Meanwhile, through the foregoing experimental examples, it was found that Gsta4, a novel differentiation factor discovered by the inventor of the present application, is an important factor for directly reprogramming somatic cells into motor neurons. In addition, Gsta4 exhibits higher differentiation efficiency than conventionally known differentiation factors, and furthermore, when Gsta4 is used together with conventional differentiation factors, the differentiation efficiency can be further increased compared to the use of the conventional differentiation factors alone.

Furthermore, the inventors of the present application clearly confirmed a therapeutic effect in an SCI mouse model, which is a paraplegia model caused by spinal cord injury

In particular, when an AAV form including Gsta4 was directly administered to the injured spinal cord of SCI mice, a therapeutic effect on the damaged spinal cord could be confirmed. In addition, when an AAV form including Gsta4, Mnx1, and Lhx3 was administered to SCI mice, not only a therapeutic effect on the damaged spinal cord but also stability and toxicity were evaluated, thereby demonstrating the suitability of the composition as a therapeutic agent.

Accordingly, the technology of the present application is expected to have industrial applicability not only as a therapeutic agent for spinal cord injury but also, more broadly, as a therapeutic agent for various neurological disorders.

[Industrial Applicability]

The present disclosure can provide a composition for treating spinal cord injury disorders and the use thereof.

[Sequence Listing Free Text]

This sequence relates to a Gsta4 (Glutathione S-Transferase Alpha 4) protein or a nucleic acid sequence encoding the Gsta4 protein.

**Claims**

1.   A pharmaceutical composition for treating a spinal cord injury disorder, the composition comprising:

    a nucleic acid sequence encoding a Gsta4 (Glutathione S-Transferase Alpha 4) protein;
    a nucleic acid sequence encoding an Lhx3 (LIM homeobox 3) protein; and
    a nucleic acid sequence encoding an MNX1 (Motor neuron and pancreas homeobox 1) protein.

2.   The pharmaceutical composition according to Claim 1, wherein the spinal cord injury disorder is selected from the group consisting of paraplegia, quadriplegia, amyotrophic lateral sclerosis (ALS), primary lateral sclerosis (PLS), progressive pseudobulbar palsy, progressive muscular atrophy, progressive bulbar palsy, and post-polio syndrome.

3.   The pharmaceutical composition according to Claim 1, wherein the nucleic acid sequence encoding the Gsta4 protein, the nucleic acid sequence encoding the Lhx3 protein, and the nucleic acid sequence encoding the MNX1 protein are independently contained in a vector, or two or more of the nucleic acid sequences are combined and contained in a vector.

4.   The pharmaceutical composition according to Claim 3, wherein the vector is an adeno-associated virus (AAV).

5.   The pharmaceutical composition according to Claim 4, wherein the adeno-associated virus is a type selected from the group consisting of AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.

6.   The pharmaceutical composition according to Claim 5, wherein the adeno-associated virus is AAV2.

7.   The pharmaceutical composition according to Claim 1, wherein the nucleic acid sequence encoding the Gsta4 protein is the sequence of SEQ ID NO: 12.

8.   The pharmaceutical composition according to Claim 1, wherein the nucleic acid sequence encoding the Lhx3 protein is the sequence of SEQ ID NO: 16.

9.   The pharmaceutical composition according to Claim 1, wherein the nucleic acid sequence encoding the MNX1 protein is the sequence of SEQ ID NO: 14.

10.   The pharmaceutical composition according to Claim 3, wherein the vector further comprises at least one selected from the group consisting of a promoter, an enhancer, a polyadenylation signal, a Kozak consensus sequence, an inverted terminal repeat (ITR), a long terminal repeat (LTR), a terminator, an internal ribosome entry site (IRES), and a 2A self-cleaving peptide.

11.   The pharmaceutical composition according to Claim 3, wherein the vector further comprises a promoter, a 2A self-cleaving peptide, and an inverted terminal repeat (ITR).

12.   The pharmaceutical composition according to Claim 11, wherein the promoter is a CMV promoter, and the 2A self-cleaving peptide is P2A or T2A.

13.   The pharmaceutical composition according to Claim 1, wherein the pharmaceutical composition is formulated as an injectable preparation.

14.   The pharmaceutical composition according to Claim 4, wherein the AAV contained in the pharmaceutical composition is administered to a subject in a dose ranging from $1 \times 10^5$ GC/kg to $1 \times 10^{15}$ GC/kg.

15.   The pharmaceutical composition according to Claim 14, wherein the AAV is administered to a subject in a dose

ranging from $1 \times 10^6$ GC/kg to $1 \times 10^{12}$ GC/kg.

16. The pharmaceutical composition according to Claim 1, wherein the pharmaceutical composition is administered to an injured spinal cord region of a subject having a spinal cord injury disorder, and the injured spinal cord region is selected from the group consisting of L1 (lumbar 1), L2 (lumbar 2), L3 (lumbar 3), L4 (lumbar 4), L5 (lumbar 5), T1 (thoracic 1), T2 (thoracic 2), T3 (thoracic 3), T4 (thoracic 4), T5 (thoracic 5), T6 (thoracic 6), T7 (thoracic 7), T8 (thoracic 8), T9 (thoracic 9), T10 (thoracic 10), T11 (thoracic 11), T12 (thoracic 12), T13 (thoracic 13), C1 (cervical 1), C2 (cervical 2), C3 (cervical 3), C4 (cervical 4), C5 (cervical 5), C6 (cervical 6), C7 (cervical 7), C8 (cervical 8), S1 (sacrum 1), S2 (sacrum 2), S3 (sacrum 3), and S4 (sacrum 4).

17. The pharmaceutical composition according to Claim 1, wherein the pharmaceutical composition is administered to a subject in a volume ranging from 10 µL to 100 µL per administration.

18. A pharmaceutical composition for treating a spinal cord injury disorder, the composition comprising: a nucleic acid sequence encoding a Gsta4 (Glutathione S-Transferase Alpha 4) protein.

19. The pharmaceutical composition according to Claim 18, wherein the spinal cord injury disorder is selected from the group consisting of paraplegia, quadriplegia, amyotrophic lateral sclerosis (ALS), primary lateral sclerosis (PLS), progressive pseudobulbar palsy, progressive muscular atrophy, progressive bulbar palsy, and post-polio syndrome.

20. The pharmaceutical composition according to Claim 18, wherein the nucleic acid sequence encoding the Gsta4 protein is contained in a vector.

21. The pharmaceutical composition according to Claim 20, wherein the vector is an adeno-associated virus (AAV).

22. The pharmaceutical composition according to Claim 21, wherein the adeno-associated virus is AAV2.

23. The pharmaceutical composition according to Claim 20, wherein the nucleic acid sequence encoding the Gsta4 protein is the sequence of SEQ ID NO: 12.

24. The pharmaceutical composition according to Claim 20, wherein the vector further comprises a promoter sequence.

25. The pharmaceutical composition according to Claim 24, wherein the promoter sequence is the sequence of SEQ ID NO: 37.

【Fig. 1】

(a) **AAV-CMV-hGSTA4**

(b) **AAV-CMV-hASCL1-T2A-NEUROG2-P2A-hISL1-T2A-hLHX3**

(c) **AAV-CMV-hBRN2-P2A-hMNX1-T2A-hNeuroD1**

(d) **AAV-CMV-hGSTA4-P2A-hMYT1L**

(e) **AAV-CMV-hMNX1-P2A-hLHX3-T2A-hGSTA4**

【Fig. 2】

【Fig. 3】

(a)

ChAT    Map2    Merge

Gsta4

(b)

【Fig. 4】

【Fig. 5】

【Fig. 6】

【Fig. 7】

【Fig. 8】

【Fig. 9】

【Fig. 10】

【Fig. 11】

【Fig. 12】

BBB (Basso, Beattie and Bresnahan) score

【Fig. 13】

Action potential (AP) firing ratio

【Fig. 14】

【Fig. 15】

Efficacy-related protein quantity

(a)

(b)

## INTERNATIONAL SEARCH REPORT

| International application No. |
| --- |
| **PCT/KR2023/009608** |

| A. | CLASSIFICATION OF SUBJECT MATTER |
| --- | --- |

**A61K 38/45**(2006.01)i; **A61K 38/17**(2006.01)i; **A61P 25/00**(2006.01)i; **A61K 9/00**(2006.01)i

According to International Patent Classification (IPC) or to both national classification and IPC

| B. | FIELDS SEARCHED |
| --- | --- |

Minimum documentation searched (classification system followed by classification symbols)

A61K 38/45(2006.01); A61K 48/00(2006.01); A61L 27/36(2006.01); A61L 27/38(2006.01); C12N 15/83(2006.01); G01N 33/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 척수(spinal cord), Glutathione S-Transferase Alpha 4(Gsta4), LIM homeobox 3(Lhx3), Motor neuron and pancreas homeobox 1(MNX1), 벡터(vector), 아데노-연관 바이러스(adeno-Associated virus, AAV)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | STROM, M. et al. Naturally occurring genetic variability in expression of gsta4 is associated with differential survival of axotomized rat motoneurons. Neuromolecular medicine. 2012, vol. 14, pp. 15-29 (published online: 08 December 2011).<br>See page 26. | 1-25 |
| Y | AKTER, M. et al. Modeling movement disorders via generation of hiPSC-derived motor neurons. Cells. 2022, vol. 11, no. 23, document no. 3796, inner pp. 1-25 (publication date: 27 November 2022).<br>See abstract, and table 6. | 1-25 |
| Y | WO 2023-004365 A1 (SIO GENE THERAPIES INC.) 26 January 2023 (2023-01-26)<br>See claims 1, 4, 19-21, 46-49 and 59. | 3-6,10-17,20-25 |
| A | KR 10-2020-0129709 A (UNIST(ULSAN NATIONAL INSTITUTE OF SCIENCE AND TECHNOLOGY)) 18 November 2020 (2020-11-18)<br>See claims 1, 4 and 7-10. | 1-25 |
| A | WO 2023-095149 A1 (RAMOT AT TEL-AVIV UNIVERSITY LTD.) 01 June 2023 (2023-06-01)<br>See claims 1-5. | 1-25 |

☐ Further documents are listed in the continuation of Box C.  ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **21 March 2024** | **22 March 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/009608** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/KR2023/009608**

| Patent document cited in search report | Publication date (day/month/year) | Patent family member(s) | Publication date (day/month/year) |
|---|---|---|---|
| WO 2023-004365 A1 | 26 January 2023 | None | |
| KR 10-2020-0129709 A | 18 November 2020 | KR 10-2217496 B1 | 19 February 2021 |
| WO 2023-095149 A1 | 01 June 2023 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Prog Brain Res*, 2002, vol. 137, 37-47

- *Mol. Cell*, 28 September 2012, vol. 47 (6), 827-838